# EUROPEAN PATENT SPECIFICATION

(11) **EP 3 529 364 B1**
(45) Date of publication and mention of the grant of the patent: **01.01.2020**
(21) Application number: 18746841.8
(22) Date of filing: 09.05.2018
(51) Int. Cl.: C12P 13/00, C12P 17/10, C12P 21/02, C12P 21/04, C07D 259/00, C07K 7/58

(54) **METHOD OF PRODUCING A NON-RIBOSOMAL PEPTIDE FROM CYANOBACTERIA**
VERFAHREN ZUR HERSTELLUNG EINER NICHT-RIBOSOMALEN PEPTIDE AUS CYANOBAKTERIEN
PRODUCTION D'UNE PEPTIDE NON RIBOSOMIQUE PAR CYANOBACTERIA

(30) Priority: 09.05.2017 EP 17170284
(43) Date of publication of application: 28.08.2019
(73) Proprietor: Cyano Biotech GmbH, 12489 Berlin (DE)
(72) Inventor: ENKE, Heike, 12307 Berlin (DE); LORENZEN, Wolfram, 10779 Berlin (DE); JAHNS, Stefan, 10243 Berlin (DE); ENKE, Dan, 12307 Berlin (DE); NIEDERMEYER, Timo, 06114 Halle (Saale) (DE); MOSCHNY, Julia, 92237 Sulzbach-Rosenberg (DE)
(74) Representative: Andresen, Heiko
(86) International application number: PCT/EP2018/062156
(87) International publication number: WO 2018/219619

(56) References cited:
- ZEMSKOV ET AL: "Total synthesis of microcystin-LF and derivatives thereof", JOURNAL OF ORGANIC CHEMISTRY, vol. 82, 15 March 2017 (2017-03-15), pages 3680-3691, XP002785549,
- NIEDERMEYER ET AL: "Selectivity and potency of microcystin congeners against OATP1B1 and OATP1B3 expressing cancer cells", PLOS ONLINE, vol. 9, 2014, pages 1-7, XP002785551,
- NIEDERMEYER ET AL: "Isolation of microcystins from the cyanobacterium Planktothrix rubescens strain No80", NATURAL PRODUCTS AND BIOPROSPECTING, vol. 4, 2014, pages 37-45, XP002785550,
- KRIES ET AL: "Reprogramming nonribosomal peptide synthetases for "clickable" amino acids", ANGEWANDTE CHEMIE, INTERNATIONAL EDITION, vol. 53, 2014, pages 10105-10108, XP002785560,
- SPICER ET AL: "Selective chemical protein modification", NATURE COMMUNICATIONS, vol. 5, 2014, pages 1-14, XP055338910,
- PATTERSON ET AL: "Finding the right (bioorthogonal) chemistry", ACS CHEMICAL BIOLOGY, vol. 9, 2014, pages 592-605, XP002785552,
- MOSCHNY: "New approaches to handle old compounds - the generation of microcystin and nodularin derivatives with "clickable" features", GA 2017 (Gesellschaft für Arzneipflanzen- und Naturstoff-Forschung e.V.) = 65th International Congress and Annual Meeting of the Society for Medicinal Plant and Natural Product Research; 03-09 September 2017, Basel, Switzerland Poster, September 2017 (2017-09), pages 1-2, XP002785553, Retrieved from the Internet: URL:https://coms.events/GA2017/data/abstra cts/en/abstract_0558.html [retrieved on 2018-10-09]
- MOSCHNY: "Julia Moschny", University of Halle Project presentation for Julia Moschny, 2017, pages 1-2, XP002785604, Retrieved from the Internet: URL:https://ag-bioarznei.pharmazie.uni-hal le.de/mitarbeiter/julia_moschny/ [retrieved on 2018-10-11]
- LIU ET AL: "Directing the heterologous production of specific cyanobacterial toxin variants", ACS CHEMICAL BIOLOGY, vol. 12, 1 June 2017 (2017-06-01), pages 2021-2029, XP055477678,
- FONTANILLO ET AL: "Microcystins: Synthesis and structure-activity relationship studies toward PP1 and PP2A", BIOORGANIC & MEDICINAL CHEMISTRY, vol. 26, 24 August 2017 (2017-08-24), pages 1118-1126, XP002785554,
- N.N.: "Microcystin-based payloads with clickable anchor groups", Cyano Biotech GmbH Project information, 2018, pages 1-2, XP002785559, Retrieved from the Internet: URL:http://cyano-biotech.com/content/curre ntprojects/learnmore_2.php [retrieved on 2018-10-10]

## Description

### FIELD OF INVENTION

This invention is in the field of cancer treatment. It is in the field of toxins for use in cancer treatment. It is in the field of non-ribosomal peptides from cyanobacteria (with microcystins, nodularins but also anabaenopeptins, oscillamides as examples) and their use in the treatment of diseases such as cancer, thrombosis, metabolic diseases but also for other applications. The invention relates to the field of microbiology, molecular biology, pharmacy and biotechnology in general and more specifically to the synthesis of modified non-ribosomal peptides including microcystins and nodularins. This invention is also in the field of enzyme inhibiting tools including phosphatase, proteinase and peptidase inhibiting biochemical tools.

More in particular, the present invention is a method of producing a modified non-ribosomal peptide from cyanobacteria.

### BACKGROUND

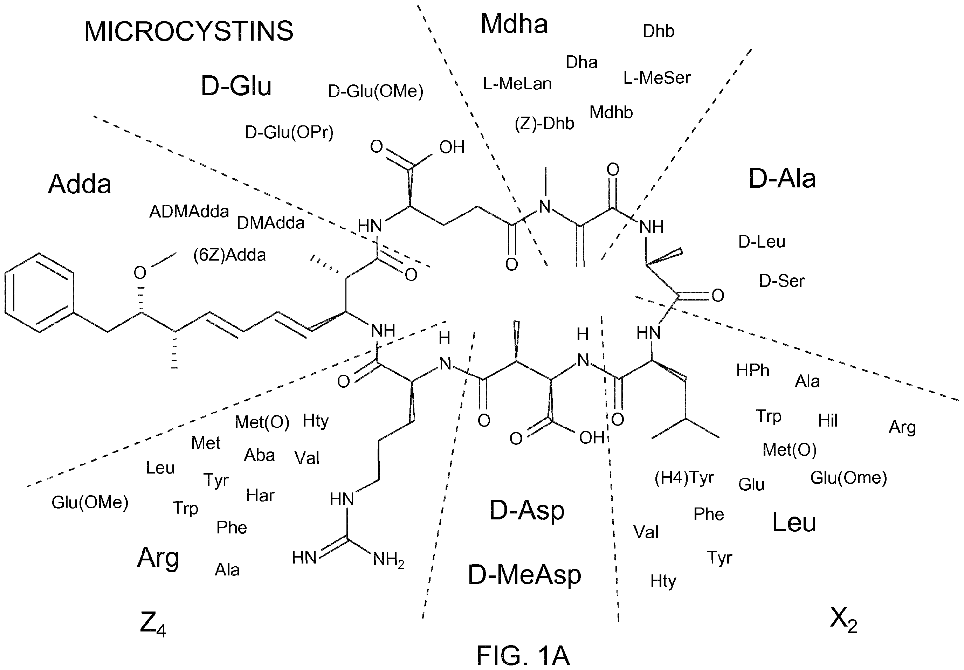

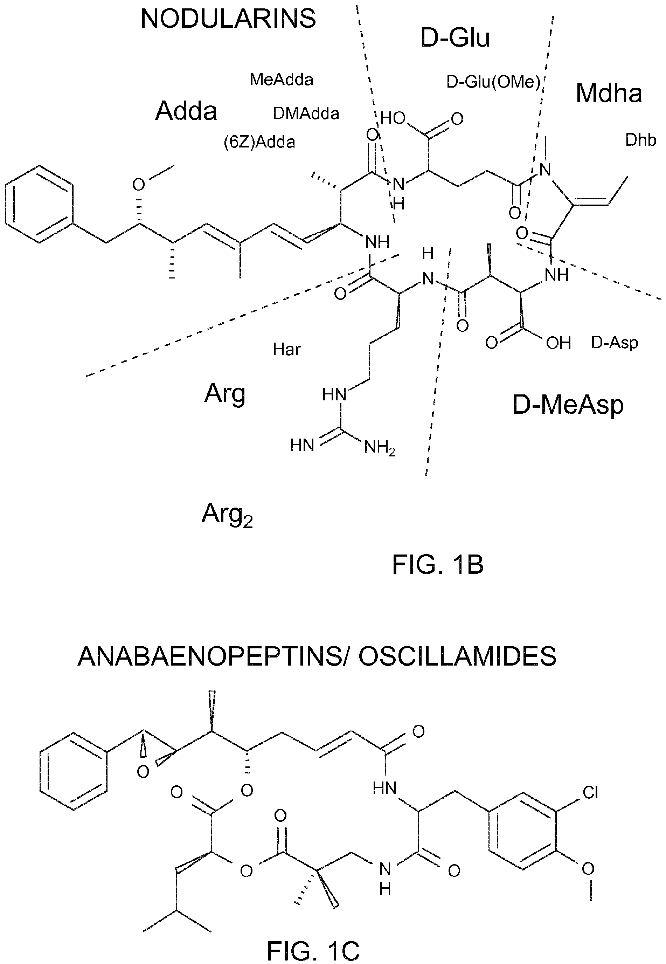

Microcystins are toxins produced naturally by cyanobacteria, also known as blue-green algae. When excess cyanobacteria grow in a lake or pond, they form an algal bloom, which often appears as a layer of green scum. However, not all green scum on a lake is an algal bloom, and not all algal blooms contain the kinds of cyanobacteria that produce microcystins. There are many microcystin congeners; microcystin-LR is one of the more toxic and well-studied congener. Microcystins are a group of cyclic heptapeptide hepatotoxins produced by a number of cyanobacterial genera. The most notable of which, and namesake, is the widespread genus Microcystis. Structurally, most microcystins consist of the generalized structure cyclo(-D-Ala1-X2-D-MeAsp3-Y4-Adda5-D-Glu6-Mdha7-). X and Y are variable L-amino acids, D-MeAsp is D-erythro-β-methylaspartic acid and Mdha is N-methyldehydroalanine. However, while X and Y are the most variable amino acids, variations can be found at all positions of the microcystin core structure (see Figure 1). Adda is the cyanobacteria unique C20-β-amino acid 3-amino-9-methoxy-2,6,8-trimethyl-10-phenyl-deca-4,6-dienoic acid. Substitutions of the variable L-amino acids at positions 2 and 4 and less frequently found alterations in the other constituent amino acids result in more than 100 reported natural microcystins to date.

Microcystins are potent inhibitors of type 1 and type 2A protein phosphatases. The IC₅₀ of microcystin-LR for example are 0.03 nM and 0.0 nM for type 1 and type 2A protein phosphatases, respectively.

Protein phosphatases 1 and 2A are two of the major phosphatases in eukaryotic cells that dephosphorylate serine and threonine residues.

Protein phosphatase 2B is inhibited 1000-fold less potently, while six other tested phosphatases and eight tested protein kinases are unaffected.

Nodularins are compounds structurally related to the microcystins, as they are evolutionary derived from microcystin and also contain the amino acid Adda found in the microcystins. They are produced especially by Nodularia species, and in contrast to microcystins they are cyclic pentapeptides with the most commonly found congener cyclo[-D-erythro-methylAsp-L-Arg-Adda-D-Glu-Mdhb], where Mdhb is N-methyldehydrobutyrate (see figure 1).

Microcystins and nodularins could serve as cancer drugs. It was hypothesized that natural microcystin variants could be isolated that are transported preferentially by the active transporter type OATP1B3 relative to OATP1B1 to advance as anticancer agents with clinically tolerable hepatic toxicity (OATP1B3 transporters are primarily found in cancer tissues, e.g. in liver cancers). Microcystin variants have been isolated and tested for cytotoxicity in cancer cells stably transfected with OATP1B1 and OATP1B3 transporters. Microcystin variants with cytotoxic OATP1B1/OATP1B3 IC₅₀ ratios that ranged between 0.2 and 32 were found, representing a 150-fold range in transporter selectivity. As the microcystin structure has a significant impact on transporter selectivity, it is potentially possible to develop analogs with even more pronounced OATP1B3 selectivity and thus enable their development as anticancer drugs. However, a more specific method of delivery would be preferred. One such method involves the novel concept disclosed herein, of adding a targeting moiety. Ideally for a targeted and highly specific cancer therapy that avoids off-target toxicities, the structural variant of a microcystin and nodularin would carry a targeting moiety (e.g. a cancer-specific monoclonal antibody) and is either not or badly transported by all OATP transporter subtypes or it is exclusively or primarily transported by the cancer-specific OATP subtype 1B3. As an example for differences in transport efficiencies among different structural variants of microcystins we refer to the following table.

Zemskov et al. (J. Org. Chem. 2017, 82, 3680-3691) discloses total chemical synthesis of Microcystin-LF and derivatives thereof.

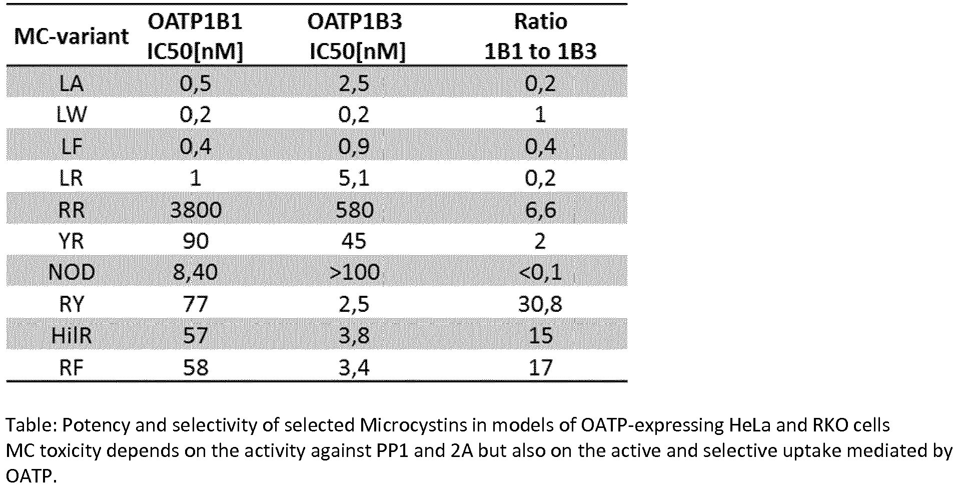

Microcystins are difficult to synthesize chemically. One more convenient way of obtaining microcystins involves the in vivo production of microcystins by cyanobacteria.

Previous experiments of academic groups intended to increase product yields of naturally produced non-ribosomal peptides (here microcystins) by feeding of amino acids, which are incorporated in at least one structural variant of the respective microcystin synthesized by the fed strain. More specific, feeding of the amino acids leucine (L, Leu) or arginine (R, Arg) to a cyanobacterial strain that produces the microcystin (MC) variants MC-LR and MC-RR (L for leucine; R for arginine) influences the yield of both variants in dependence of the fed amino acid. Furthermore, feeding of amino acids which are incorporated in at least one structural variant of the respective microcystin synthesized by the fed strain might also influence biomass production.

In addition, it also has been shown that feeding of amino acids that represent slightly modified versions of the amino acids which are naturally incorporated into the respective non-ribosomal peptide produced by the fed strain might be also incorporated into the respective non-ribosomal peptide. This approach is generally known as mutasynthesis. For cyanobacterial non-ribosomal peptides, however, this approach has to date been restricted to simple analogs of natural amino acids such as homotyrosine instead of tyrosine (differing by only one methylene group) or halogenated amino acids (differing by only one halogen atom) such as chloro-tyrosine instead of tyrosine. Feeding of more extensively modified amino acids or of amino acids and their analogs that are different from the amino acids that are naturally incorporated into the non-ribosomal peptide have not been reported to date. Moreover, it has been described in the literature that feeding of modified amino acids to be incorporated into microcystins is not possible. After the priority date of the present application, generation of microcystin derivatives *in vivo* by supplementing unnatural amino acid derivatives to growth media of Microcystis CBT480 was reported (Moschny et al., GA 2017, 03-09 September 2017, Basel, Switzerland).

There is a need for modified non-ribosomal peptides from cyanobacteria including modified cytotoxins from cyanobacteria like for instance modified microcystins (e.g. in connection with the optimization of microcystin-based cancer lead compounds). There is a need for methods of producing non-ribosomal peptides like microcystins as well as coupling microcystins to targeting units (e.g. in connection with the construction of antibody-drug conjugates for targeted therapy of cancers, infection diseases, thrombosis and other kinds of targeted therapies).

### SUMMARY OF INVENTION

The problem was solved by producing modified non-ribosomal peptides (e.g. microcystins, nodularins, anabaenopeptins, oscillamides, etc.) by means of incorporating one or more modified substrates into those non-ribosomal peptides.

The invention relates to a method of producing a modified non-ribosomal peptide from cyanobacteria, e.g. a modified microcystin and/or modified nodularin (together cytotoxic agents, CA), comprising the steps of:
a) growing a non-ribosomal peptide producing cyanobacteria strain in a culture media,
b) adding one or more modified substrates, preferably modified amino acids, to said culture, and
c) cultivating the strain in the presence of said modified substrates,
wherein the modified substrate is a modified amino acid, which comprises an anchor group directly accessible or transformable for use in conjugation chemistry incl. click chemistry, for the attachment of a targeting moiety or a label.

The invention also relates to a method of producing a compound for targeted therapy comprising a non-ribosomal peptide linked to a targeting moiety, with the steps:
A) Providing a targeting moiety and a non-ribosomal peptide comprising at least one modified amino acid, wherein the at least one modified amino acid comprises an anchor group directly accessible or transformable for use in conjugation chemistry incl. click chemistry by performing a method as defined hereinabove,
B) Attaching said targeting moiety to said non-ribosomal peptide via chemical conjugation to said anchor group.

### DEFINITIONS

Herein, **non-ribosomal peptides** are a class of peptide secondary metabolites synthesized by non-ribosomal peptide synthetases, which, unlike the ribosomes, are independent of messenger RNA. Each non-ribosomal peptide synthetase can synthesize only one type of peptide. Non-ribosomal peptides often have cyclic and/or branched structures, can contain non-proteinogenic amino acids including D-amino acids, carry modifications like N-methyl and N-formyl groups, or are glycosylated, acylated, halogenated, or hydroxylated. Cyclization of amino acids against the peptide "backbone" is often performed, resulting in oxazolines and thiazolines; these can be further oxidized or reduced. On occasion, dehydration is performed on serines, resulting in dehydroalanine. This is just a sampling of the various manipulations and variations that non-ribosomal peptides can perform. Non-ribosomal peptides are often dimers or trimers of identical sequences chained together or cyclized, or even branched. Non-ribosomal peptides are a very diverse family of natural products with an extremely broad range of biological activities and pharmacological properties. They are often toxins, siderophores, or pigments. Non-ribosomal peptide antibiotics, cytostatics, and immunosuppressants are in commercial use.

In contrast to non-ribosomal peptides from other microbial producers cyanobacterial non-ribosomal peptides possess an extraordinary high number of structural variants within one class of non-ribosomal peptides (see table). Furthermore, many cyanobacteria produce hybrid structures of non-ribosomal peptides and polyketides. Consequently, the multienzyme complexes for those hybrid structures of non-ribosomal peptides and polyketides are also built up by a non-ribosomal peptide synthetase part and a polyketide synthase part. Herein, a non-ribosomal peptide can be also a hybrid of a non-ribosomal peptide and a polyketide (e.g. the compound class of microcystins).

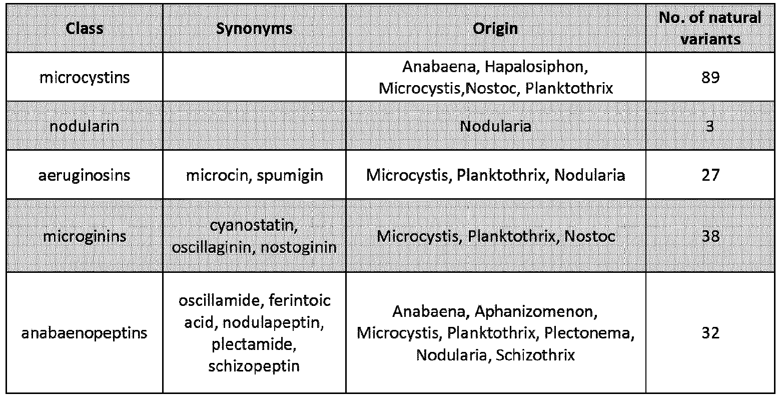

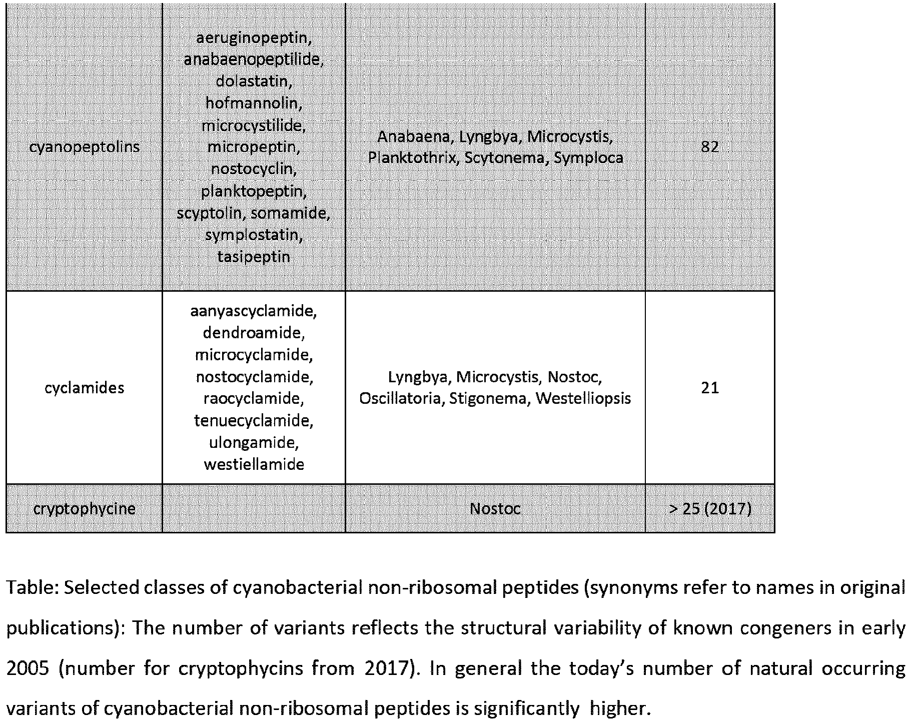

**Table: Natural occurring cryptophycins**

| | | | | | | | | | |
|---|---|---|---|---|---|---|---|---|---|
| 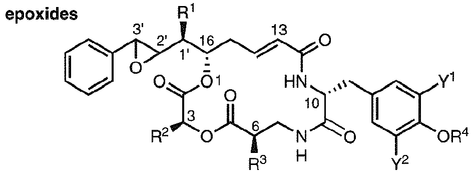 | | | | | | | | | |

| Compound | Epoxide | R¹ | R² | R³ | R⁴ | Y¹ | Y² | C13=C14 | |
|---|---|---|---|---|---|---|---|---|---|
| C-1 | β | Me | *i-*Bu | Me | Me | Cl | H | *trans* | |
| C-2 | β | Me | *i*-Bu | Me | Me | H | H | *trans* | |
| C-16 | β | Me | *i-*Bu | Me | H | Cl | H | *trans* | |
| C-21 | β | Me | *i*-Bu | H | Me | Cl | H | *trans* | |
| C-23 | β | Me | *i*-Bu | Me | H | Cl | Cl | *trans* | |
| C-24 | β | Me | *i*-Bu | H | Me | H | H | *trans* | |
| C-28 | β | H | *i*-Bu | Me | Me | Cl | H | *trans* | |
| C-31 | β | Me | *i*-Bu | Me | Me | Cl | Cl | *trans* | |
| C-38 | α | Me | *i*-Bu | Me | Me | Cl | H | *trans* | |
| C-50 | β | Me | *n*-Pr | Me | Me | Cl | H | *trans* | |
| C-54 | β | Me | *s*-Bu | Me | Me | Cl | H | *trans* | |
| C-176 | β | Me | *i*-Bu | H | H | Cl | H | *trans* | |
| C-326 | β | Me | *i*-Bu | H | Me | Cl | Cl | *trans* | |
| C-327 | β | Me | *i*-Bu | Me | Me | Cl | H | *cis* | |
| 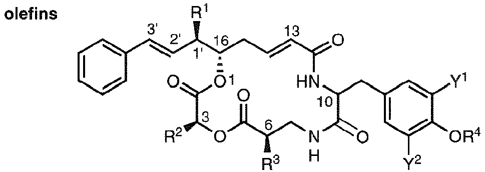 | | | | | | | | | |

| Compound | R¹ | R² | R³ | Config. at C10 | | R⁴ | | Y¹ | Y² |
|---|---|---|---|---|---|---|---|---|---|
| C-3 | Me | *i*-Bu | Me | *R* | | Me | | Cl | H |
| C-4 | Me | *i*-Bu | Me | *R* | | Me | | H | H |
| C-17 | Me | *i*-Bu | Me | *R* | | H | | Cl | H |
| C-18 | Me | *s*-Bu | Me | *R* | | Me | | Cl | H |
| C-19 | Me | *i*-Pr | Me | *R* | | Me | | Cl | H |
| C-29 | Me | *i*-Bu | H | *R* | | Me | | Cl | H |
| C-40 | H | *i*-Bu | Me | *R* | | Me | | Cl | H |
| C-43 | Me | *i*-Bu | Me | *R* | | H | | H | H |
| C-45 | Me | *i*-Bu | Me | *R* | | H | | Cl | Cl |
| C-46 | Me | *i*-Bu | Me | *S* | | Me | | Cl | H |
| C-49 | Me | *n*-Pr | Me | *R* | | Me | | Cl | H |
| C-175 | Me | *i*-Bu | Me | *R* | | Me | | Cl | Cl |
| 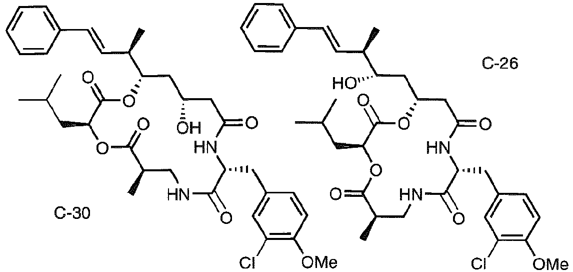 | | | | | | | | | |

Herein, a **microcystin** has the general structure of D-Ala₁-X₂-D-MeAsp₃-Z₄-Adda₅-D-Glu₆-Mdha₇, where structural variations may in principle occur at all positions but most frequently at X and Z (see Figure 1). These are the variable L-amino acids. D-MeAsp is D-erythro-b-methyl aspartic acid, Mdha is N-methyldehydroalanine, and Adda is 3-amino-9-methoxy-2,6,8-trimethyl-10-phenyldeca-4,6-dienoic acid. Demethylation at position 3 and/or 7 and methylation at position 6 is also within the scope of the invention as well as further modifications at the position 1, 5 and 7 as indicated in figure 1.

Herein we demonstrate multiple combinations of the variable L-amino acids (X and Z) in positions 2 and 4 and modifications in the other D-amino acids.

Herein, a **nodularin** is a monocyclic pentapeptide consisting of cyclo[-D-erythro-methylAsp (iso-linkage)-L-Arg-Adda-D-Glu(iso-linkage)-Mdhb], where Mdhb stands for N-methyldehydrobutyrate and Adda is the particular C20-amino acid: 3-amino-9-methoxy-2,6,8-trimethyl-10-phenyldeca-4,6-dienoic acid whereas all positions can naturally be slightly modified as indicated in figure 1. Nodularin closely resembles microcystins with respect to structure and biological activity.

Modifications of microcystins and nodularins shall not occur at the position for Adda and D-Glu as these two positions are essential for the inhibiting activity against PP1 and PP2A.

Herein, microcystin and nodularin as well as further cytotoxic non-ribosomal peptides from cyanobacteria in all their modified variations are referred to as cytotoxic agents, or **CA** (see table with selected cytotoxic non-ribosomal peptides from cyanobacteria).

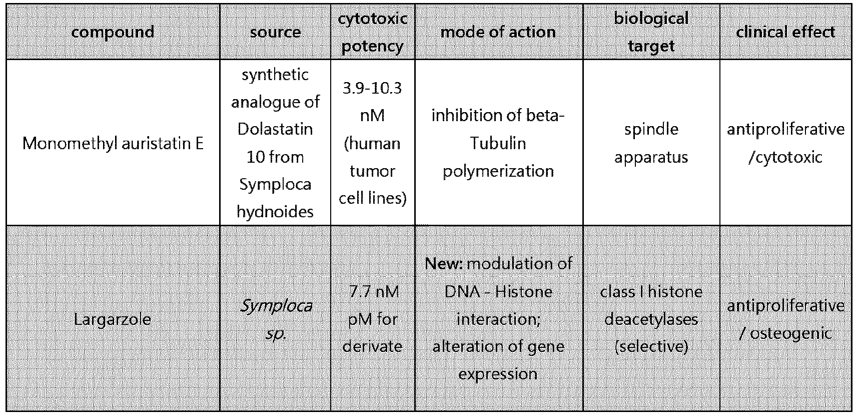

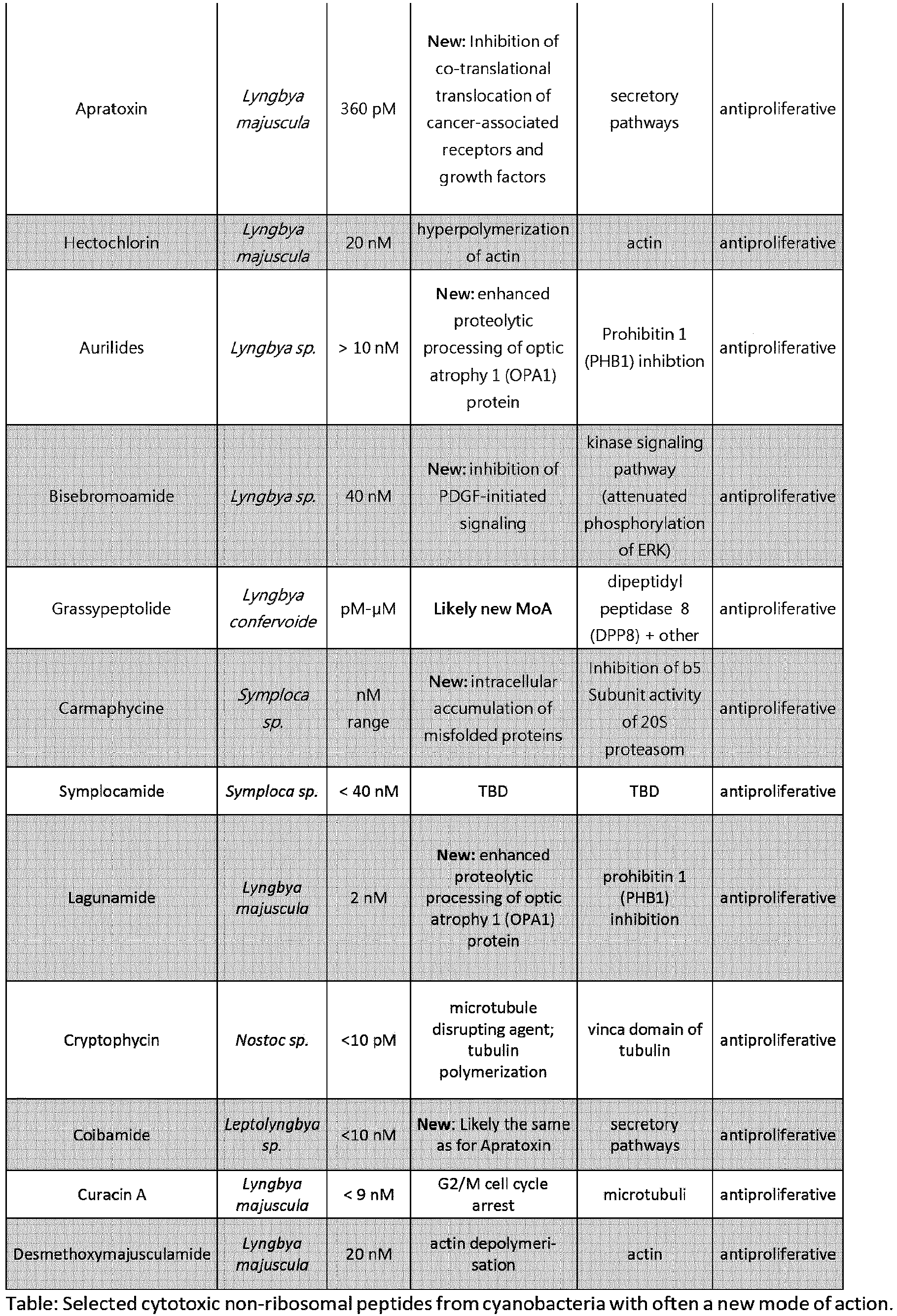

Herein, a CA producing cyanobacterial strain is referred to as a **CA-STRAIN.**

Herein, anabaenopeptin and oscillamide are cyclic peptides that are characterized by a lysine in position 5 and the formation of the ring by an N-6-peptide bond between Lys and the carboxy group of the amino acid in position 6. A side chain of one amino acid unit is attached to the ring by an ureido bond formed between the a-N of Lys and the a-N of the side chain amino acid. All other positions in the ring and side chain are variable.

Herein **targeting moieties** are proteins (mainly antibodies and their fragments), peptides, nucleic acids (aptamers), small molecules, or others (vitamins or carbohydrates) as well as nano particles. Monoclonal antibodies (mAbs) are preferred as escort molecules for the targeted delivery of the altered and modified non-ribosomal peptides incl. altered and modified microcystins or nodularins. However, small molecules can also act as targeting moieties as they might influence the physicochemical properties of said peptides. One example for this is the coupling with hydrophilic moieties such as sugars, e.g. to increase the solubility of said peptide in water. Furthermore, the attached small molecule can have the purpose of altering the peptides in vivo pharmacokinetic properties, e.g. attachment of a functional group prone to in vivo metabolism can increase hepatic clearance and reduce hepatic toxicity, or can influence transporter selectivity and therefore the (active) uptake of the modified non-ribosomal peptide by cells.

Herein an ADC (ADC for antibody-drug conjugate) is a CA linked to a targeting moiety (TM) directly or via a linker (L) whereas by definition of an ADC the targeting moiety is an antibody

The term antibody (AB) herein is used in the broadest sense and specifically covers monoclonal antibodies, polyclonal antibodies, dimers, multimers, multispecic antibodies (e.g., bispecific antibodies), and antibody fragments, so long as they exhibit the desired biological activity. Antibodies may be murine, human, humanized, chimeric, or derived from other species. An antibody is a protein generated by the immune system that is capable of recognizing and binding to a specific antigen. A target antigen generally has numerous binding sites, also called epitopes, recognized by complementarity-determining regions (CDRs) on multiple antibodies. Each antibody that specifically binds to a different epitope has a different structure. Thus, one antigen may have more than one corresponding antibody. An antibody includes a full-length immunoglobulin molecule or an immunologically active portion of a full-length immunoglobulin molecule, i.e., a molecule that contains an antigen binding site that immuno-specifically binds an antigen of a target of interest or part thereof, such targets including but not limited to, cancer cells, microbial cells or cells that produce autoimmune antibodies associated with an autoimmune disease. The immuno globulin can be of any type (e.g., IgG, IgE, IgM, IgD, and IgA), class (e.g., IgGI, IgG2, IgG3, IgG4, IgA1 and IgA2) or subclass of immunoglobulin molecule. The immunoglobulins can be derived from any species, including human, murine, or rabbit origin.

**Antibody fragments** (AB fragments) comprise a portion of a full length antibody, generally the antigen binding or variable region thereof. Examples of antibody fragments include Fab, Fab', F(ab')2, and Fv fragments; diabodies; linear antibodies; fragments produced by a Fab expression library, anti-idiotypic (anti-Id) antibodies, CDR (complementary determining region), and epitope-binding fragments of any of the above which immuno-specifically bind to cancer cell antigens, viral antigens or microbial antigens, single-chain antibody molecules; multi-specific antibodies formed from antibody fragments.

The **linker,** attaches the antibody or AB fragment or targeting moiety or label to the CA through covalent bond(s). The linker is a bifunctional or multifunctional moiety which can be used to link one or more drug moiety (D whereas D = CA) and an antibody unit (Ab) to form antibody-drug conjugates (ADC). The linker (L) may be stable outside a cell, i.e. extracellular, or it may be cleavable by enzymatic activity, hydrolysis, or other metabolic conditions. Antibody-drug conjugates (ADC) can be conveniently prepared using a linker having reactive functionality for binding to the drug moiety (here the CA) and to the antibody. Herein, the ADC is a CA linked to a targeting moiety. A linker can also include a spacer that might be of advantage to obtain favorable spacial distances between the linker, drug and targeting moieties.
A cysteine thiol, an amine, e.g. N-terminus or amino acid side chain such as lysine, or any other modification of the antibody (AB), as described below, can form a bond with a functional group of a linker reagent, drug moiety (D) or drug-linker reagent (D-L). The linkers are preferably stable extracellularly. Before transport or delivery into a cell, the antibody-drug conjugate (ADC) is preferably stable and remains intact, i.e. the antibody remains linked to the drug moiety. The linkers are stable outside the target cell and may be cleaved at some rate inside the cell. An effective linker will: (i) maintain the specific binding properties of the antibody; (ii) allow intracellular delivery of the conjugate or drug moiety; (iii) remain stable and intact, i.e. not cleaved, until the conjugate has been delivered or transported to its targeted site; and (iv) maintain a cytotoxic, cell-killing effect or a cytostatic effect of the CA. Stability of the ADC may be measured by standard analytical techniques such as mass spectroscopy, HPLC, and the separation/analysis technique LC/ MS. Covalent attachment of the antibody and the CA requires the linker to have two reactive functional groups, i.e. bivalency in a reactive sense. Bivalent linker reagents which are useful to attach two or more functional or biologically active moieties, such as peptides, nucleic acids, drugs, toxins, antibodies, haptens, and reporter groups are known.

In another embodiment, the linker may be substituted with a sulfonate substituent or other substituents which may increase water solubility of the reagent and facilitate the coupling reaction of the linker reagent with the antibody or the CA, or facilitate the coupling reaction of AB-L with D, or D-L with AB, depending on the synthetic route employed to prepare the ADC. Nucleophilic groups on antibodies include, but are not limited to: (i) N-terminal amine groups, (ii) side chain amine groups, e.g. lysine, (iii) side chain thiol groups, e.g. cysteine, and (iv) sugar hydroxyl or amino groups where the antibody is glycosylated. Amine, thiol, and hydroxyl groups are nucleophilic and capable of reacting to form covalent bonds with electrophilic groups on linker moieties, linker reagents and CA (=D) including: (i) active esters such as NHS esters, HOBt esters, haloformates, and acid halides; (ii) alkyl and benzyl halides such as haloacetamides; (iii) aldehydes, ketones, carboxyl, and maleimide groups. Certain antibodies have reducible interchain disulfides, i.e. cysteine bridges. Antibodies may be made reactive for conjugation with linker reagents by treatment with a reducing agent such as DTT (dithiothreitol). Each cysteine bridge will thus form, theoretically, two reactive thiol nucleophiles. Additional nucleophilic groups can be introduced into antibodies through the reaction of lysines with 2-iminothiolane (Traut's reagent) resulting in conversion of an amine into a thiol. Reactive thiol groups may be introduced into the antibody (or fragment thereof) by introducing one, two, three, four, or more cysteine residues (e.g., preparing mutant antibodies comprising one or more non native cysteine amino acid residues). US 2007/0092940 discloses cysteine engineered antibodies by introduction of reactive cysteine amino acids.

**Modified substrate** means any amino acid and any related compound carrying at least one amino group and one carboxyl group that enable peptide bound formation of the modified substrate in a respective non-ribosomal peptide and which is naturally not incorporated into the non-ribosomal peptides synthesized by a specific cyanobacterial strain.

A **modified** amino acid or modified substrate may comprise an amino acid linker component including those occurring naturally, as well as minor amino acids and non-naturally occurring amino acid analogs, such as citrulline. Amino acid linker components can be designed and optimized in their selectivity for enzymatic cleavage by a particular enzymes, for example, a tumor-associated protease, cathepsin B, C and D, or a plasmin protease. Amino acid side chains include those occurring naturally, as well as minor amino acids and non-naturally occurring amino acid analogs, such as citrulline.

### DETAILED DESCRIPTION OF THE INVENTION

The invention relates to a method of producing a modified non-ribosomal peptide, especially cytotoxic non-ribosomal peptides such as modified microcystin and/or modified nodularin (both CA), comprising the steps of:
a) growing a microcystin and/or nodularin producing cyanobacteria strain (CA-STRAIN) in a culture media,
b) adding one or more modified substrates preferably modified amino acids to said culture, and
c) cultivation the strain in the presence of said modified substrates.

The invention relates to a method of producing a modified non-ribosomal peptide from cyanobacteria, comprising the steps of:
a) growing a non-ribosomal peptide producing cyanobacteria strain in culture media,
b) adding one or more modified substrates to said culture, and
c) growing the strain in the presence of said modified substrates,
d) wherein the modified substrate is a modified amino acid, which comprises an anchor group directly accessible or transformable for use in conjugation chemistry incl. click chemistry, for the attachment of a targeting moiety or a label, or a linker or for any other structural modification

The modified substrate carries an anchor group directly accessible or transformable for use in conjugation chemistry incl. click chemistry, for the attachment of a targeting moiety or a label or a linker or for any other structural modification of the non-ribosomal peptide. This will allow for example connecting antibodies to the CA.

The following figure A illustrate the general principle of the invention using the example of producing a modified microcystin YR (MC-YR) by feeding either the modified substrate 4-azido-L-phenylalanine carrying a clickable azido group as anchor group (left side of the figure) or by feeding the modified substrate O-propargyl-L-tyrosine carrying the clickable propargyl group (also known as alkyne group) as anchor group (left side of the figure). Both substrates with their respective clickable anchor groups lead to replacement of the tyrosine at position 2 (see arrow in the upper chemical structure). In case of feeding of 4-azido-L-phenylalanine the resulting modified microcystin is MC-4-azido-FR (F L-phenylalanine; R for arginine). In case of feeding of O-propargyl-L-tyrosine the resulting modified microcystin is MC-*O*-propargyl-YR (Y for tyrosine; R for arginine). The generation of both modified microcystins can be detected on the basis of their molecular mass by using mass spectrometry (MS).

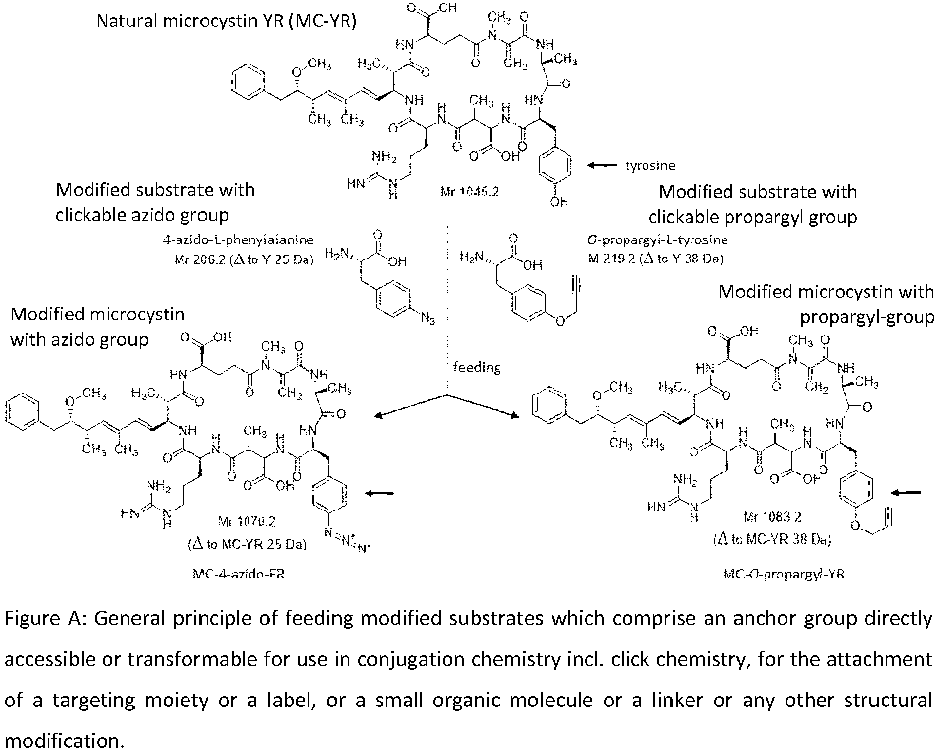

The two anchor groups (the azido group and the propargyl group, also known as alkyne group) of the two modified microcystins described above can be directly used for conjugation chemistry, more specific for click chemistry. Hereby the respective click reaction is based on the reaction between these two groups with each other. That means an azido group reacts with a propargyl group (alkyne group) forming a triazole conjugate as shown in the figure B below. Therefore both groups the azido group and the propargyl (alkyne) group can be used as anchor groups of modified substrates.

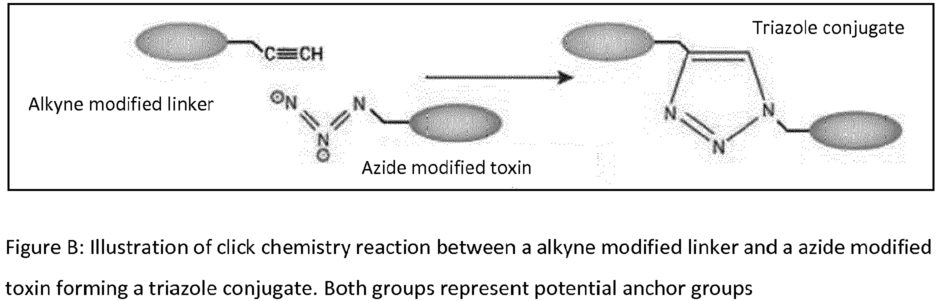

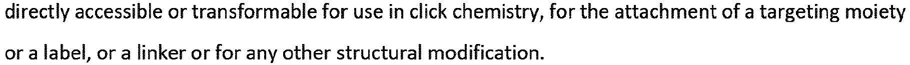

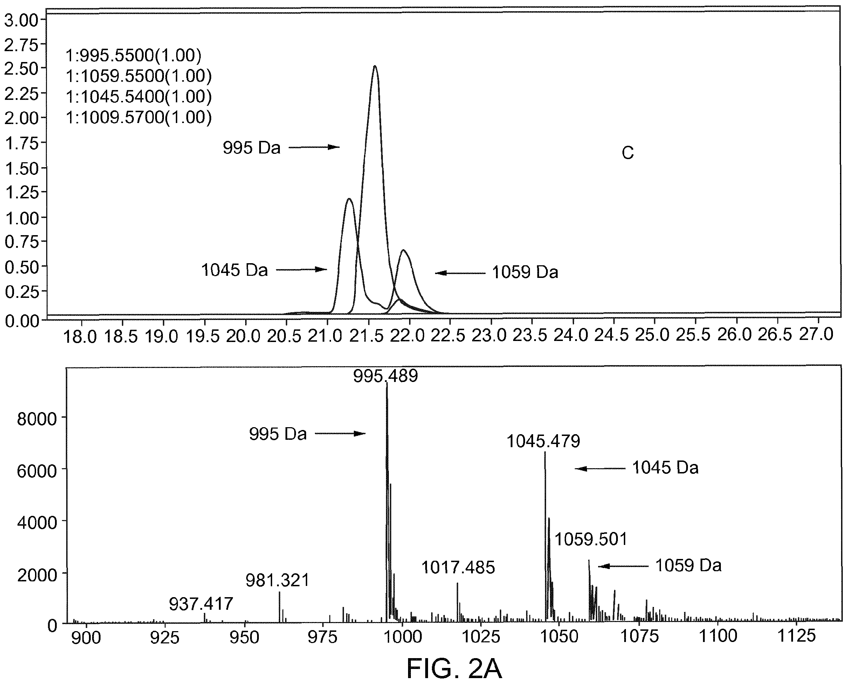

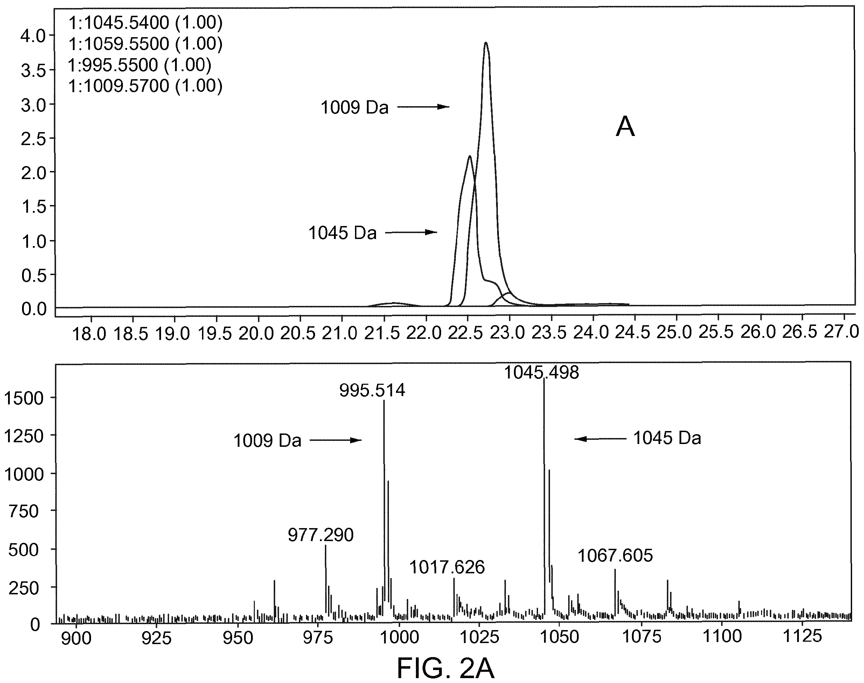

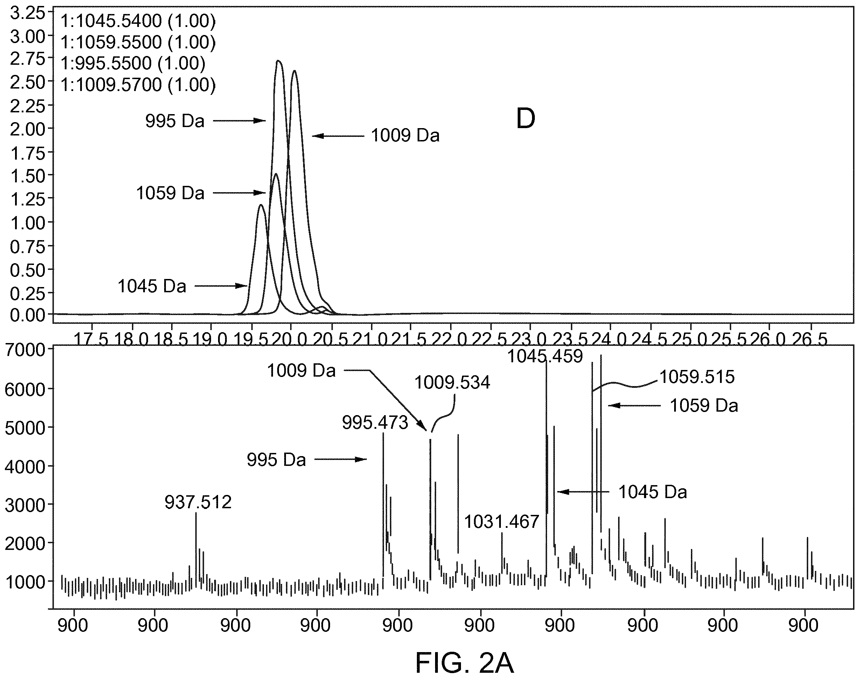

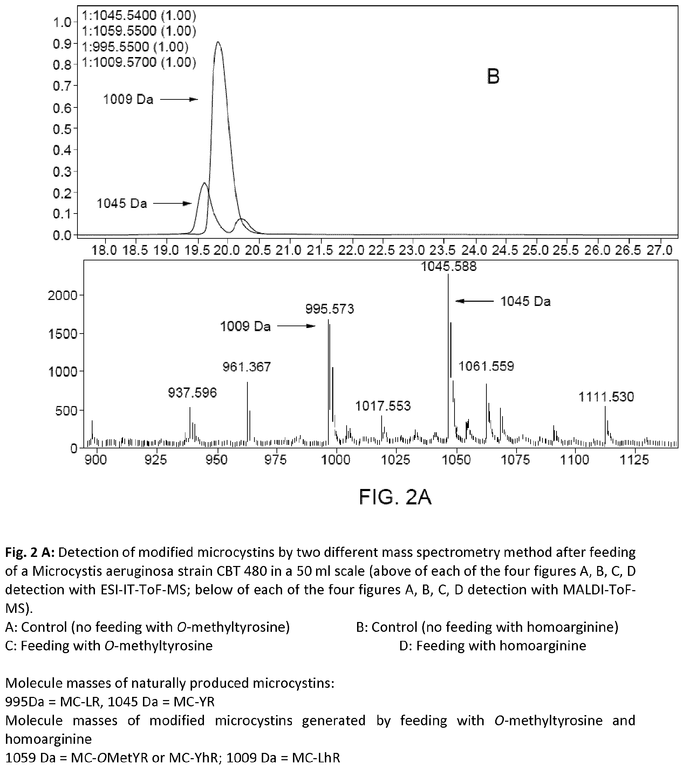

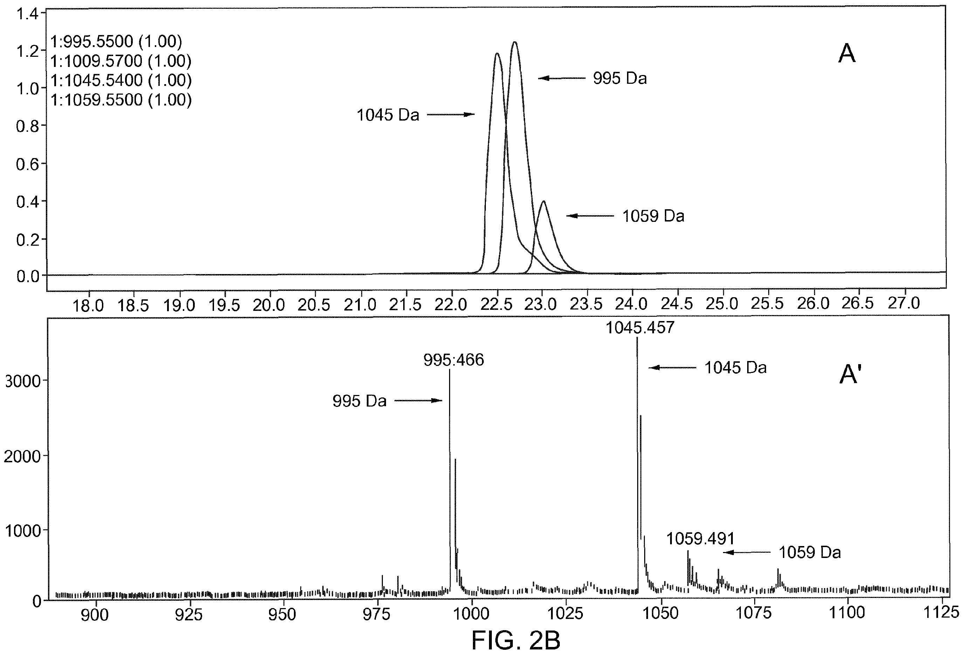

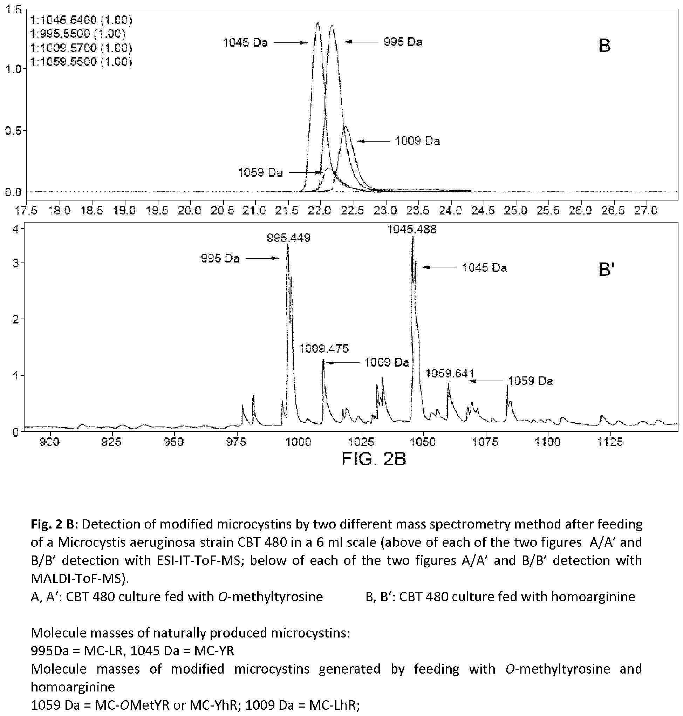

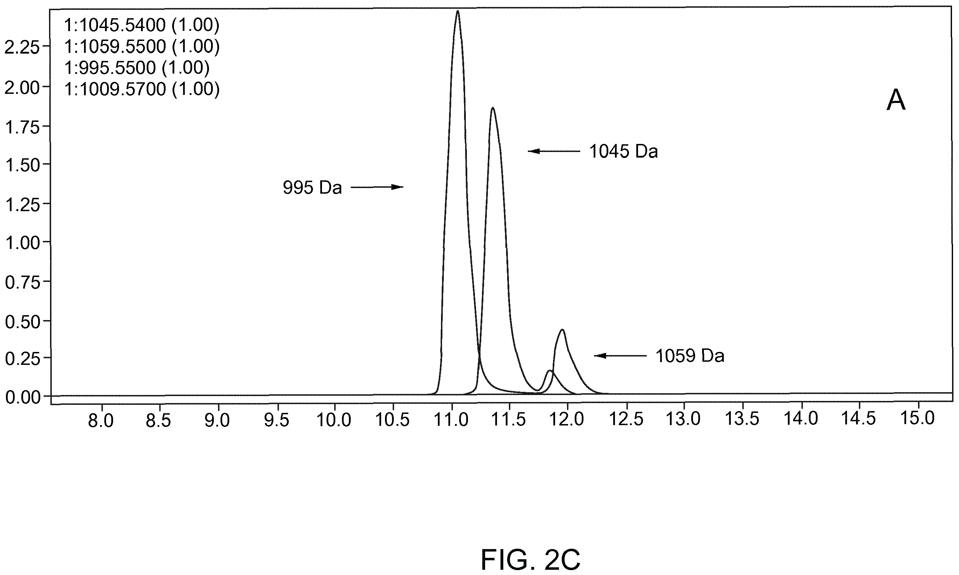

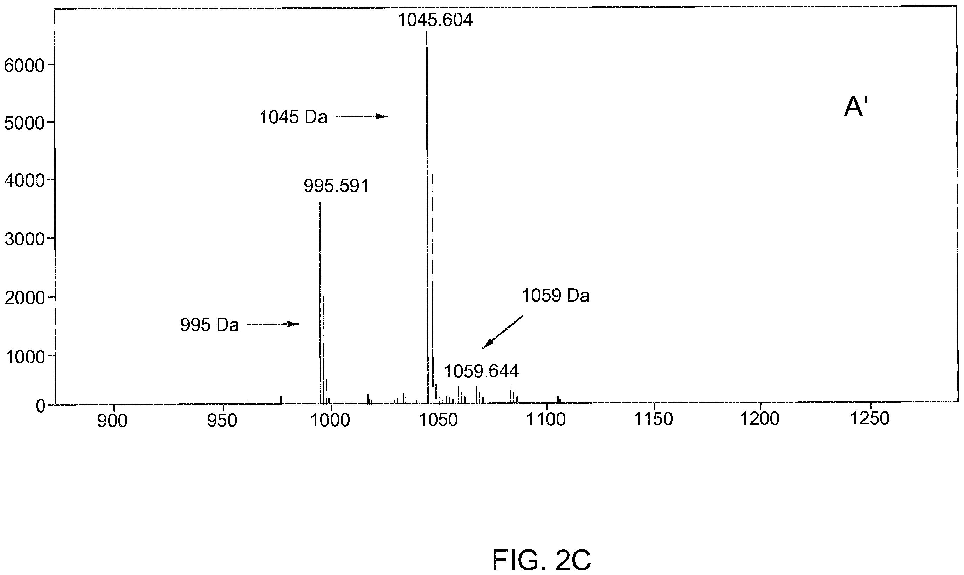

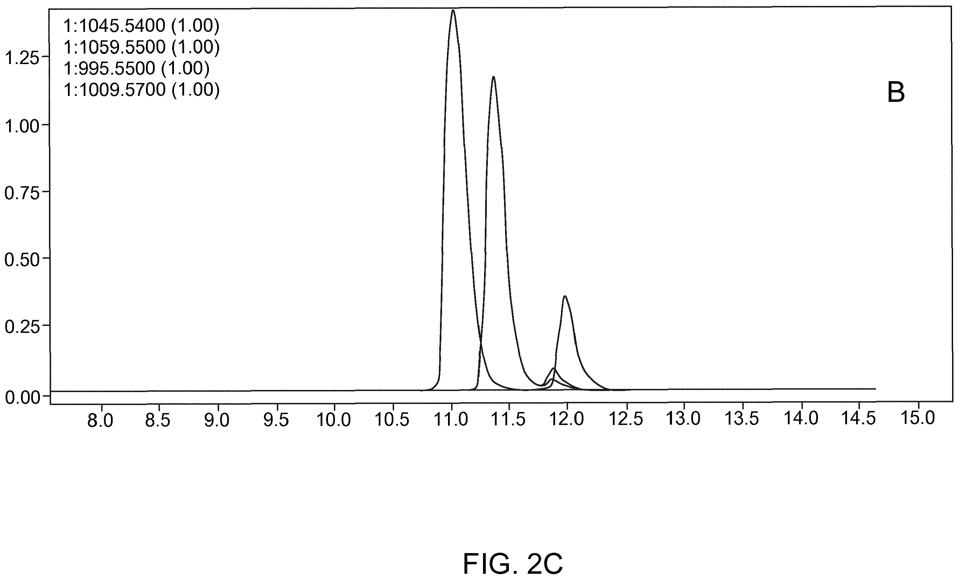

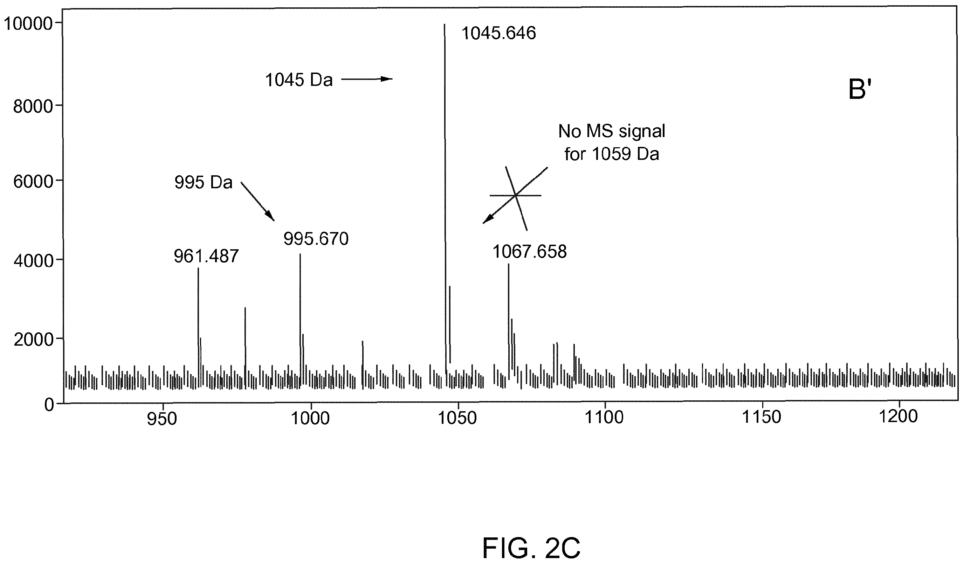

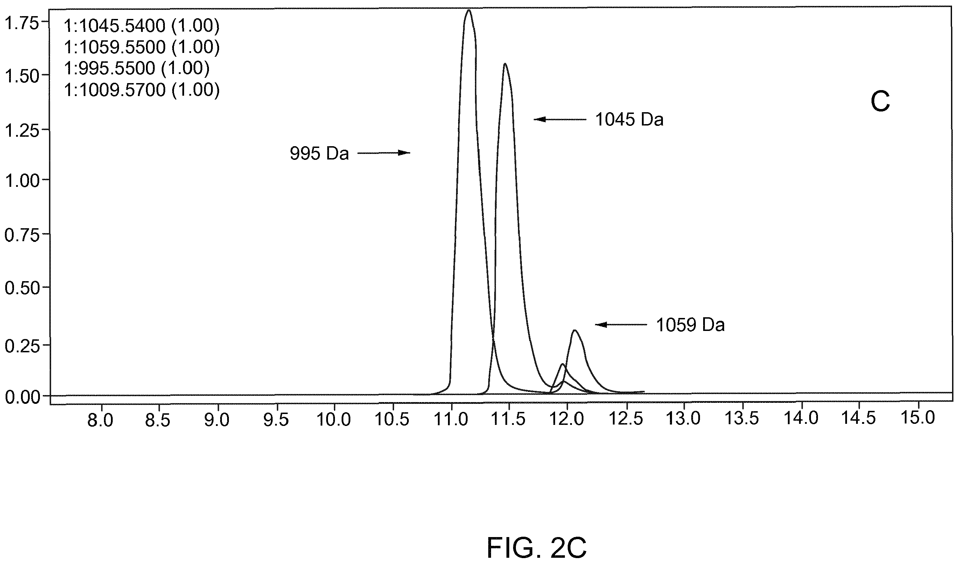

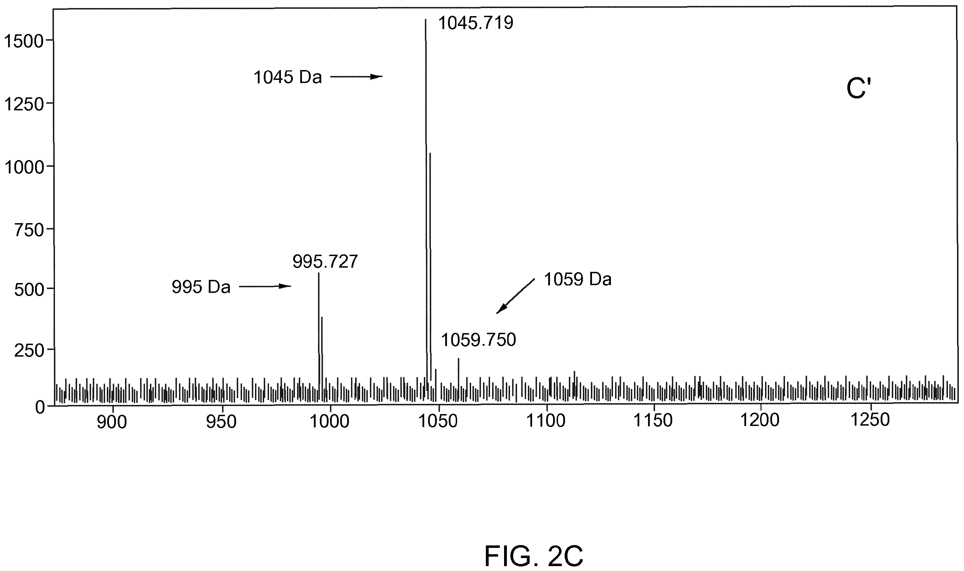

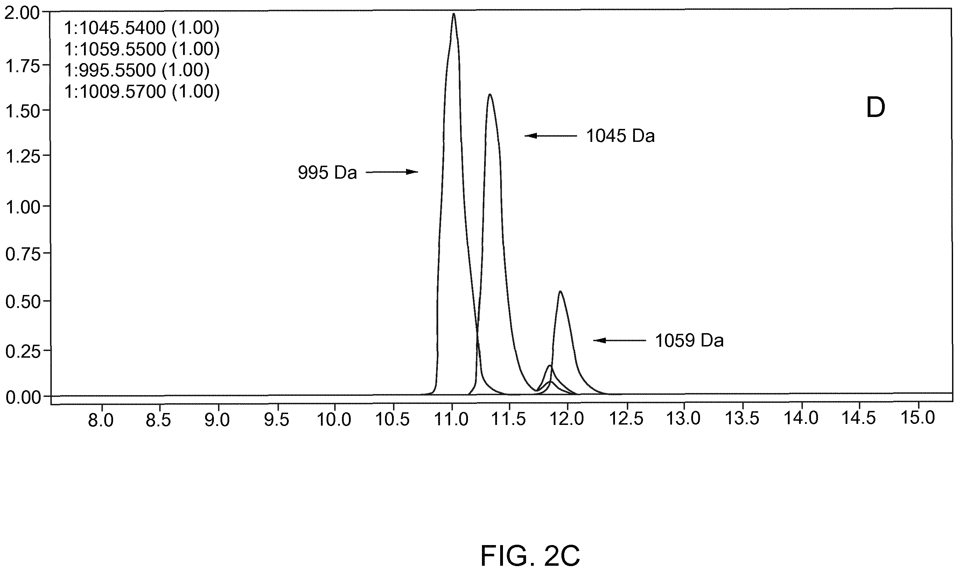

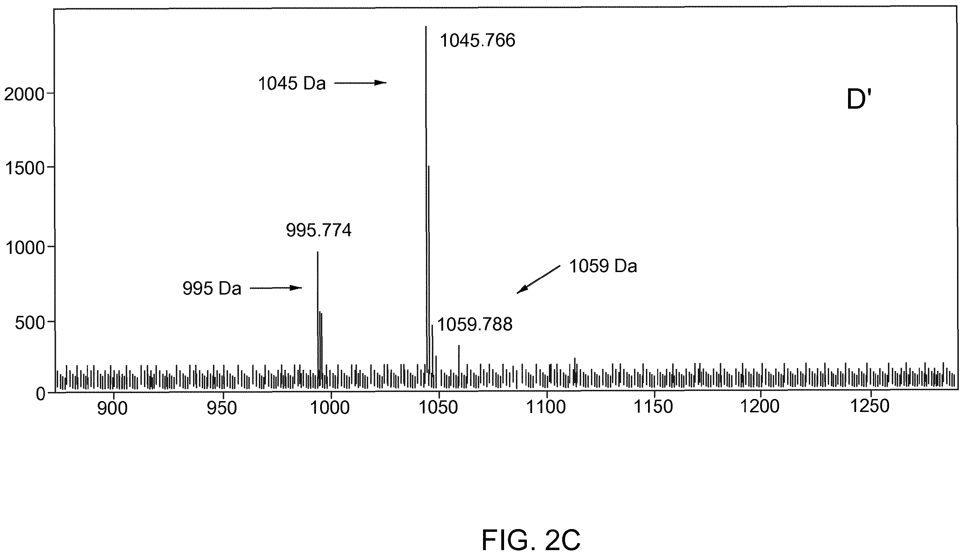

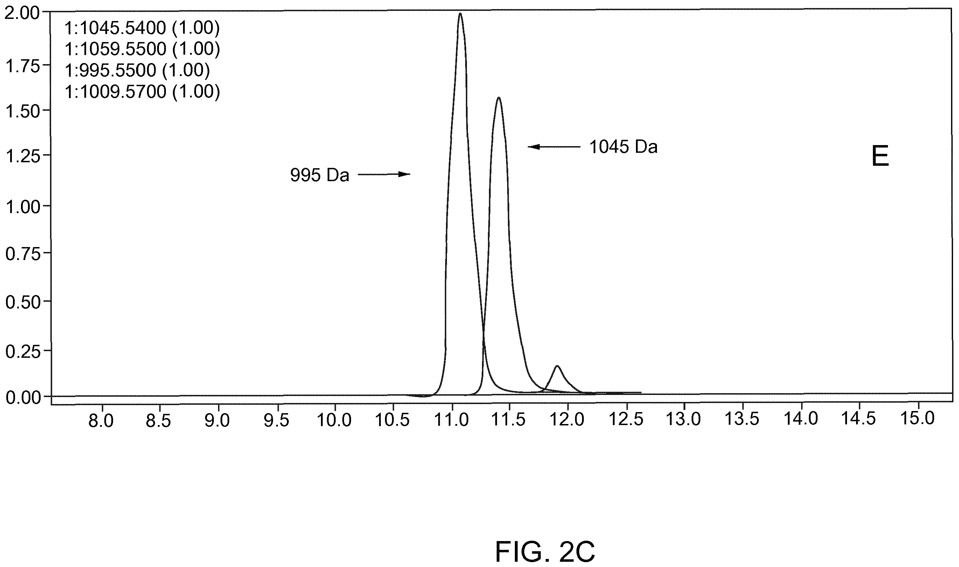

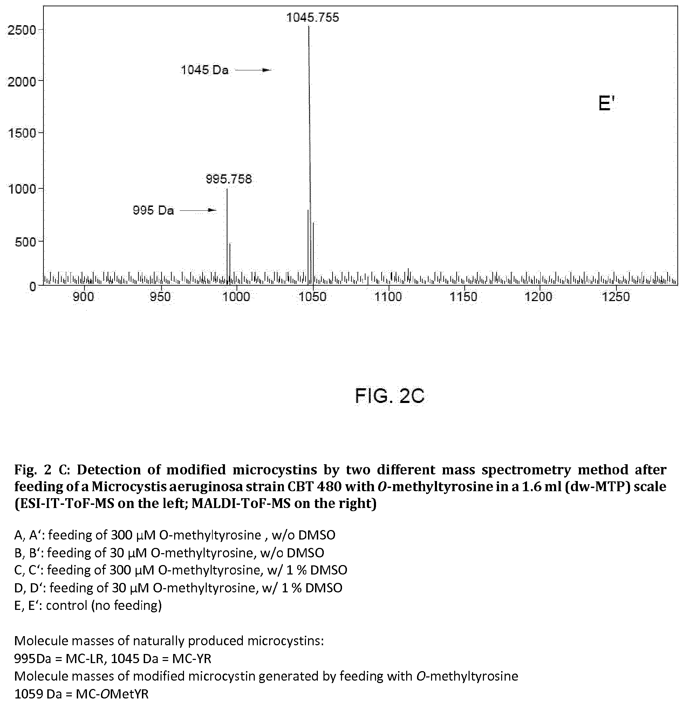

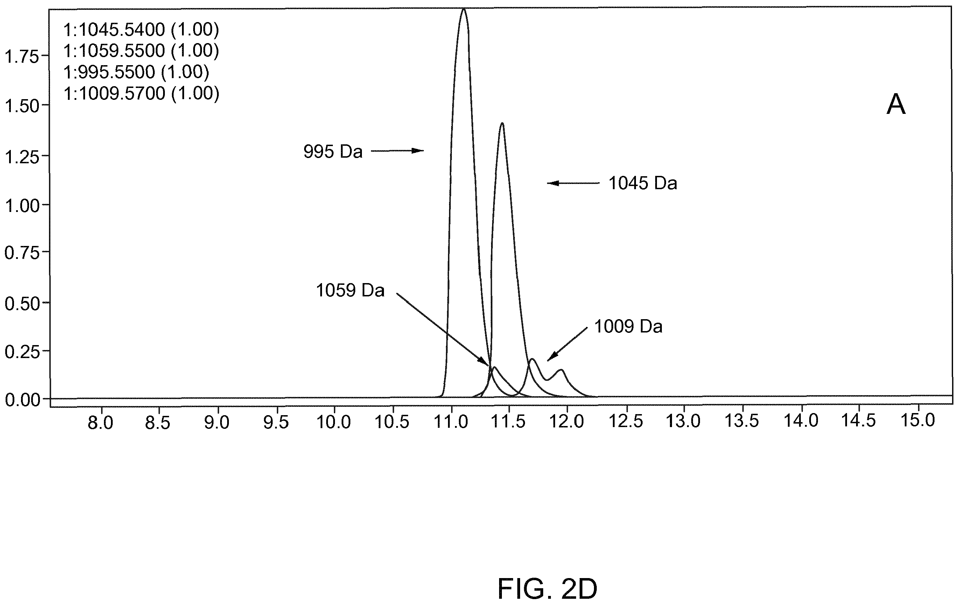

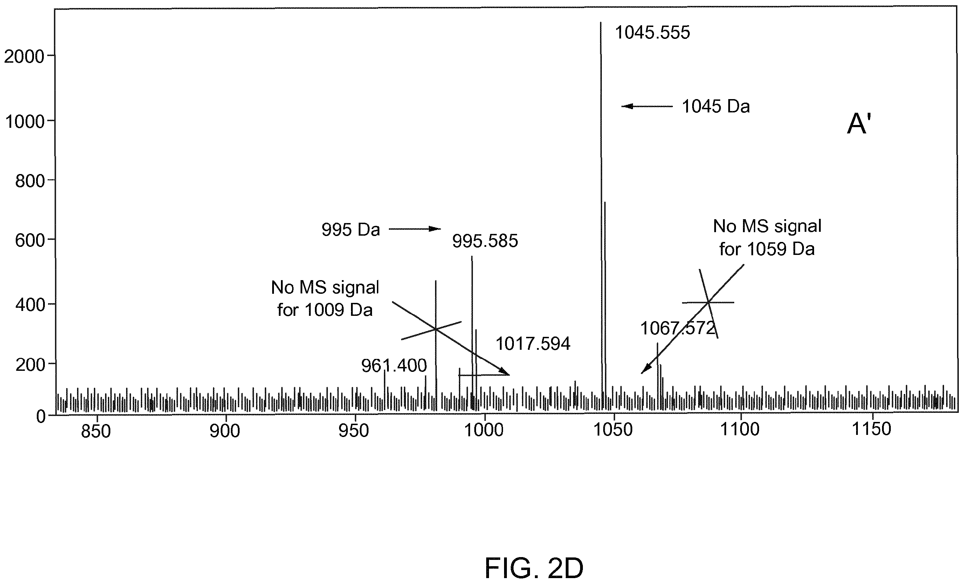

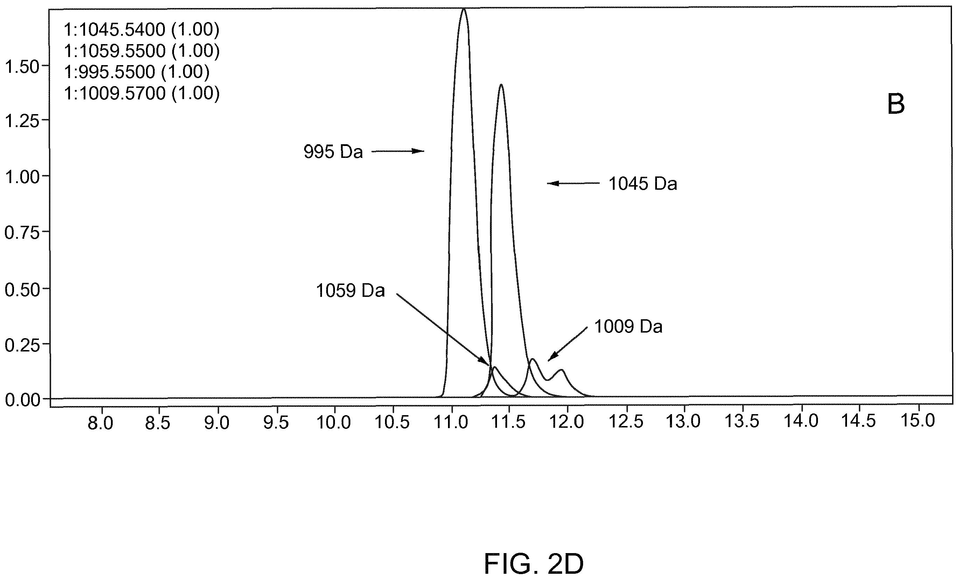

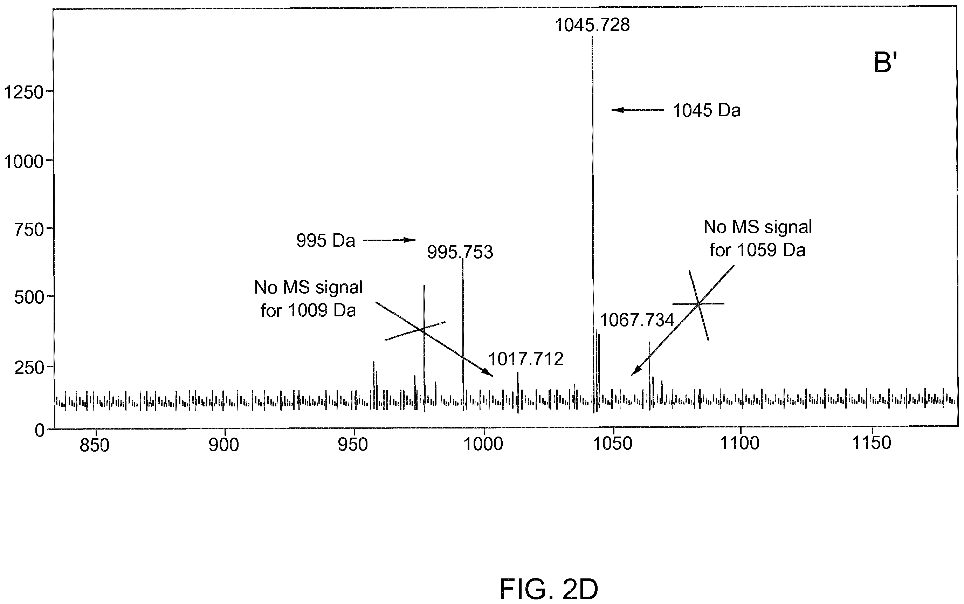

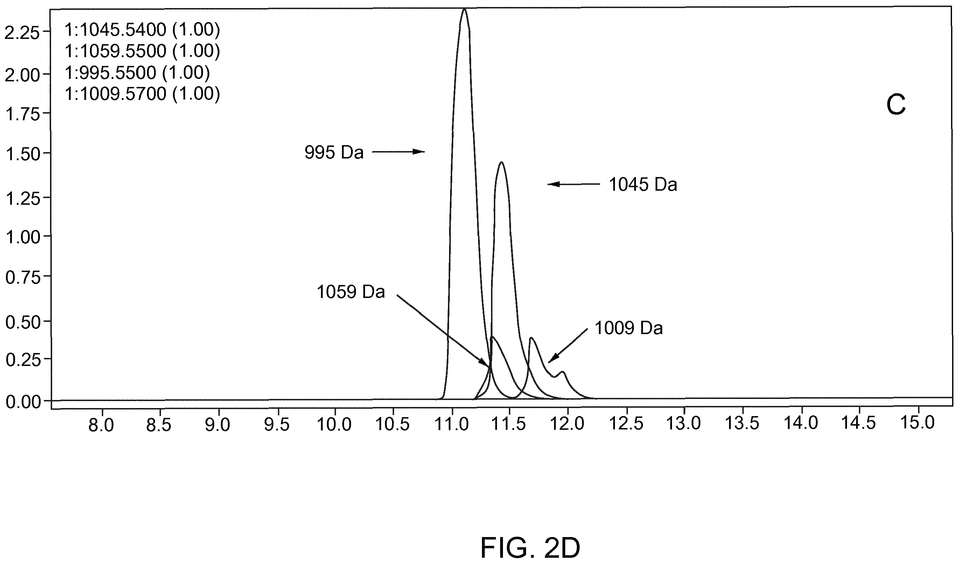

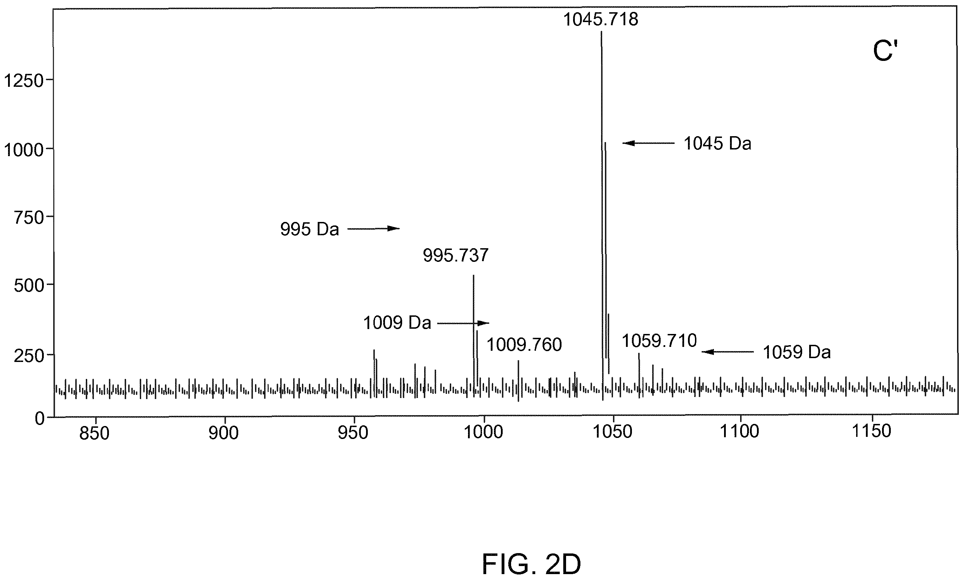

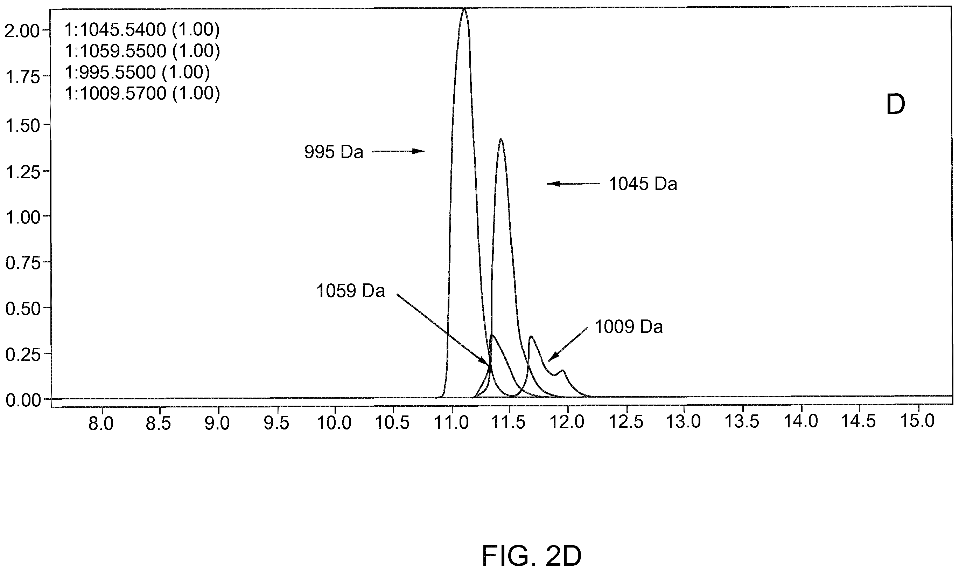

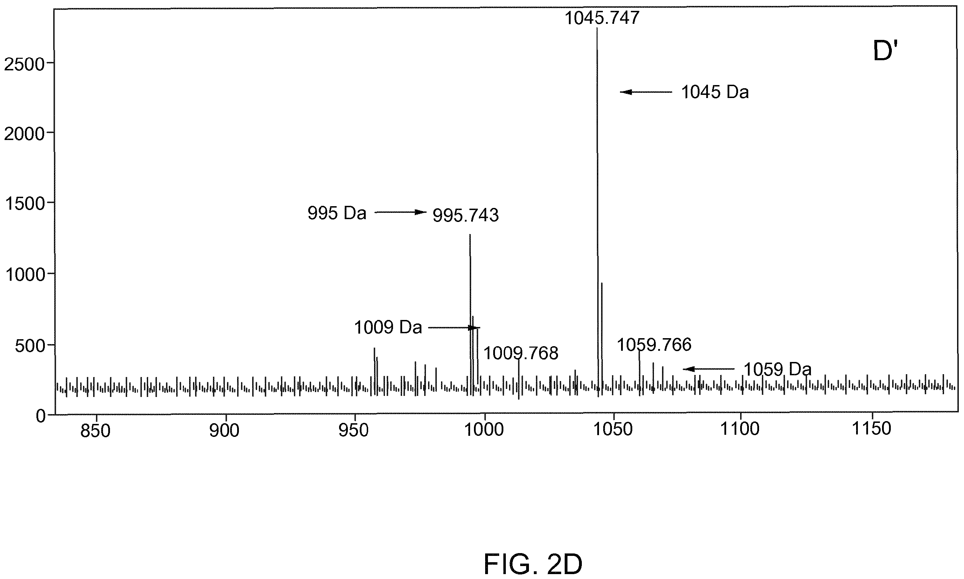

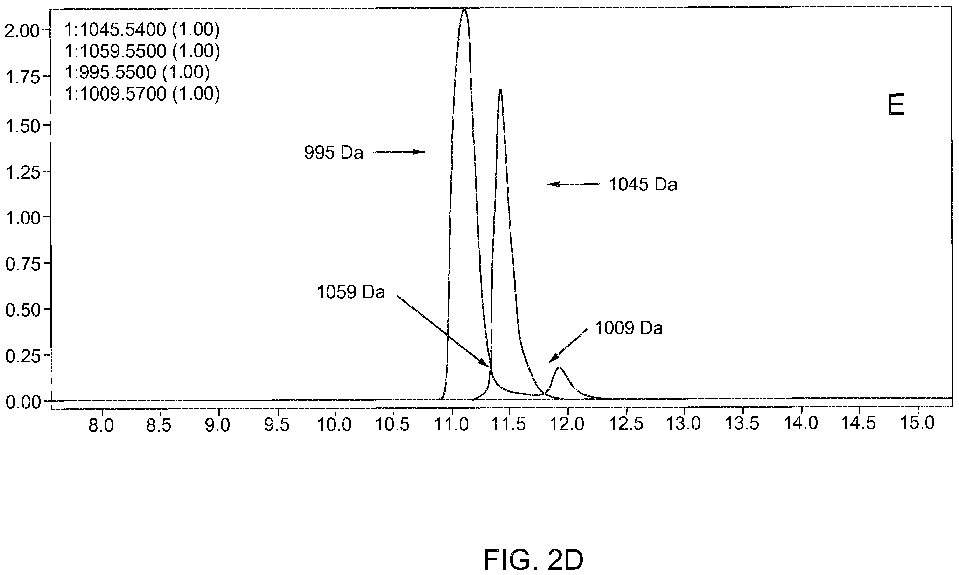

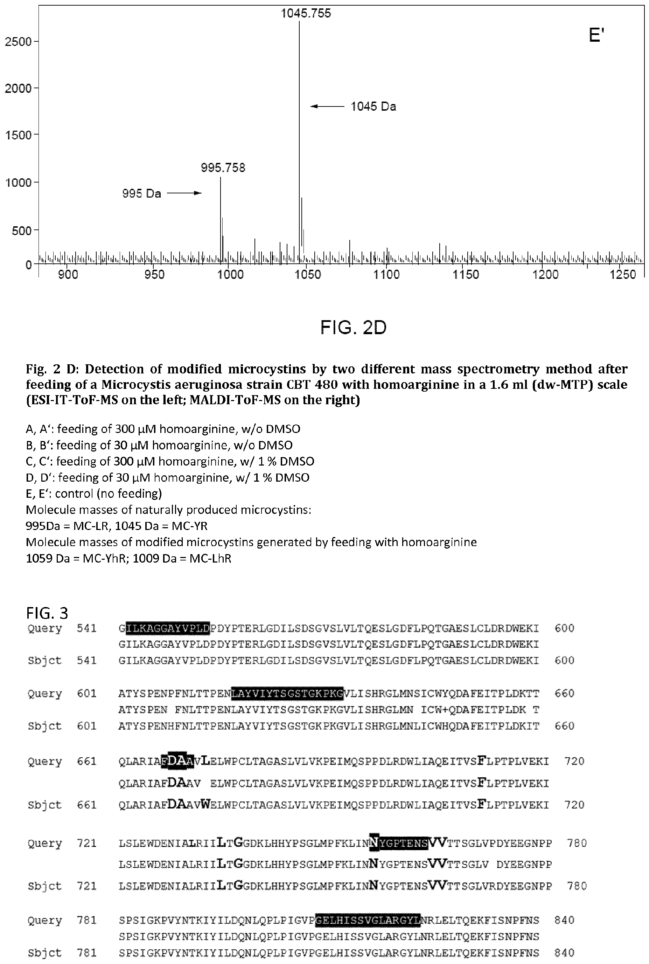

The selection of a suitable strain for the feeding of modified substrates needs to be identified by screening (feeding experiments with a high number of diverse modified substrates incl. the use and variation of strain-specific cultivation conditions for a high number of strains). Such screening is preferably done in small scale cultures (e.g. in 1.6 ml to 10 ml scale) in order to assure throughput and efficiency. In addition the detection of modified non-ribosomal peptides is preferably done by mass spectrometry (MS) whereas the MS method is preferably suited for analyses of small scale cultures without the need of extensive extractions and sample preparations, e.g. MALDI-ToF-MS (see figure 2).

In the context of the establishment of a screening for strains that can be fed with modified substrates for the generation of novel non-ribosomal peptides the inventors found that feeding of O-methyl-tyrosine and homo-arginine at the same time to a strain producing MC-YR and MC-LR (Y-for tyrosine; R-for arginine; L for leucine) resulted in the incorporation of O-methyl-tyrosine instead of tyrosine and the incorporation of homo-arginine instead of arginine. Consequently, by feeding of these two modified amino acids the fed strain additionally produced MC-Y-homo-R, MC-O-methyl-YR, MC-O-methyl-Y-homo-R, and MC-L-homo-R (see figure 2).

Furthermore the selection of suitable substrates for feedings is ideally done based on non-ribosomal peptides naturally produced by a specific strain, e.g. naturally produced microcystins and nodularins. Hereby not only substrates which are directly derived from the native substrates can be selected.

The fact that strains might produce several structural variants with different amino acids at a specific position of the non-ribosomal peptide significantly increases the number of suitable substrates. This counts even more if a strain naturally produces variants with structurally distant amino acids at a specific position, e.g. the Microcystis strain CBT 480 primarily produces MC-LR and MC-YR as major microcystins in comparable amounts (besides further structural variants produced in minor amounts), although the hydrophobic aliphatic amino acid leucine (L) is structurally rather distant from the aromatic amino acid tyrosine (Y).

Interestingly, by genome sequencing of said strain Microcystis CBT 480 the inventors found that despite the fact that the two major microcystin variants MC-LR and MC-YR are synthesized by this strain, there is only one microcystin synthetase gene cluster encoded in the genome of said Microcystis strain CBT480. Subsequent DNA sequence comparison with another microcystin synthetase gene cluster from Microcystis strain CBT265 (also PCC7806) which primarily produces MC-LR did not reveal significant sequence differences which might explain the differences in the abundance of microcystin variants produced by both strains (see figure 3). This led the inventors to the conclusion that the feature of being a suited strain for the generation of modified non-ribosomal peptides by feeding of modified substrates is not solely connected to the multienzyme complex of the non-ribosomal peptide synthetase encoded by the respective gene cluster. It is rather a strain-specific feature which needs to be screened for in an approach of cultivation of a high number of strains using strain-specific conditions, feeding of a high number of modified substrates to the strains and analyzing of the resulting secondary metabolite spectra of fed strains (see figure 2) by mass spectrometry (MS).

Therefore the inventors conclude, that preferably, the strain is selected by cultivation/feeding/chemical analysis screening and the chemical structures of the produced non-ribosomal peptides are known such that the suited modified substrates can be selected and the incorporation of the modified substrate into the non-ribosomal peptide during cultivation occurs at a defined position.

Preferable cyanobacterial strains can be of a variety of suitable genera, including but not limited to genera of the group comprising Microcystis, Planktothrix, Oscillatoria, Nostoc, Anabaena, Aphanizomenon, Hapalosiphon, Nodularia, Lyngbya, Phormidium, Spirulina, Halospirulina, Arthrospira, Trichodesmium, Leptolyngbya, Plectonema, Myxosarcina, Pleurocapsa, Pseudanabaena, Geitlerinema, Euhalothece, Calothrix, Tolypothrix, Scytonema, Fischerella, Mastigocladus, Westiellopsis, Stigonema, Chlorogloeopsis, Cyanospira, Cylindrospermopsis, Cylindrospermum, Microchaete, Rivularia, Autosira, Trichonema, Trichodesmium, Symploca, Starria, Prochlorothrix, Microcoleus, Limnothrix, Crinalium, Borzia, Chroococcidiopsis, Cyanocystis, Dermocarpella, Staniera, Xenococcus, Chamaesiphon, Chroococcus, Cyanobacterium, Cyanobium, Cyanothece, Dactylococcopsis, Gloeobacter, Gloeocapsa, Gloeothece. The only pre-requisite for strains to be used by the here described method is the synthesis of a least one non-ribosomal peptide by the respective strain.

Preferably the non-ribosomal peptide(s) produced by the cyanobacterial strain can be a variety of CA, including but not limited to cytotoxic non-ribosomal peptides of the group comprising microcystins, nodularins, cryptophycins, largarzoles, apratoxins, hectochlorines, aurilides, bisebromoamides, grassypeptolides, carmaphycins, symplocamides, lagunamides, coibamides, desmethoxy-majusculamides, curacins or it can be a non-ribosomal peptide with another bioactivity including but not limited to bioactive non-ribosomal peptides of the group comprising aeruginosins (synonyms: microcin, spumigin), microginins (synonyms: cyanostatin, oscillaginin, nostoginin), anabaenopeptins (oscillamide, ferintoic acid, nodulapeptin, plectamide, schizopeptin), cyanopaptolins (synonyms: aeruginopeptin, anabaenopeptilide, dolostatin, hofmannoline, microcystillide, micropeptin, nostocyclin, nostopeptin, oscillapeptilide, oscillapeptin, planctopeptin,scyptolin, somamide, symplostatin, tasipeptin), cyclamides (synonyms: aanyascyclamide, dendroamide, microcyclamide, nostocyclamide, raocyclamide, tenuecycyclamide, ulongamide, westiellamide. The term in this list refers to names in original publications.

Preferably, the modified substrate is a modified amino acid. Preferably, the modified substrate allows for conjugation chemistry (incl. click chemistry).

The modified substrate allows for a broad diversity of functional groups being placed into the non-ribosomal peptide, e.g. the CA for coupling to the label or targeting moiety, etc.

Preferably, it allows for conjugation chemistry including click chemistry whereas conjugation chemistry is characterized as all chemical reactions that are able to join together two molecules with functional groups that can react with each other and whereas click chemistry is characterized as chemical reactions with a high thermodynamic driving force that drives it quickly and irreversibly to high yield of a single reaction product, with high reaction specificity (in some cases, with both regio- and stereospecificity) generating minimal and inoffensive byproducts. Click reactions are not disturbed by water. These qualities make click reactions (beside other, e.g. diagnostic applications) particularly suitable to the problem of an efficient and site-specific coupling of drug-like molecules at different positions of the drug and with different click chemistry to different positions of monoclonal antibodies via suited linker peptides or without linkers in order to create the ADC for the targeted treatment of diseases such as cancer, infection diseases, thrombosis and other diseases and disorders.

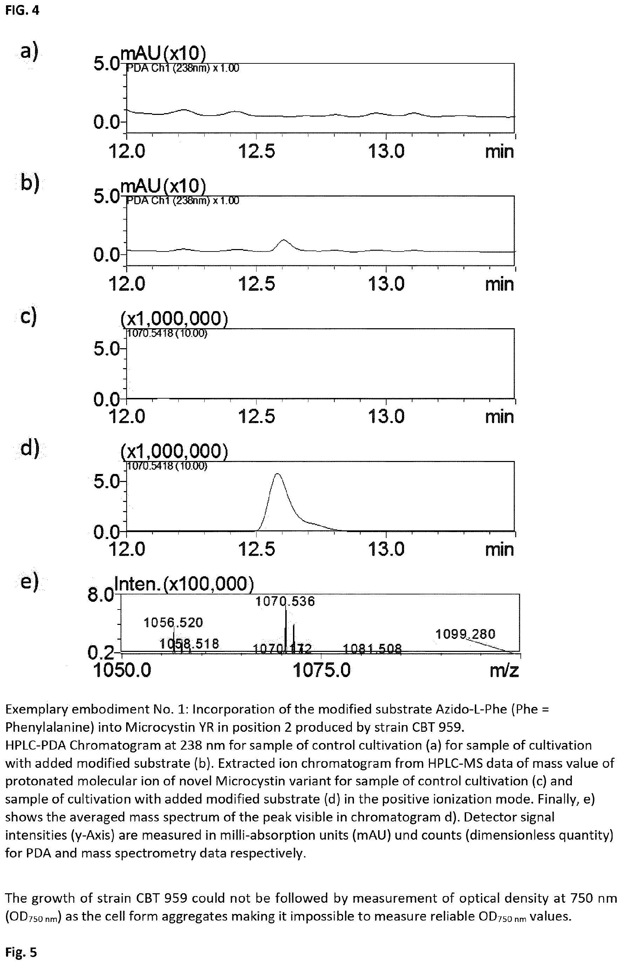

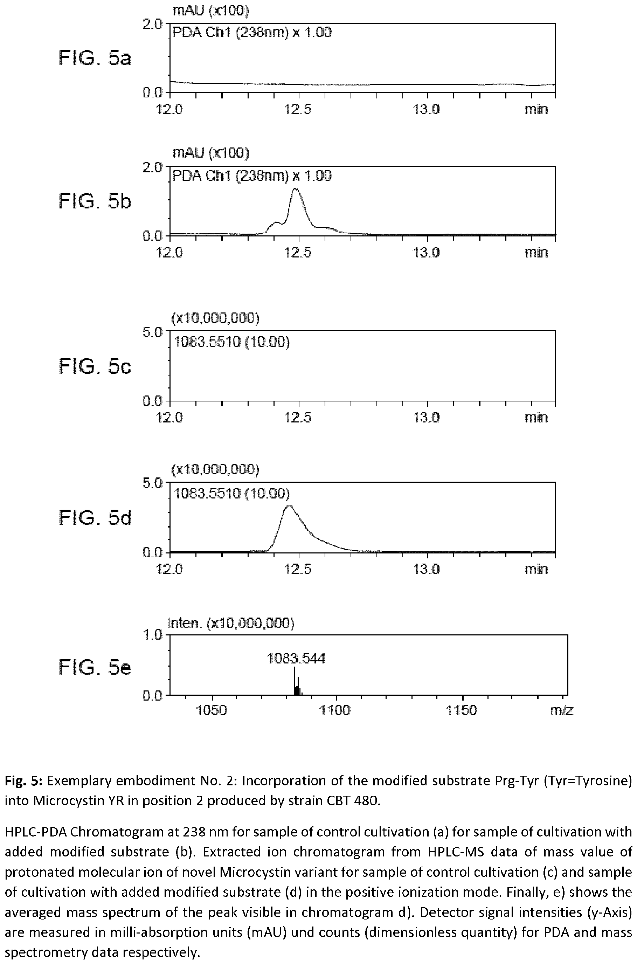

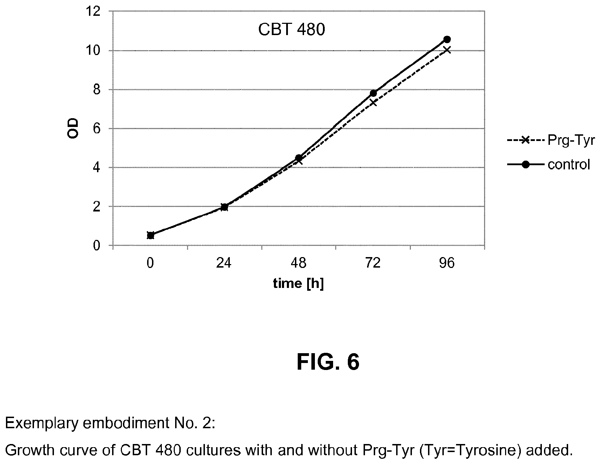

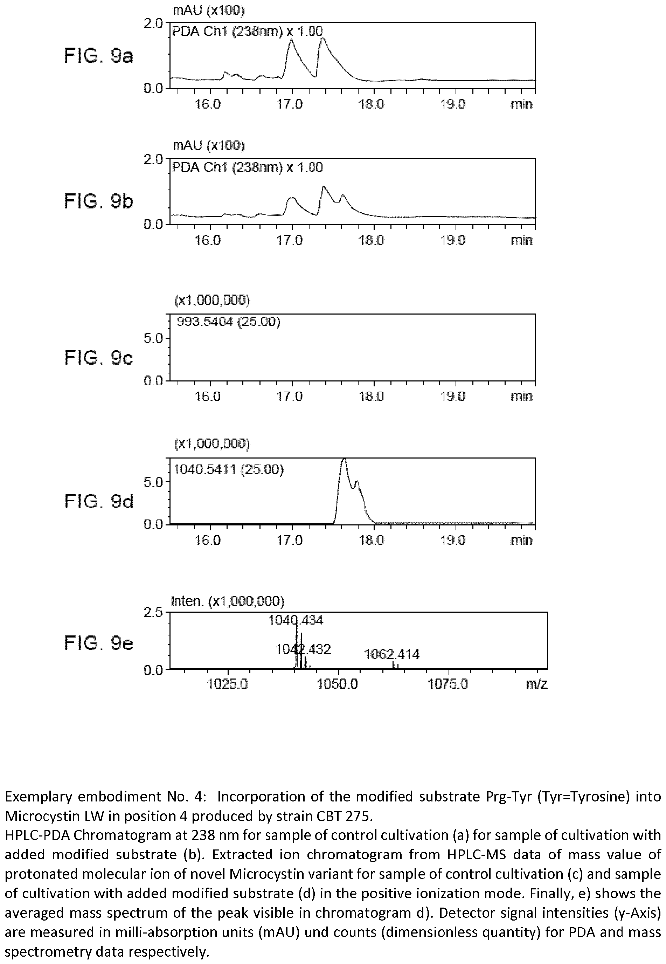

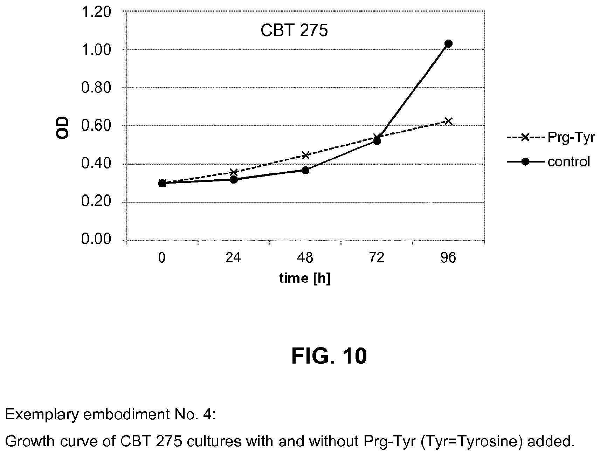

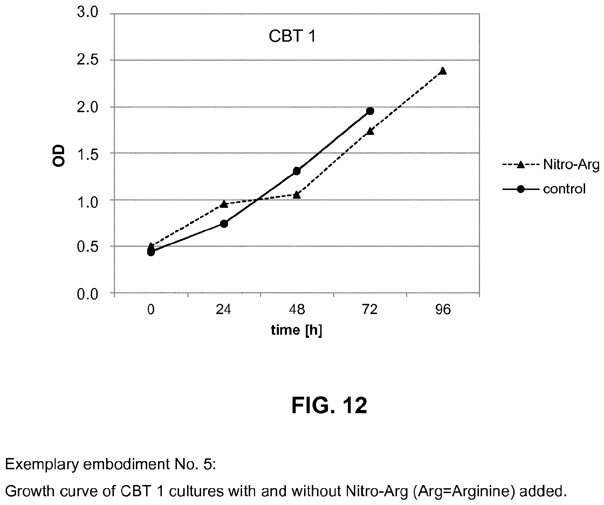

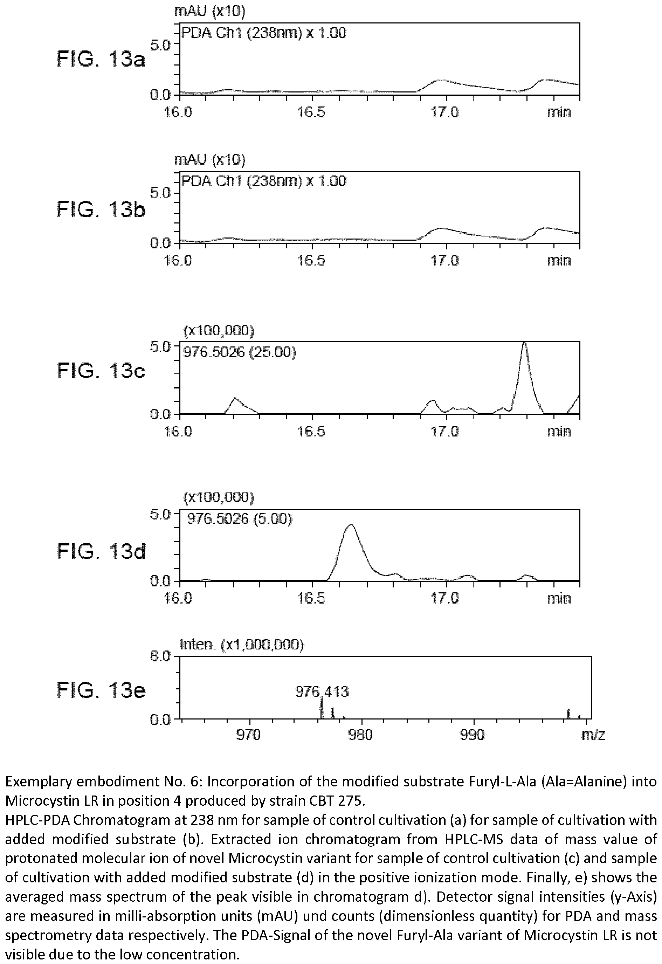

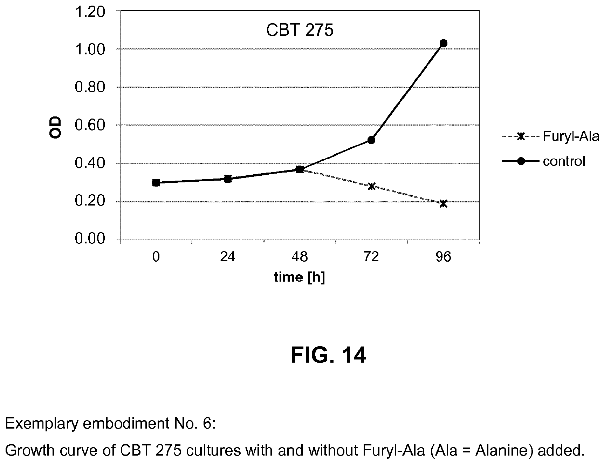

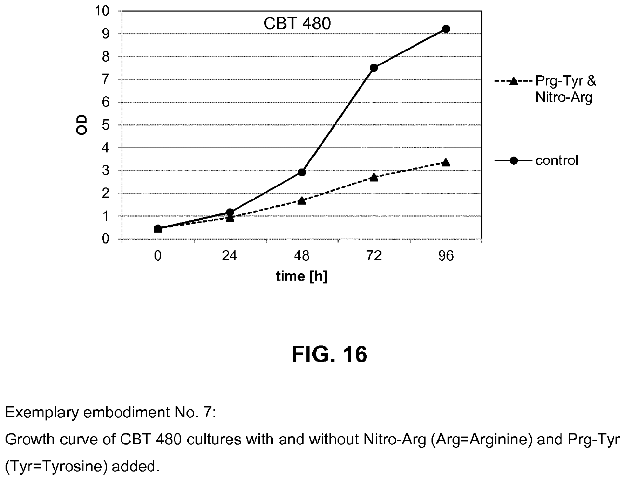

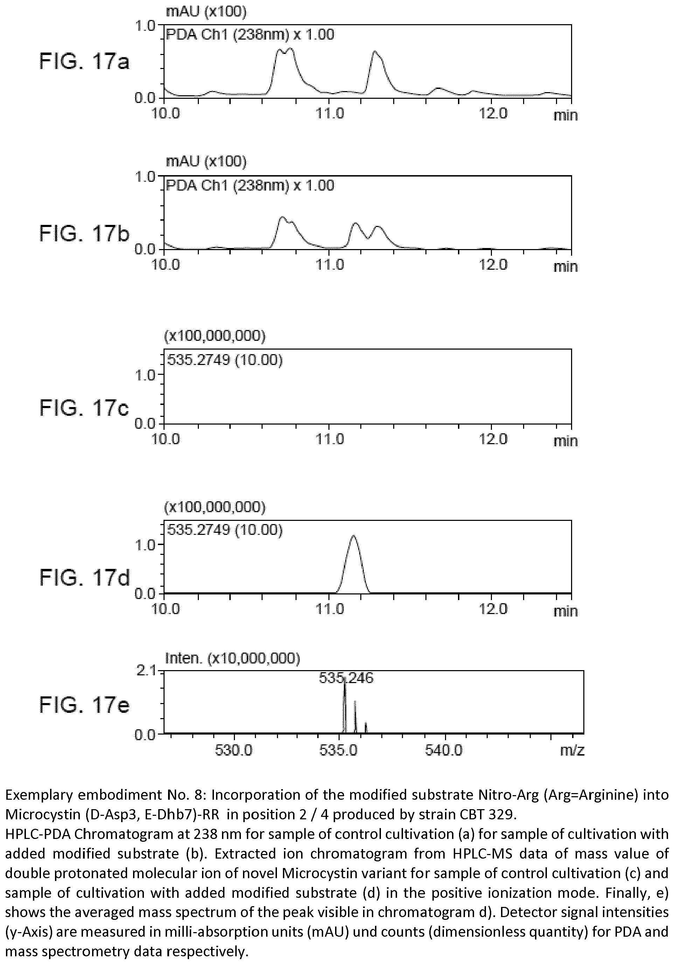

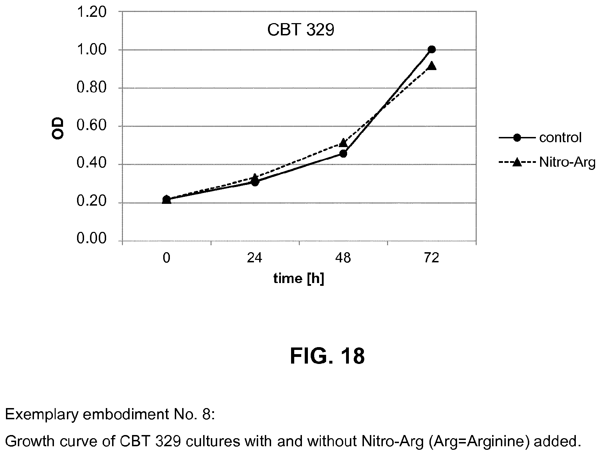

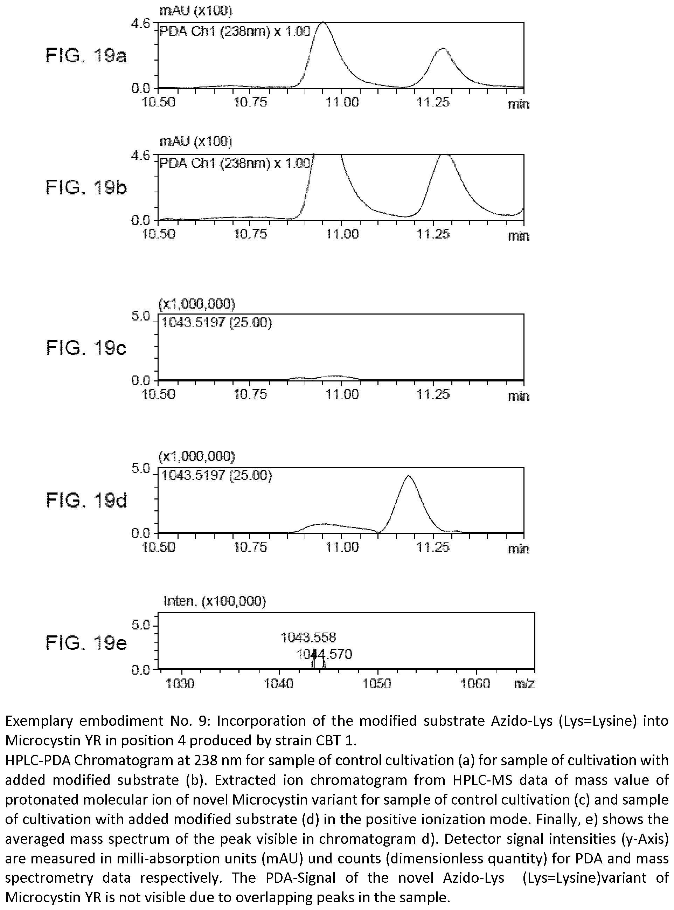

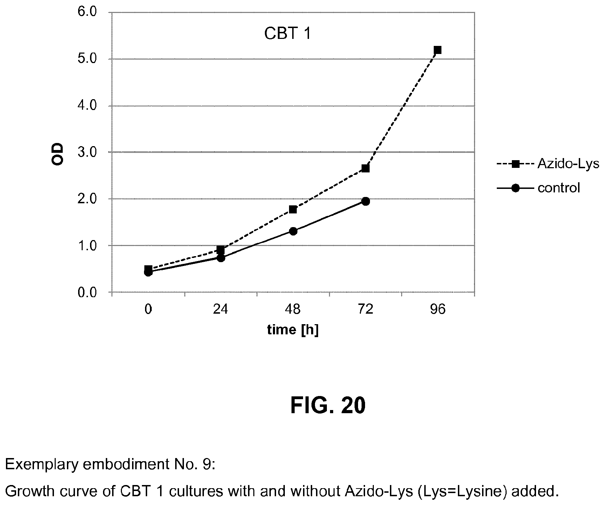

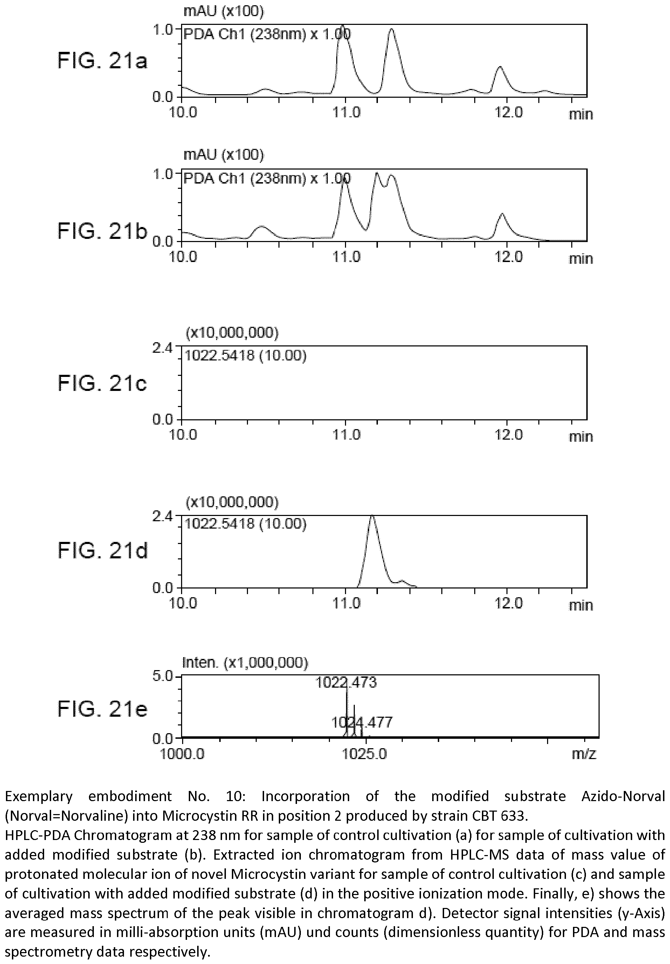

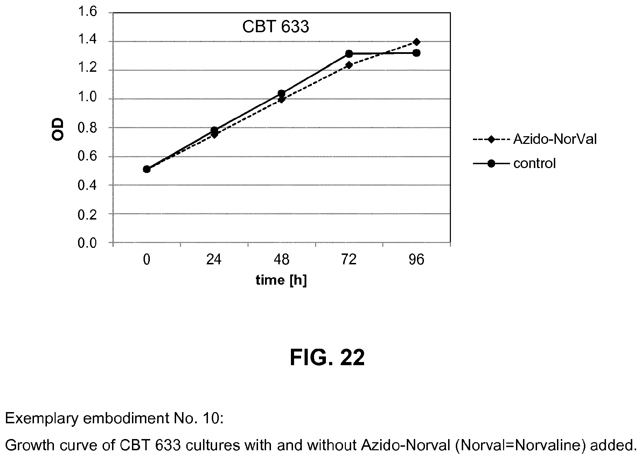

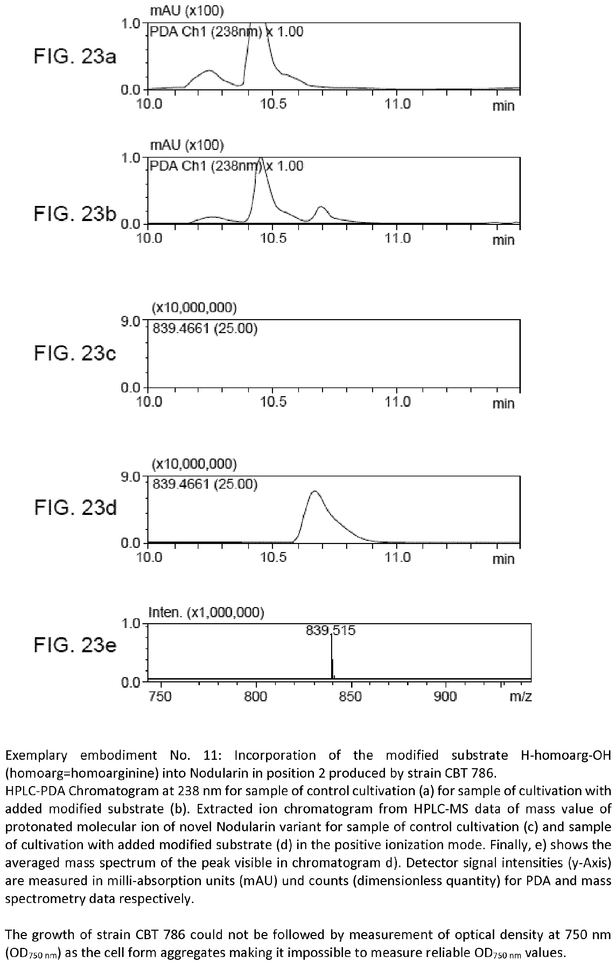

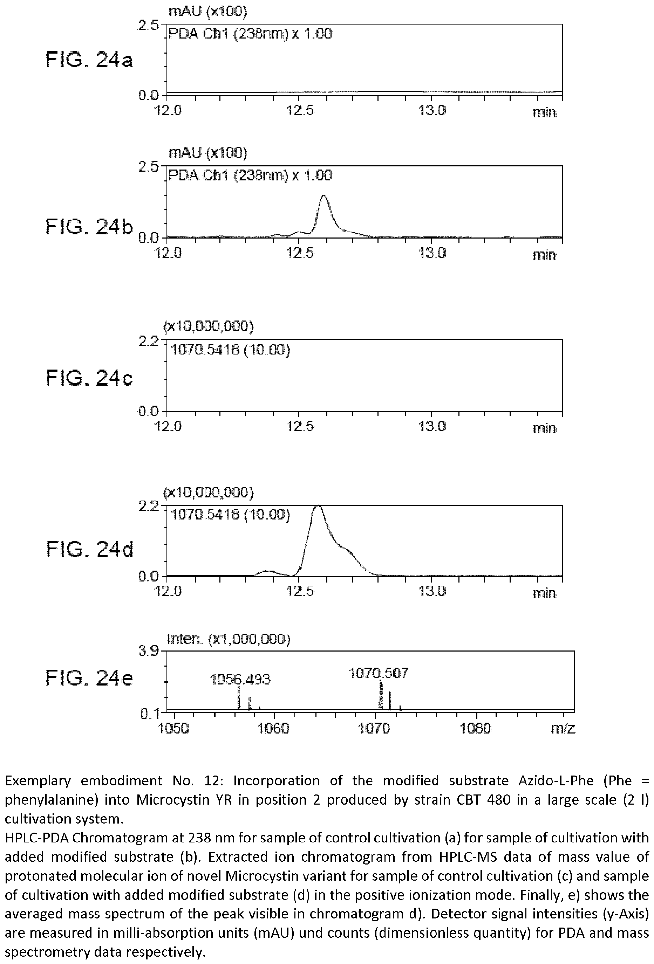

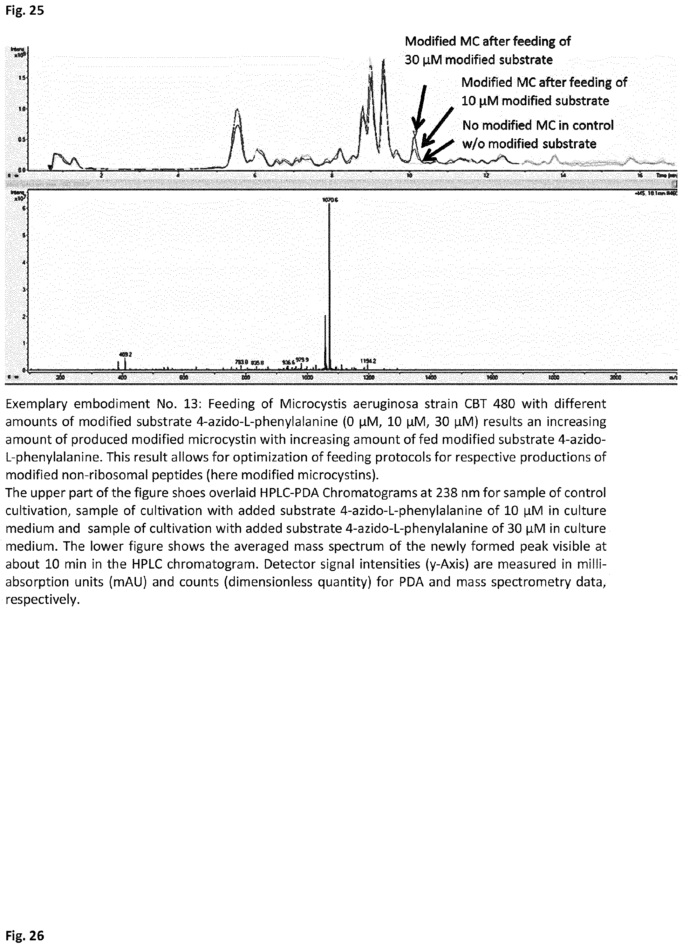

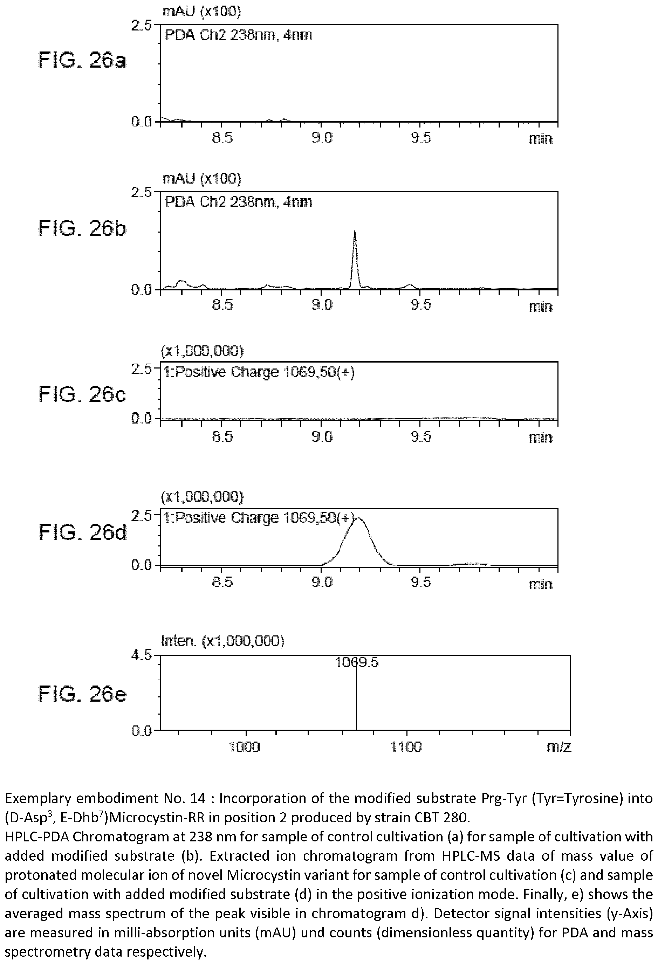

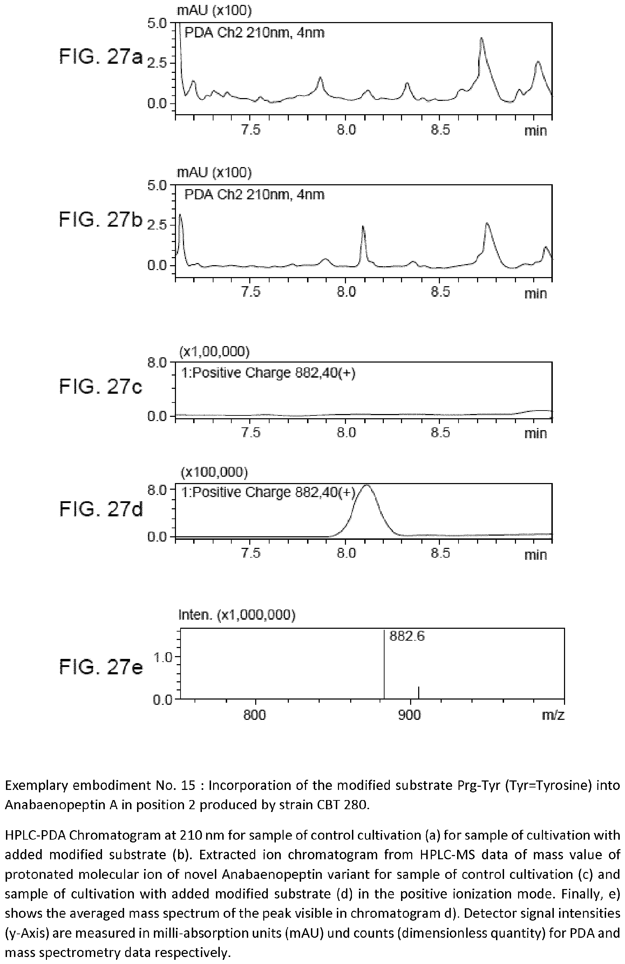

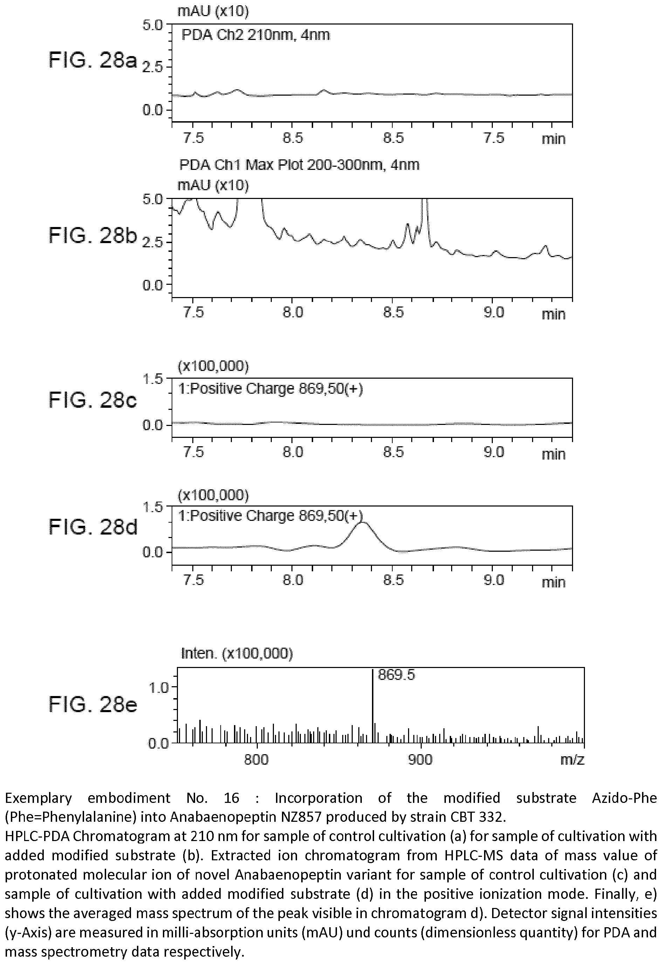

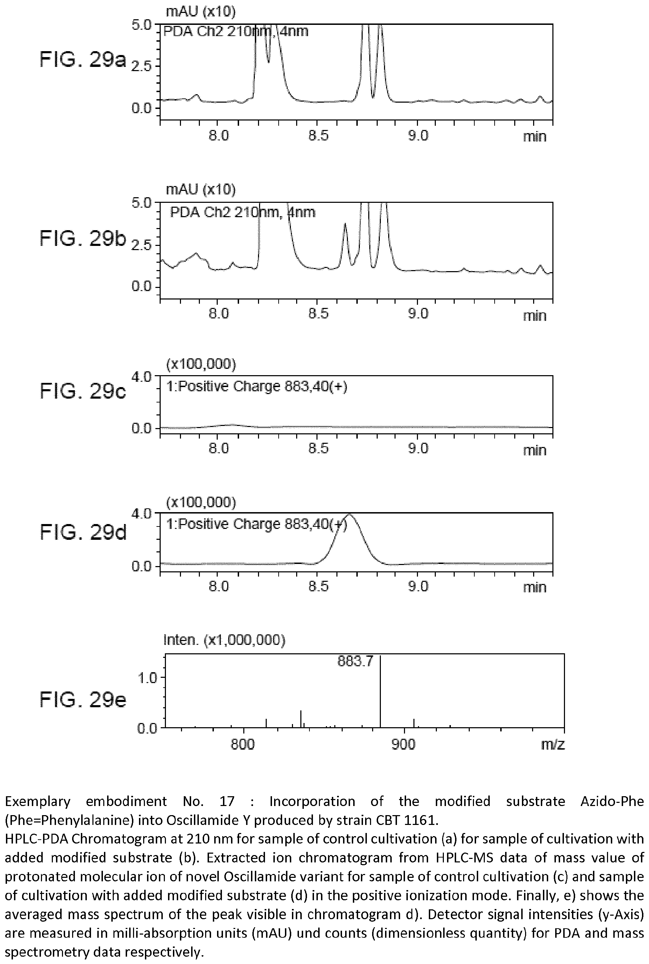

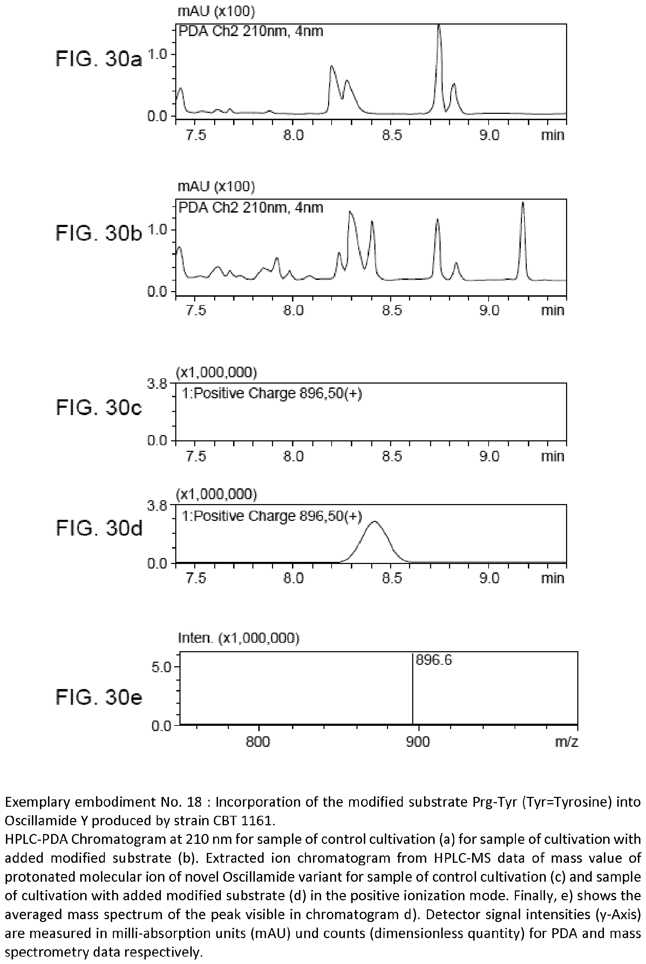

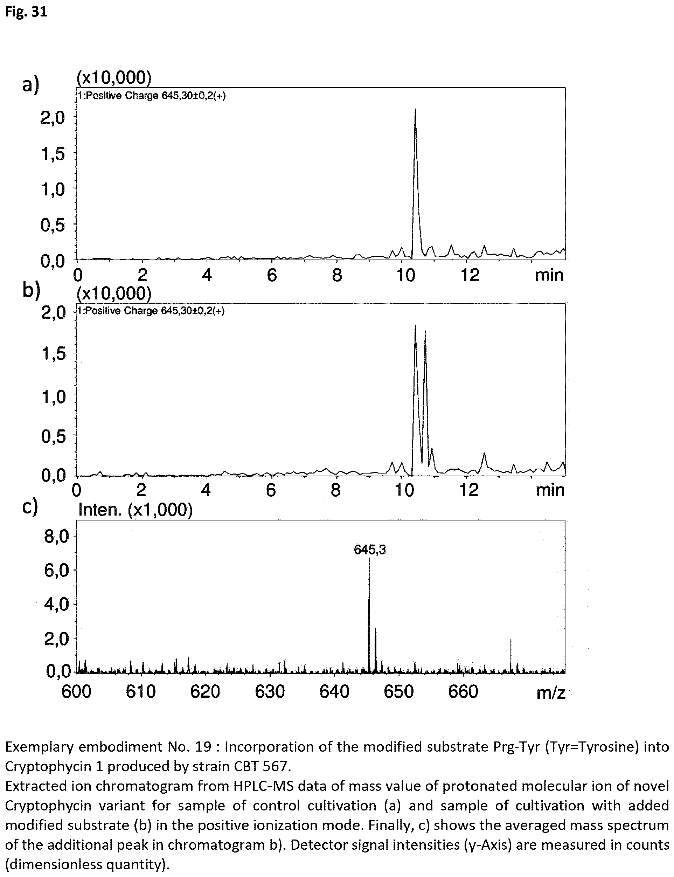

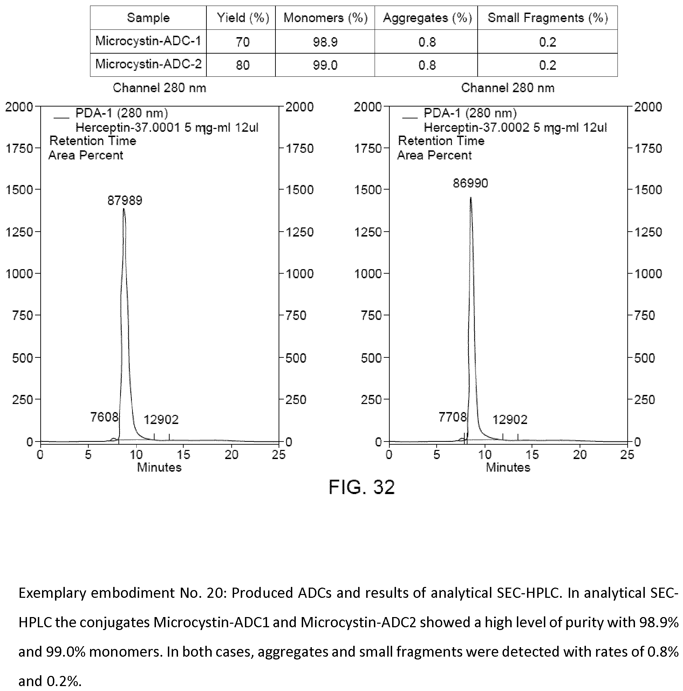

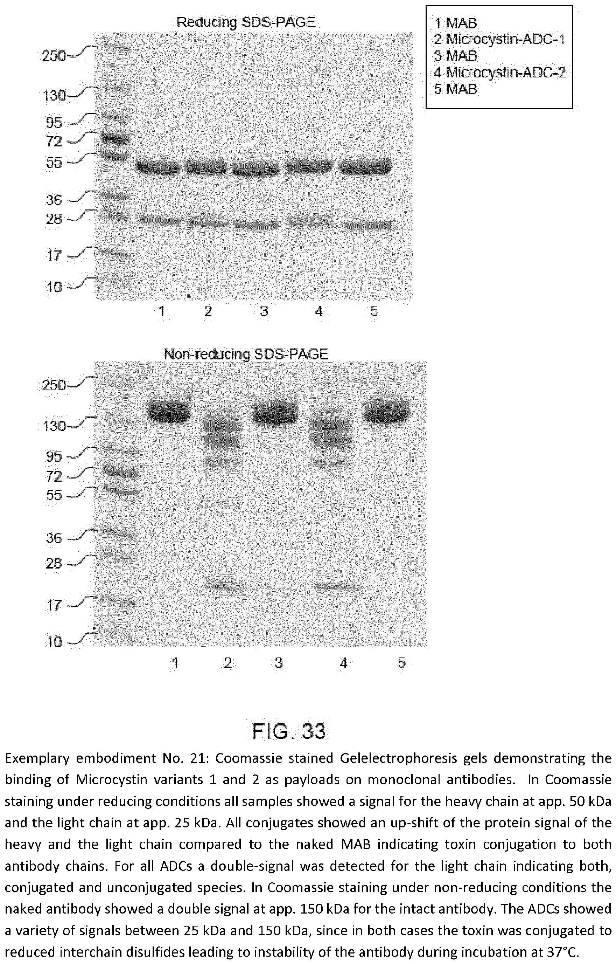

The main difference of the here described method of introduction of conjugation chemistry incl. click chemistry into the drug-like molecules compared to conventional methods is based on the way of introduction (via feeding of pre-selected cyanobacteria strains with pre-selected substrates and their site-specific introduction into the non-ribosomal peptides), the access to a broad diversity of suited modified substrates with structural features and different kind of conjugation and click chemistry, resp., and the resulting structural diversity of clickable drug-like molecules (see figure 4 - 30). In this connection the here described method of introduction of conjugation chemistry incl. click chemistry into the drug-like molecules also differs from conventional methods by the possibility of the structurally optimization of drug-like molecules which is further enhanced by the parallel use of modified substrates w/o conjugation chemistry incl. click chemistry (see figure 2A - D, 23). Finally the here described method of introduction of conjugation chemistry incl. click chemistry, into the drug-like molecules allows for the microbiological production of structurally optimized clickable non-ribosomal peptides in larger scales for industrial production(see figure 24 and 25).

The cyanobacteria strain may be selected from the group comprising Microcystis, Planktothrix, Oscillatoria, Nostoc, Anabaena, Aphanizomenon, Hapalosiphon, Nodularia, Lyngbya, Phormidium, Spirulina, Halospirulina, Arthrospira, Trichodesmium, Leptolyngbya, Plectonema, Myxosarcina, Pleurocapsa, Pseudanabaena, Geitlerinema, Euhalothece, Calothrix, Tolypothrix, Scytonema, Fischerella, Mastigocladus, Westiellopsis, Stigonema, Chlorogloeopsis, Cyanospira, Cylindrospermopsis, Cylindospermum, Microchaete, Rivularia, Autosira, Trichonema, Trichodesmium, Symploca, Starria, Prochlorothrix, Microcoleus, Limnothrix, Crinalium, Borzia, Chroococcidiopsis, Cyanocystis, Dermocarpella, Staniera, Xenococcus, Chamaesiphon, Chroococcus, Cyanobacterium, Cyanobium, Cyanothece, Dactylococcopsis, Gloeobacter, Gloeocapsa, Gloeothece.

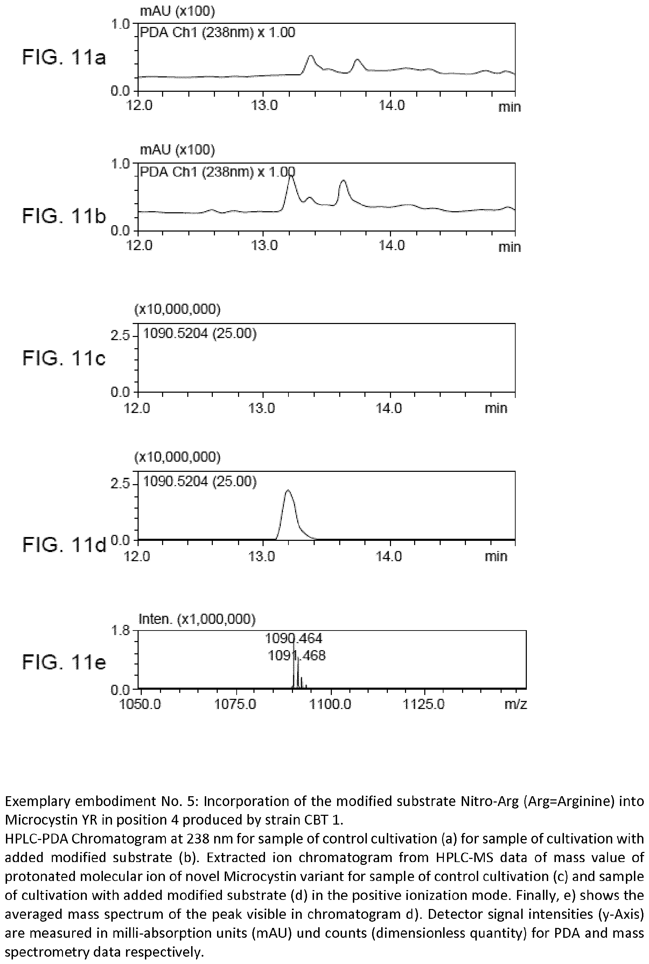

The inventors for the first time have incorporated modified amino acids into non-ribosomal peptides from cyanobacteria which carry so called clickable anchor groups which allow for the fast and easy binding of the entire molecule to e.g. linkers or other functional units like e.g. antibodies (see figure 4 -10,15/16 or whereas the fed substrates carry functional groups that are easily accessible to additional modification towards clickable anchor groups see figure 11 - 18).

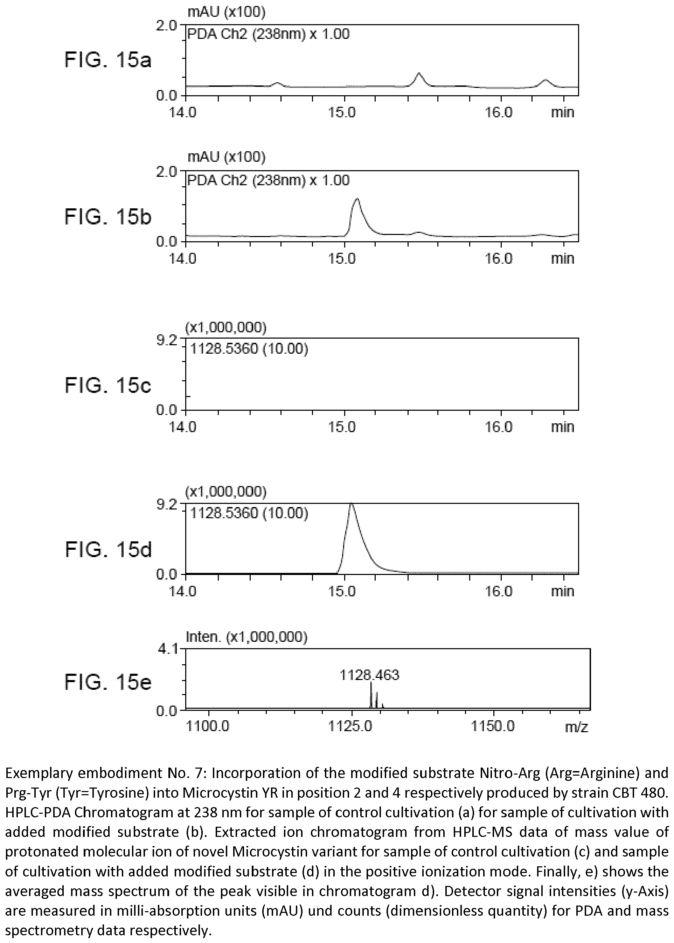

It is shown that feeding of any combination of a clickable substrate with e.g. amino acids naturally occurring in the respective non-ribosomal peptide, modified versions of these amino acids or any other modified substrates might potentially lead to an incorporation of the fed substrate combinations into the non-ribosomal peptide (see figure 15-18).

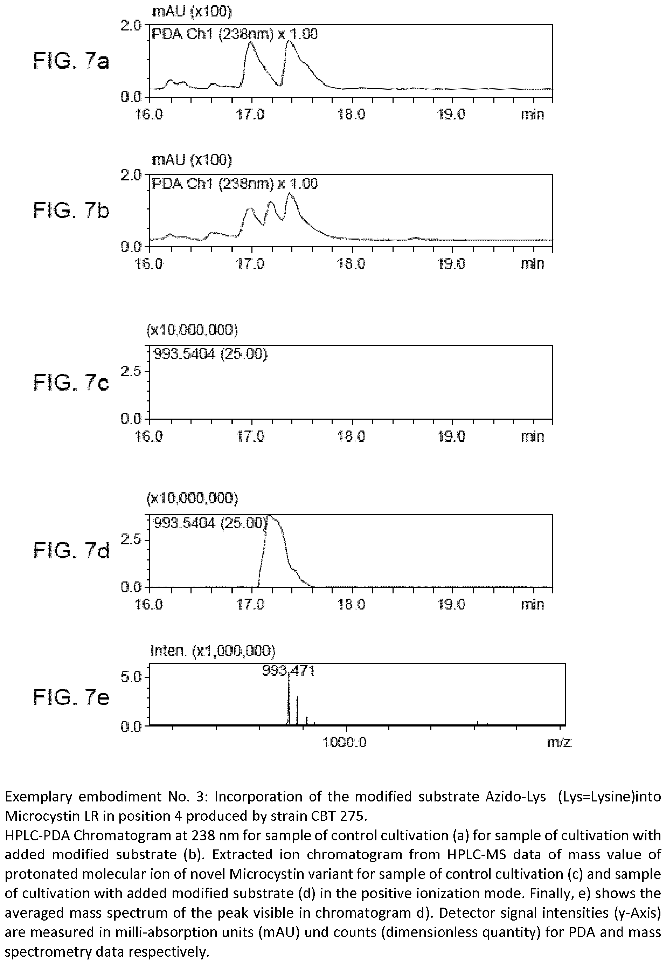

The inventors show for the first time that a successfully fed substrate (e.g. the modified amino acid) is structurally not necessarily directly related to the substrate that is naturally incorporated into the respective non-ribosomal peptide (e.g. the respective non-modified amino acid) (see figure 7/8,13/14, 19-22, 26). That means in the past successful feedings were only regarded to structural variants directly derived from the naturally (native) incorporated amino acid (e.g. o-methyl-tyrosine or chloro-tyrosine instead of tyrosine or homo-arginine instead of arginine). Considering the new results of the inventors it is obvious that the structural and functional diversity of non-ribosomal peptides generated by feeding significantly increases if also substrates can be used that are structurally not directly derived from the substrate which is naturally incorporated into the respective non-ribosomal peptide.

The invention relates to a method, as defined in the claims, of producing a modified non-ribosomal peptide, preferably a modified CA, wherein the strain and the modified substrates are selected and the chemical structure(s) of the produced non-ribosomal peptide(s) is/are known such that the incorporation of the modified substrates during cultivation into the non-ribosomal peptide occurs at a defined position.

Preferably, if the non-ribosomal peptide is a CA which is microcystin and the one or more modified substrates are incorporated at any position other than Addas and DGIu₆, which has the following general structure:
D-Ala₁-X₂-D-MeAsp₃-Z₄-Adda₅-DGIu₆-Mdha₇,
and wherein X₂ and Z₄ are positions of preferred incorporation of said modified amino acid. If the CA is nodularin the one or more modified substrates are incorporated at any position other than Adda3 and DGIu₄, which has the following general structure:
D-MeAsp₁-Arg₂-Adda₃-DGlu₄-Mdhb₅.

Preferably, if the non-ribosomal peptide is a CA which is microcystin, the modified position is X₂ or Z₄ and the modified substrate is a modified amino acid (see figure 4 - 14).

Also, preferably if the non-ribosomal peptide is a CA which is microcystin, the modified position is X₂ and Z₄ and the modified substrate is a modified amino acid (see figures 15 - 18).

The modified substrate, preferably modified amino acid, preferably contains an anchor group directly accessible or transformable for use in conjugation chemistry (incl. click chemistry), for the attachment of a targeting moiety or a label or for additional structural modifications (see figures 4 - 30).

In the method according to the invention, the conjugation chemistry reaction (incl. click chemistry reaction) of the clickable substrate is selected from reactions comprising copper(I)-catalyzed azide-alkyne cycloaddition, strain promoted azide-alkyne cycloaddition, alkyne-azide cycloaddition, or alkyne-tetrazine inverse-demand Diels-Alder reaction. Additional conjugation chemistry can be selected from reactions exploiting the specific reactivities of primary amines, thiols, aldehydes, carboxyls, and oximes. Therefore, the anchor group of at least one modified substrate which is directly accessible for use in conjugation chemistry (incl. click chemistry), for the attachment of a targeting moiety can be selected from the group of:
- Azido groups that can subsequently be modified e.g. by reaction with alkynes, activated alkenes, or phosphines, whereas the azido group of the cytotoxin reacts with the respective functional group of a linker, antibody, or other functional molecule such as a fluorescent dye or polymer matrix.
- Alkyne (e.g. propargy or diaryl-strained cyclooctyne) groups that can subsequently be modified e.g. by reaction with azides, whereas the alkyne group of the cytotoxin reacts with the respective functional group of a linker, antibody, or other functional molecule such as a fluorescent dye or polymer matrix.
- Tetrazines that can subsequently be modified e.g. by reaction with alkynes or alkenes, whereas the tetrazine group of the cytotoxin reacts with the respective functional group of a linker, antibody, or other functional molecule such as a fluorescent dye or polymer matrix.
- Primary amines that can subsequently be modified e.g. by reaction with isothiocyanates, isocyanates, acyl azides, NHS esters, sulfonyl chlorides, aldehydes, glyoxals, epoxides, oxiranes, carbonates, aryl halides, imidoesters, carbodiimides, anhydrides, phosphines, or fluorophenyl esters, whereas the amino group of the cytotoxin reacts with the respective functional group of a linker, antibody, or other functional molecule such as a fluorescent dye or polymer matrix.
- Thiols that can subsequently be modified e.g. by reaction with maleimides, haloacetyls, pyridyldisulfides, thiosulfonares, cyanobenzothiazoles, or vinylsulfones, whereas the thiol group of the cytotoxin reacts with the respective functional group of a linker, antibody, or other functional molecule such as a fluorescent dye or polymer matrix.
- Aldehydes that can subsequently be modified e.g. by reaction with amines, aminothiols, Ellman's Reagent, alkoxyamines, hydrazides or thiols, whereas the aldehyde group of the cytotoxin reacts with the respective functional group of a linker, antibody, or other functional molecule such as a fluorescent dye or polymer matrix.
- Carboxyls that can subsequently be modified e.g. by reaction with carbodiimides, whereas the cyrboxy group of the cytotoxin reacts with the respective functional group of a linker, antibody, or other functional molecule such as a fluorescent dye or polymer matrix.
- Oximes that can subsequently be modified e.g. by reaction with acetophenones such as p-acetylphenylalanine, whereas the oxime group of the cytotoxin reacts with the respective functional group of a linker, antibody, or other functional molecule such as a fluorescent dye or polymer matrix.

Also claimed is the introduction of at least one modified substrate with a functional group that is directly transformable for use in conjugation chemistry (incl. click chemistry) for the attachment of a targeting moiety. One examples for this is the introduction of a substrate containing a nitro group that can be reduced to yield a primary amino group, which, as described above, can be used for conjugation chemistry (incl. click chemistry). Another example is the introduction of a substrate containing a furanyl that can subsequently be modified e.g. by photoreaction with nucleophiles such as hydrazines, whereas the furanyl group reacts after activation to an unsaturated dicarbonyl residue with the respective nucleophilic functional group of a targeting moiety like a linker, antibody, or other functional molecule such as a fluorescent dye or a polymer matrix (see figures 11-18).

Tyrosine containing microcystins can also be functionalized using 4-phenyl-3H-1,2,4-triazoline-3,5(4H)-diones (PTADs) to introduce additional conjugation chemistry (incl. click chemistry) amenable functional groups as described above.

Ideally, modified amino acids which are directly accessible or transformable for use in conjugation chemistry (incl. click chemistry), are selected from the group of the following table (see figures 4 - 30 and tables 1 to 4 for respective execution examples):

| **Systematic name** | **CAS Number** | **Short name** | **Supplier** | **Order number** |
|---|---|---|---|---|
| (2S)-2-amino-3-azidopropanoic acid hydrochloride | 105661-40-3 | Azido-L-Ala | Iris Biotech GmbH | HAA1880 |
| (2S)-2-amino-6-azidohexanoic acid hydrochloride | 159610-92-1 | Azido-Lys | Iris Biotech GmbH | HAA1625 |
| (S)-2-Amino-5-azidopentanoic acid hydrochloride | 156463-09-1 | Azido-Norval | Iris Biotech GmbH | HAA1620 |
| (2S)-2-amino-3-(4-prop-2-ynoxyphenyl)propanoic acid hydrochloride | 610794-20-2 | Prg-Tyr | Iris Biotech GmbH | HAA1971 |
| (2S)-2-amino-5-(N'-nitrocarbamimidamido)pentanoic acid | 2149-70-4 | Nitro-Arg | Sigma-Aldrich Chemie GmbH | 2149-70-4 |
| (2S)-2-amino-3-(furan-2-yl)propanoic acid | 127682-08-0 | Furyl-Ala | Iris Biotech GmbH | HAA2930 |
| (S)-Amino-6-((prop-2-ynyloxy)carbonylamino)hexanoic acid hydrochloride | 1428330-91-9 | Lys(Poc) | Iris Biotech GmbH | HAA2090 |
| N-Propargyl-Lysine | 1428330-91-9 | Prg-Lys | SiChem | SC-8002 |
| (2S)-2-Amino-3-(4-azidophenyl)propanoic acid | 33173-53-4 | Azido-L-Phe | Iris Biotech GmbH | HAA1850 |
| L-α-Amino-ε-guanidinohexanoic acid | 156-86-5 | H-homo-Arg-OH | Bachem | 4016423 |

The invention relates to a method according to the claims, wherein one of the following microcystins is produced, D-Ala₁-X₂-D-MeAsp₃-Z₄-Adda₅-DGluₑ-Mdha₇,

| Position | 1 | 2 | 3 | 4 | 5 | 6 | 7 |
|---|---|---|---|---|---|---|---|
| Possible amino acids | Ala₁ | X₂ | D-MeAsp₃ | Z₄ | Adda₅ | DGlu₆ | Mdha₇ |
| | D-Ala | variable | D-MeAsp | variable | Adda | D-Glu | Mdha |
| | D-Ser | | D-Asp | | DM-Adda | D-Glu(OCH₃) | Dha |
| | D-Leu | | | | (6Z)Adda | | L-Ser |
| | | | | | ADM-Adda | | L-MeSer |
| | | | | | | | Dhb |
| | | | | | | | MeLan |

wherein

| | | | | |
|---|---|---|---|---|
| Ala₁ | X₂ | D-MeAsp₃ | Z₄ | Mdha₇ |

comprise the position of the incorporation of at least one modified substrate wherein preferably the modified substrate which are directly accessible or transformable for use in conjugation (click) chemistry is an amino acid selected from the group of:

| **Systematic name** | **CAS Number** | **Short name** | **Supplier** | **Order number** |
|---|---|---|---|---|
| (2S)-2-amino-3-azidopropanoic acid hydrochloride | 105661-40-3 | Azido-L-Ala | Iris Biotech GmbH | HAA1880 |
| (2S)-2-amino-6-azidohexanoic acid hydrochloride | 159610-92-1 | Azido-Lys | Iris Biotech GmbH | HAA1625 |
| (S)-2-Amino-5-azidopentanoic acid hydrochloride | 156463-09-1 | Azido-Norval | Iris Biotech GmbH | HAA1620 |
| (2S)-2-amino-3-(4-prop-2-ynoxyphenyl)propanoic acid hydrochloride | 610794-20-2 | Prg-Tyr | Iris Biotech GmbH | HAA1971 |
| (2S)-2-amino-5-(N'-nitrocarbamimidamido)pentanoic acid | 2149-70-4 | Nitro-Arg | Sigma-Aldrich Chemie GmbH | 2149-70-4 |
| (2S)-2-amino-3-(furan-2-yl)propanoic acid | 127682-08-0 | Furyl-Ala | Iris Biotech GmbH | HAA2930 |
| (S)-Amino-6-((prop-2-ynyloxy)carbonylamino)hexanoic acid hydrochloride | 1428330-91-9 | Lys(Poc) | Iris Biotech GmbH | HAA2090 |
| N-Propargyl-Lysine | 1428330-91-9 | Prg-Lys | SiChem | SC-8002 |
| (2S)-2-Amino-3-(4-azidophenyl)propanoic acid | 33173-53-4 | Azido-L-Phe | Iris Biotech GmbH | HAA1850 |
| L-α-Amino-ε-guanidinohexanoic acid | 156-86-5 | H-homo-Arg-OH | Bachem | 4016423 |

The invention also relates to a method according to the claims, wherein one of the following nodularins is produced,

| Position | 1 | 2 | 3 | 4 | 5 |
|---|---|---|---|---|---|
| Possible amino acid | MeAsp₁ | Arg₂ | Adda₃ | DGlu₄ | Mdhb₅ |
| | D-MeAsp | Homo-Arg | Adda | D-Glu | Mdhb |
| | D-Asp | | DM-Adda | D-Glu(OCH₃) | Dhb |
| | | | (6Z)Adda | | |
| | | | Me-Adda | | |

Wherein

| | | |
|---|---|---|
| MeAsp₁ | Arg₂ | Mdhb₅ |

comprise the position for the at least one modified substrate, wherein preferably the modified substrate is an amino acid selected from the group of:

| **Systematic name** | **CAS Number** | **Short name** | **Supplier** | **Order number** |
|---|---|---|---|---|
| (2S)-2-amino-3-azidopropanoic acid hydrochloride | 105661-40-3 | Azido-L-Ala | Iris Biotech GmbH | HAA1880 |
| (2S)-2-amino-6-azidohexanoic acid hydrochloride | 159610-92-1 | Azido-Lys | Iris Biotech GmbH | HAA1625 |
| (S)-2-Amino-5-azidopentanoic acid hydrochloride | 156463-09-1 | Azido-Norval | Iris Biotech GmbH | HAA1620 |
| (2S)-2-amino-3-(4-prop-2-ynoxyphenyl)propanoic acid hydrochloride | 610794-20-2 | Prg-Tyr | Iris Biotech GmbH | HAA1971 |
| (2S)-2-amino-5-(N'-nitrocarbamimidamido)pentanoic acid | 2149-70-4 | Nitro-Arg | Sigma-Aldrich Chemie GmbH | 2149-70-4 |
| (2S)-2-amino-3-(furan-2-yl)propanoic acid | 127682-08-0 | Furyl-Ala | Iris Biotech GmbH | HAA2930 |
| (S)-Amino-6-((prop-2-ynyloxy)carbonylamino)hexanoic acid hydrochloride | 1428330-91-9 | Lys(Poc) | Iris Biotech GmbH | HAA2090 |
| N-Propargyl-Lysine | 1428330-91-9 | Prg-Lys | SiChem | SC-8002 |
| (2S)-2-Amino-3-(4-azidophenyl)propanoic acid | 33173-53-4 | Azido-L-Phe | Iris Biotech GmbH | HAA1850 |
| L-α-Amino-ε-guanidinohexanoic acid | 156-86-5 | H-homo-Arg-OH | Bachem | 4016423 |

Ideally, the nodularin is modified at the Arg₂ position.

The invention also relates to a method according to the claims, wherein one of the following anabaenopeptins is produced,

| Position | 1 | 3 | 4 | 5 | 6 |
|---|---|---|---|---|---|
| Possible amino acid | Tyr | Val | HTyr | MeAla | Phe |
| | Arg | lie | MeHTyr | MeLeu | Tyr |
| | Lys | | HPhe | MeHTyr | lie |
| | Phe | | | MeTyr | Leu |
| | Ile | | | | |
| | HArg | | | | |

Wherein

| | |
|---|---|
| Tyr | Phe |

comprise the position for the at least one modified substrate, wherein preferably the modified substrate is an amino acid selected from the group of:

| **Systematic name** | **CAS Number** | **Short name** | **Supplier** | **Order number** |
|---|---|---|---|---|
| (2S)-2-amino-3-azidopropanoic acid hydrochloride | 105661-40-3 | Azido-L-Ala | Iris Biotech GmbH | HAA1880 |
| (2S)-2-amino-6-azidohexanoic acid hydrochloride | 159610-92-1 | Azido-Lys | Iris Biotech GmbH | HAA1625 |
| (S)-2-Amino-5-azidopentanoic acid hydrochloride | 156463-09-1 | Azido-Norval | Iris Biotech GmbH | HAA1620 |
| (2S)-2-amino-3-(4-prop-2-ynoxyphenyl)propanoic acid hydrochloride | 610794-20-2 | Prg-Tyr | Iris Biotech GmbH | HAA1971 |
| (2S)-2-amino-5-(N'-nitrocarbamimidamido)pentanoic acid | 2149-70-4 | Nitro-Arg | Sigma-Aldrich Chemie GmbH | 2149-70-4 |
| (2S)-2-amino-3-(furan-2-yl)propanoic acid | 127682-08-0 | Furyl-Ala | Iris Biotech GmbH | HAA2930 |
| (S)-Amino-6-((prop-2-ynyloxy)carbonylamino)hexanoic acid hydrochloride | 1428330-91-9 | Lys(Poc) | Iris Biotech GmbH | HAA2090 |
| N-Propargyl-Lysine | 1428330-91-9 | Prg-Lys | SiChem | SC-8002 |
| (2S)-2-Amino-3-(4-azidophenyl)propanoic acid | 33173-53-4 | Azido-L-Phe | Iris Biotech GmbH | HAA1850 |
| L-α-Amino-ε-guanidinohexanoic acid | 156-86-5 | H-homo-Arg-OH | Bachem | 4016423 |

The invention also relates to a method according to the claims, wherein one of the following oscillamides is produced,

| Position | 1 | 3 | 4 | 5 | 6 |
|---|---|---|---|---|---|
| Possible amino acid | Tyr | Met | HTyr | MeAla | Phe |
| | Arg | Ile | | MeHTyr | |

Wherein

| | |
|---|---|
| Tyr | HTyr |

comprise the position for the at least one modified substrate, wherein preferably the modified substrate is an amino acid selected from the group of:

| **Systematic name** | **CAS Number** | **Short name** | **Supplier** | **Order number** |
|---|---|---|---|---|
| (2S)-2-amino-3-azidopropanoic acid hydrochloride | 105661-40-3 | Azido-L-Ala | Iris Biotech GmbH | HAA1880 |
| (2S)-2-amino-6-azidohexanoic acid hydrochloride | 159610-92-1 | Azido-Lys | Iris Biotech GmbH | HAA1625 |
| (S)-2-Amino-5-azidopentanoic acid hydrochloride | 156463-09-1 | Azido-Norval | Iris Biotech GmbH | HAA1620 |
| (2S)-2-amino-3-(4-prop-2-ynoxyphenyl)propanoic acid hydrochloride | 610794-20-2 | Prg-Tyr | Iris Biotech GmbH | HAA1971 |
| (2S)-2-amino-5-(N'-nitrocarbamimidamido)pentanoic acid | 2149-70-4 | Nitro-Arg | Sigma-Aldrich Chemie GmbH | 2149-70-4 |
| (2S)-2-amino-3-(furan-2-yl)propanoic acid | 127682-08-0 | Furyl-Ala | Iris Biotech GmbH | HAA2930 |
| (S)-Amino-6-((prop-2-ynyloxy)carbonylamino)hexanoic acid hydrochloride | 1428330-91-9 | Lys(Poc) | Iris Biotech GmbH | HAA2090 |
| N-Propargyl-Lysine | 1428330-91-9 | Prg-Lys | SiChem | SC-8002 |
| (2S)-2-Amino-3-(4-azidophenyl)propanoic acid | 33173-53-4 | Azido-L-Phe | Iris Biotech GmbH | HAA1850 |
| L-α-Amino-ε-guanidinohexanoic acid | 156-86-5 | H-homo-Arg-OH | Bachem | 4016423 |

The invention also relates to a method according to the claims, wherein modified cryptophycins are produced, wherein the O-methyl-chloro-Tyrosine in cryptophycin 1 comprise the position for the at least one modified substrate, wherein preferably the modified substrate is an amino acid selected from the group of:

| **Systematic name** | **CAS Number** | **Short name** | **Supplier** | **Order number** |
|---|---|---|---|---|
| (2S)-2-amino-3-azidopropanoic acid hydrochloride | 105661-40-3 | Azido-L-Ala | Iris Biotech GmbH | HAA1880 |
| (2S)-2-amino-6-azidohexanoic acid hydrochloride | 159610-92-1 | Azido-Lys | Iris Biotech GmbH | HAA1625 |
| (S)-2-Amino-5-azidopentanoic acid hydrochloride | 156463-09-1 | Azido-Norval | Iris Biotech GmbH | HAA1620 |
| (2S)-2-amino-3-(4-prop-2-ynoxyphenyl)propanoic acid hydrochloride | 610794-20-2 | Prg-Tyr | Iris Biotech GmbH | HAA1971 |
| (2S)-2-amino-5-(N'-nitrocarbamimidamido)pentanoic acid | 2149-70-4 | Nitro-Arg | Sigma-Aldrich Chemie GmbH | 2149-70-4 |
| (2S)-2-amino-3-(furan-2-yl)propanoic acid | 127682-08-0 | Furyl-Ala | Iris Biotech GmbH | HAA2930 |
| (S)-Amino-6-((prop-2-ynyloxy)carbonylamino)hexanoic acid hydrochloride | 1428330-91-9 | Lys(Poc) | Iris Biotech GmbH | HAA2090 |
| N-Propargyl-Lysine | 1428330-91-9 | Prg-Lys | SiChem | SC-8002 |
| (2S)-2-Amino-3-(4-azidophenyl)propanoic acid | 33173-53-4 | Azido-L-Phe | Iris Biotech GmbH | HAA1850 |
| L-α-Amino-ε-guanidinohexanoic acid | 156-86-5 | H-homo-Arg-OH | Bachem | 4016423 |

In the method according to the invention, the at least one modified amino acid comprises an anchor group directly accessible or transformable for use in conjugation chemistry (incl. click chemistry), for the attachment of a targeting moiety and/or a label via a linker or w/o a linker between the modified amino acid and the targeting moiety and/or a label. Such anchor groups are described above for the modified substrates.

In the method according to the invention, the conjugation chemistry reaction (incl. click chemistry reaction) of the clickable substrate is selected from the group comprising copper(I)-catalyzed azide-alkyne cycloaddition, strain-promoted azide-alkyne cycloaddition, alkyne-azide cycloaddition, or alkyne-tetrazine inverse-demand Diels-Alder reaction. Additional conjugation chemistry can be selected from reactions exploiting the specific reactivities of primary amines, thiols, aldehydes, carboxyls, and oximes.

However, regarding the modification of the CA of microcystins and nodularins by the introduction of modified substrates most preferred are the genera Microcystis, Planktothrix, Oscillatoria, Nostoc, Anabaena, Aphanizomenon, Hapalosiphon, Nodularia.

A modified non-ribosomal peptide, including a modified CA compound comprising at least one modified amino acid, wherein the at least one modified amino acid comprises an anchor group directly accessible or transformable for use in conjugation chemistry (incl. click chemistry), for the attachment of a targeting moiety and/or a label via a linker or w/o a linker between the modified amino acid and the targeting moiety and/or a label, is also disclosed.

Ideally, the at least one modified CA compound is a microcystin, a nodularin or a cryptophycin or one of the CA listed in the above table with cyanobacterial CA.

Preferably, the modified amino acid in the CA is linked to a targeting moiety or a label.

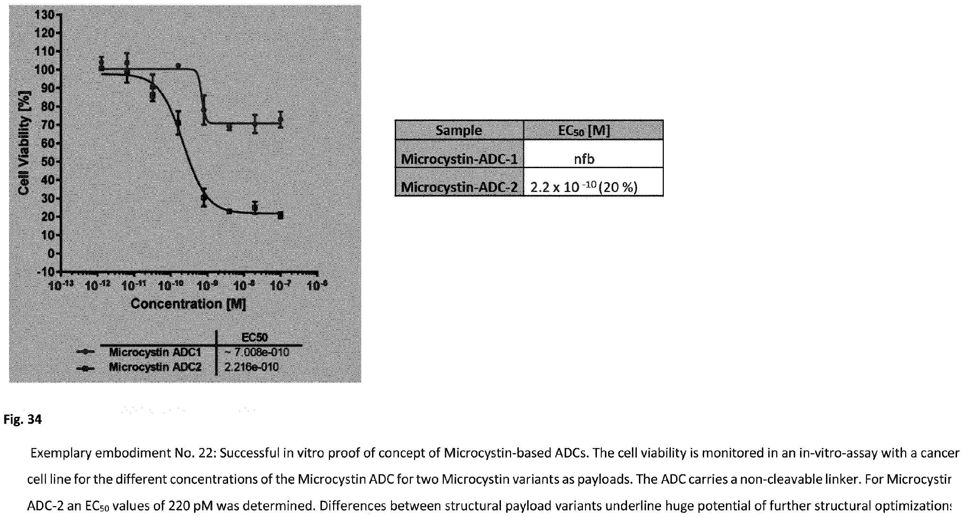

Concerning the targeting moiety (TM), in one embodiment, the ADC specifically binds to a receptor encoded by an ErbB gene. The TM may bind specifically to an ErbB receptor selected from EGFR, HER2, HER3 and HER4. The ADC may specifically bind to the extracellular domain (ECD) of the HER2 receptor and inhibit the growth of tumor cells which overexpress HER2 receptor (see figure 34). The antibody of the ADC may be a monoclonal antibody, e.g. a murine monoclonal antibody, a chimeric antibody, or a humanized antibody. A humanized antibody may be huMAb4D5-1, huMAb4D5-2, huMAb4D5-3, huMAb4D5-4, huMAb4D5-5, huMAb4D5-6, huMAb4D5-7 or huMAb4D5-8 (trastuzumab). The antibody may be an antibody fragment, e.g. a Fab fragment.

The ADC produced by the method of the invention may be useful in the treatment of cancer including, but are not limited to, antibodies against cell surface receptors and tumor-associated antigens (TAA). Such tumor-associated antigens are known in the art, and can prepared for use in generating antibodies using methods and information which are well known in the art. In attempts to discover effective cellular targets for cancer diagnosis and therapy, researchers have sought to identify transmembrane or otherwise tumor-associated polypeptides that are specifically expressed on the surface of one or more particular type(s) of cancer cell as compared to on one or more normal non-cancerous cell(s). Often, such tumor-associated polypeptides are more abundantly expressed on the surface of the cancer cells as compared to on the surface of the non-cancerous cells. The identification of such tumor-associated cell surface antigen polypeptides has given rise to the ability to specifically target cancer cells for destruction via targeted antibody-based therapies.

Examples of TAA include, but are not limited to, Tumor-Associated Antigens listed below. Tumor-associated antigens targeted by antibodies include all amino acid sequence variants and isoforms possessing at least about 70%, 80%, 85%, 90%, or 95% sequence identity relative to the sequences identified in the cited references, or which exhibit substantially the same biological properties or characteristics as a TAA having a sequence found in the cited references. For example, a TAA having a variant sequence generally is able to bind specifically to an antibody that binds specifically to the TAA with the corresponding sequence listed.
BMPR1B (bone morphogenetic protein receptor-type IB, Genbank accession no. NM-001203);
E16 (LAT1, SLC7A5, Genbank accession no. NM-003486);
STEAP1 (six transmembrane epithelial antigen of prostate, Genbank accession no. NM-012449); 0772P (CA125, MUC16, Genbank accession no. AF361486);
MPF (MPF, MSLN, SMR, megakaryocyte potentiating factor, mesothelin, Genbank accession no. NM-005823);
Napi3b (NAPI-3B, NPTIIb, SLC34A2, solute carrier family 34 (sodium phosphate), member 2, type II sodium-dependent phosphate transporter 3b, Genbank accession no. NM-006424);
Sema 5b (F1110372, KIAA1445, Mm.42015, SEMA5B, SEMAG, Semaphorin 5b Hlog, sema domain, seven thrombospondin repeats (type 1 and type 1-like), transmembrane domain (TM) and short cytoplasmic domain, (semaphorin) 5B, Genbank accession no. AB040878);
PSCA hlg (2700050C12Rik, C530008016Rik, RIKEN cDNA 2700050C12, RIKEN cDNA 2700050C12 gene, Genbank accession no. AY358628);
ETBR (Endothelin type B receptor, Genbank accession no. AY275463);
MSG783 (RNF124, hypothetical protein F1120315, Genbank accession no. NM-017763);
STEAP2 (HGNC-8639, IPCA-1, PCANAP1, STAMP1, STEAP2, STMP, prostate cancer associated gene 1, prostate cancer associated protein 1, six transmembrane epithelial antigen of prostate 2, six transmembrane prostate protein, Genbank accession no. AF455138);
TrpM4 (BR22450, F1120041, TRPM4, TRPM4B, transient receptor potential cation channel, subfamily M, member 4, Genbank accession no. NM-017636);
CRIPTO (CR, CR1, CRGF, CRIPTO, TDGF1, teratocarcinoma-derived growth factor, Genbank accession no. NP-003203 or NM-003212);
CD21 (CR2 (Complement receptor 2) or C3DR(C3d/Epstein Barr virus receptor) or Hs.73792 Genbank accession no. M26004);
CD79b (CD79B, CD79β, IGb (immunoglobulin-associated beta), B29, Genbank accession no. NM-000626 or 11038674);
FcRH2 (IFGP4, IRTA4, SPAP1A (SH2 domain containing phosphatase anchor protein 1a), SPAP1B, SPAP1C, Genbank accession no. NM-030764, AY358130);
HER2 (ErbB2, Genbank accession no. M11730); Coussens L., et al Science (1985) 230(4730):1132-1139);
NCA (CEACAM6, Genbank accession no. M18728); Barnett T., et al Genomics 3, 59-66, 1988;
MDP (DPEP1, Genbank accession no. BC017023);
IL20Rα (IL20Ra, ZCYTOR7, Genbank accession no. AF184971);
Brevican (BCAN, BEHAB, Genbank accession no. AF229053);
EphB2R (DRT, ERK, HekS, EPHT3, Tyro5, Genbank accession no. NM-004442);
ASLG659 (B7h, Genbank accession no. AX092328); US20040101899 (Claim 2);
PSCA (Prostate stem cell antigen precursor, Genbank accession no. AJ297436);
GEDA (Genbank accession No. AY260763); AAP14954 lipoma HMGIC fusion-partner-like protein/pid=AAP14954.1 Homo sapiens (human);
(26) BAFF-R (B cell-activating factor receptor, BLyS receptor 3, BR3, Genbank accession No. AF116456); BAFF receptor/pid=NP-443177.1-Homo sapiens; Thompson, J. S., et al Science 293 (5537), 2108-2111 (2001); WO2004058309; WO2004011611; WO2003045422 (Example; Page 32-33); WO2003014294 (Claim 35; FIG. 6B); WO2003035846 (Claim 70; Page 615-616); WO200294852 (Col 136-137); WO200238766 (Claim 3; Page 133); WO200224909 (Example 3; FIG. 3); Cross-references: MIM:606269; NP-443177.1; NM-052945-1; AF132600
CD22 (B-cell receptor CD22-B isoform, BL-CAM, Lyb-8, Lyb8, SIGLEC-2, FU22814, Genbank accession No. AK026467);
CD79a (CD79A, CD79α, immunoglobulin-associated alpha, a B cell-specific protein that covalently interacts with Ig beta (CD79B) and forms a complex on the surface with Ig M molecules, transduces a signal involved in B-cell differentiation);
CXCR5 (Burkitt's lymphoma receptor 1, a G protein-coupled receptor that is activated by the CXCL13 chemokine, functions in lymphocyte migration and humoral defense, plays a role in HIV-2 infection and perhaps development of AIDS, lymphoma, myeloma, and leukemia);
HLA-DOB (Beta subunit of MHC class II molecule (la antigen) that binds peptides and presents them to CD4+T lymphocytes);
P2X5 (Purinergic receptor P2X ligand-gated ion channel 5, an ion channel gated by extracellular ATP, may be involved in synaptic transmission and neurogenesis, deficiency may contribute to the pathophysiology of idiopathic detrusor instability);
CD72 (B-cell differentiation antigen CD72, Lyb-2); 359 aa), pl: 8.66, MW: 40225 TM: 1 [P] Gene Chromosome: 9p13.3, Genbank accession No. NP-001773.1);
LY64 (Lymphocyte antigen 64 (RP105), type I membrane protein of the leucine rich repeat (LRR) family, regulates B-cell activation and apoptosis, loss of function is associated with increased disease activity in patients with systemic lupus erythematosis);
FcRH1 (Fc receptor-like protein 1, a putative receptor for the immunoglobulin Fc domain that contains C2 type Ig-like and ITAM domains, may have a role in B-lymphocyte differentiation);
IRTA2 (FcRH5, Immunoglobulin superfamily receptor translocation associated 2, a putative immunoreceptor with possible roles in B cell development and lymphomagenesis;
TENB2 (TMEFF2, tomoregulin, TPEF, HPP1, TR, putative transmembrane proteoglycan, related to the EGF/heregulin family of growth factors and follistatin);
MUC1 (Tumor-associated MUC1 glycopeptide epitopes); Human adenocarcinomas overexpress a hypoglycosylated, tumor-associated form of the mucin-like glycoprotein MUC1 containing abnormal mono- and disaccharide antigens, such as Tn, sialyl-Tn, and TF, as well as stretches of unglycosylated protein backbone in the variable number of tandem repeats (VNTR) region.

The ADC which can be produced based on the method of the present invention may be used to treat various diseases or disorders in a patient, such as cancer and autoimmune conditions including those characterized by the overexpression of a disease-associated antigen, including but not limited to tumor-associated antigen. Exemplary conditions or disorders include infection diseases, thrombosis and others and specifically benign or malignant tumors; leukemia and lymphoid malignancies; other disorders such as neuronal, glial, astrocytal, hypothalamic, glandular, macrophagal, epithelial, stromal, blastocoelic, inflammatory, angiogenic and immunologic disorders. Cancer types susceptible to ADC treatment include those which are characterized by the overexpression of certain tumor associated antigens or cell surface receptors, e.g. HER2.

One example is the treatment of cancer in a mammal, wherein the cancer is characterized by the overexpression of an ErbB receptor. The mammal optionally does not respond, or responds poorly, to treatment with an unconjugated anti-ErbB antibody. The treatment (not part of the scope) comprises administering to the mammal a therapeutically effective amount of an antibody-drug conjugate compound. The growth of tumor cells that overexpress a growth factor receptor such as HER2 receptor or EGF receptor may be inhibited by administering to a patient an ADC, produced by the method of the invention, which binds specifically to said growth factor receptor and a chemotherapeutic agent wherein said antibody-drug conjugate and said chemotherapeutic agent are each administered in amounts effective to inhibit growth of tumor cells in the patient (see figure 34).

Examples of cancer to be treated include, but are not limited to, carcinoma, lymphoma, blastoma, sarcoma, and leukemia or lymphoid malignancies. More particular examples of such cancers include squamous cell cancer (e.g. epithelial squamous cell cancer), lung cancer including small-cell lung cancer, non-small cell lung cancer, adenocarcinoma of the lung and squamous carcinoma of the lung, cancer of the peritoneum, hepatocellular cancer, gastric or stomach cancer including gastrointestinal cancer, gastrointestinal stromal tumor (GIST), pancreatic cancer, glioblastoma, cervical cancer, ovarian cancer, liver cancer, bladder cancer, hepatoma, breast cancer, colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, as well as head and neck cancer.

### EXAMPLES

Successful feedings of modified substrates were performed in different cultivation systems and scales allowing for screening (small scales of up to 10 ml; see figure 2A - D) and for production (2 - 20 L scales; see figure 24, 25) of modified non-ribosomal peptides. The different screening scales comprise:
1.6 ml cultures cultivated in ca. 2.2 ml deep-well microtiter plates (dw-MTP) whereas CO₂ supply was assured by intense shaking of 600 rpm and a constant CO₂ concentration of 5 % in the head space above the dw-MTP. Illumination occurred via LED panel or vial fluorescence bulbs for 24 hours a day. Light intensity was adjusted in dependence of the strain and its growth phase between 35-250 µmol/s*m². The temperature was strain-specific varied between 20 °C and 30 °C.

A cultivation according to the method is thus preferred wherein the shaking is between 400-800 rpm and a constant CO₂ concentration of 1 to 10 % in the head space, preferably 3 to 8 % in the head space. 10 ml cultures cultivated in 40 ml polystyrene tubes whereas CO₂ supply was assured by intense shaking of 250-350 rpm and a constant CO₂ concentration of 5% below the culture vessel. Hereby CO₂ was introduced into the culture via a CO₂ permeable polypropylene membrane on the bottom of the culture vessels. Illumination occurred via fluorescence bulbs for 24 hours a day and light intensity was again adjusted in dependence of the strain and its growth phase between 35-250 µmol/s*m². The temperature was again strain-specific varied between 20 °C and 30 °C.

A cultivation according to the method is thus preferred wherein the illumination occurred via fluorescence bulbs for 24 hours a day and is between 20-450 µmol/s*m2.

50 ml cultures cultivated in glass flasks whereas CO₂ supply was assured by bubbling with constant CO₂ concentration of 5 %. The cultures were mixed via stirring with a magnetic stir bar at 100 rpm. Illumination occurred via fluorescence bulbs for 24 hours a day and intensity was adjusted in dependence of strain and growth phase between 35-250 µmol/s*m2.

In addition, feeding experiments were also performed in a production scale between 2 L and 20 L whereas CO₂ supply and mixing was assured by bubbling with constant CO₂ concentration of 0.5-5.0 %. Illumination occurred via fluorescence bulbs and intensity was adjusted in dependence of strain and growth phase between 35-250 µmol/s*m2

Optionally, the cultivations were performed under day-night-cycles of 16 hours light/8 hours at the same light intensities during the day period as described above.

Optionally, the cultivations were performed with different light sources (e.g. LED lights or sulfurplasma lamps) and using strain-specific variations of light intensity, CO₂ concentration, shaking/stirring intensity and media composition.

Exemplary feeding scheme for the 10 ml scale:
All strains were cultivated in BG11 medium (see below), according to strain-specific cultivation conditions determined before.

Cells were pre-cultivated in Erlenmeyer flasks under low light conditions (30 µmol/s*m2) for 4 days at 25°C and on a shaker at 70 rpm.

For the feeding experiment in the 10 ml scale, the cells were inoculated at optical density at 750 nm (OD750 nm) of 0.5 in ca. 40 ml polystyrene tubes. The medium was buffered by addition of TES to a concentration of 10 mM in the medium. Optionally DMSO (dimethyl sulfoxide) was added to a concentration of 1 % in the medium.

The feeding of cultures started at inoculation by adding the respective modified substrate(s) to a concentration of 10 µM in the medium. Daily additions of modified substrates remained constant over 4 days by feeding of additional 10 µM per day (day 1-4). Alternatively, additions of modified substrate(s) were done on day one and day three after inoculation by feeding of the modified substrate(s) to a concentration of 30 µM in the medium at each of the days. Growth of cultures was monitored daily by measurements of optical density at 750 nm (OD750 nm). Cultivation was finished by adding methanol to the culture to an end concentration of 20 %. Subsequently extraction was done via a standard solid phase extraction procedure using C18-modified silica cartridges.

For other scales mentioned above the protocols were similar and only slightly varied. For example, at 2 and 20 L scale the medium was not always buffered and due to the slower growth rate the duration of cultivation was prolonged for another week. Furthermore, in some cases increased amounts of added modified substrates up to 300 µM media concentration were used (if strain tolerated such concentrations) in order to increase the yield of modified non-ribosomal peptides.

**Table: Recipe for BG11 medium which has been used for feeding experiments**

| **Component** | **mg/L** | **mM** |
|---|---|---|
| **NaNO₃** | 1500 | 17,6 |
| **K₂HPO₄**^{∗}**3H₂O** | 40 | 0,23 |
| **MgSO₄**^{∗}**7H₂O** | 75 | 0,3 |
| **CaCl₂**^{∗}**2H₂O** | 36 | 0,24 |
| **Na₂CO₃** | 20 | 0,19 |
| **Ferric ammon.citrate** | 6 | 0,021 |
| **Citric acid** | 6 | 0,031 |
| **Na₂EDTA**^{∗}**2H₂O** | 1 | 0,0027 |

| **Trace elements** | **µg/L** | **µM** |
|---|---|---|
| **H₃BO₃** | 2.86 | 46.3 |
| **MnCl₂·4H₂O** | 1.8 | 9.15 |
| **ZnSO₄·7H₂O** | 0.22 | 0.77 |
| **Na₂MoO₄·2H₂O** | 0.390 | 1.61 |
| **CuSO₄·5H₂O** | 0.079 | 0.32 |
| **Co(NO₃)₂·6H₂O** | 0.0494 | 0.17 |

For the following strains feeding of at least one modified and clickable substrate were demonstrated.

| **Cyano Biotech Strain ID No.** | **Genera** | **Main non-ribosomal peptide variants produced** |
|---|---|---|
| 1 | Microcystis | MC-YR |
| 265 | Microcystis | MC-LR |
| | | MC-LR, Cyanopeptolin A, B, C, D und 963A; Microcyclamide, Aeruginosin, Aerucyclamide A, B, C, D |
| 275 | Microcystis | MC-LR |
| | | MC-LW |
| | | MC-LF |
| 280 | Planktothrix | MC-LR |
| 329 | Planktothrix | (D-Asp3, Dhb7)MC-RR |
| 332 | Planktothrix | (D-Asp3, Dhb7)MC-RR, Anabaenopeptin A, B, E/F, NZ867 |
| 480 | Microcystis | MC-LR |
| | | MC-YR |
| 633 | Microcystis | MC-RR |
| 786 | Nodularia | NOD |
| 861 | Microcystis | MC-RY |
| | | MC-LY |
| 959 | Microcystis | MC-LR |
| | | MC-YR |
| 1161 | Planktothrix | (D-Asp3, E-Dhb7)MC-RR |
| | | Anabaenopeptin A,E/F,B |
| | | Oscillamide Y |

MC is microcystin, the two letters behind MC define the amino acids at the variable positions 2 and 4 whereas R is arginine, Y is tyrosine, L is leucine, W is tryptophan, and F ist phenylalanine. D-MAsp3 is D-erythro-β-methylaspartic acid at position 3 and Dhb7 is dehydrobutyrate at position 7. NOD is Nodularin.

Figure 4 - 30 illustrate incorporations of modified amino acids into non-ribosomal peptides, more specific into microcystins, nodularins, anabaenopeptins and oscillamides at different positions and produced by different genera and strains, resp. which carry clickable anchor groups or anchor groups that are easily accessible to additional modification towards conjugable anchor groups.

The following tables summarize results of feeding experiments of different cyanobacterial genera and strains, resp. with one or two modified substrates each comprising an anchor group directly accessible or transformable for use in conjugation chemistry (incl. click chemistry), for the attachment of a targeting moiety and/or a label via a linker or w/o a linker between the modified amino acid and the targeting moiety and/or a label.

**Table 1: Part 1 of summary of results of feeding one modified substrate to different cyanobacterial strains of the genera Microcystis and Planktothrix. MC - microcystin with lette behind MC indicating the amino acids at the variable position 2 and 4 in the one-letter-code.**

| **CBTstrain no.** | **Genera/ Species** | **NRP variants naturally produced by the strain** | **Naturally produced NRP variant which is effected by fed modified substrate** | **Sum formula of naturally produced NRP variant** | **Monoiso-topic mass of naturally produced NRP variant** | **Naturally incorporated amino acid which is replaced by modified substrate** | **Sum formula (zwitterion) of natural substrate** | **Monoisotopic mass (Zwitterion) of natural substrate** | **Position of naturally incor porated amino acid in NRP** | **Short name of modified substrate** | **Sum formula (zwitterion) of modified substrate** | **Monoisotopic mass (zwitter-ion) of modified substrate** | **Mass difference between natural und modified substrate (Da)** | **Calculated monoisotopic mass of mutasynthesis product (novel microcystin)** | **Measured monoisotopic mass of mutasyn-thesis product [M+H]+** | **MS Peak EIC (Mass spectrometry)** | **UV Peak PDA (HPLC)** |
|---|---|---|---|---|---|---|---|---|---|---|---|---|---|---|---|---|---|
| 1 | Microcystis sp. | MC-LR | MC-YR | C52H72N10O13 | 1044,528032 | Arg | C6H14N4O2 | 174,111679 | 2 | Azido-Lys | C6H12N4O2 | 172,0960 | 2,0157 | 1042,5124 | 1043,5197 | yes | yes |
| | | MC-YR | | | | | | | | | | | | | | | |
| 1 | Microcystis sp. | MC-LR | MC-YR | C52H72N10O13 | 1044,528032 | Tyr | C9H11NO3 | 181,073898 | 4 | Prg-Tyr | C12H13NO3 | 219,0895 | -38,0156 | 1082,5437 | 1083,5510 | yes | yes |
| | | MC-YR | | | | | | | | | | | | | | | |
| 1 | Microcystis sp. | MC-LR | MC-YR | C52H72N10O13 | 1044,528032 | Arg | C6H14N4O2 | 174,111679 | 2 | Nitro-Arg | C6H13N5O4 | 219,0968 | -44,9851 | 1089,5131 | 1090,5204 | yes | yes |
| | | MC-YR | | | | | | | | | | | | | | | |
| 1 | Microcystis sp. | MC-LR | MC-YR | C52H72N10O13 | 1044,528032 | Tyr | C9H11NO3 | 181,073898 | 4 | Azido-L-Phe | C9H10N4O2 | 206,0804 | -25,0065 | 1069,5345 | 1070,5418 | yes | yes |
| | | MC-YR | | | | | | | | | | | | | | | |
| 265 | Microcystis aeruginosa | MC-LR, Cyanopeptolin A, B, C, D und 963A; Microcyclamide Aeruginosin, AerucyclamideA, B, C, D | MC-LR | C49H74N10O12 | 994,548767 | Arg | C6H14N4O2 | 174,111679 | 4 | Prg-Tyr | C12H13N03 | 219,0895 | -44,9779 | 1039,5266 | 1040,5339 | yes | no |
| 265 | Microcystis aeruginosa | MC-LR, Cyanopeptolin A, B, C, D und 963A; Microcyclamide Aeruginosin, AerucyclamideA, B, C, D | MC-LR | C49H74N10O12 | 994,548767 | Leu | C6H13NO2 | 131,094635 | 2 | Prg-Tyr | C12H13N03 | 219,0895 | -87,9949 | 1082,5437 | 1083,5510 | yes | no |
| 275 | Microcystis aeruginosa | MC-LR | MC-LR | C49H74N10O12 | 994,548767 | Arg | C6H14N4O2 | 174,111679 | 4 | Nitro-Arg | C6H13N5O4 | 219,0968 | -44,9851 | 1039,5338 | 1040,5411 | yes | undetermined |
| | | MC-LW | | | | | | | | | | | | | | | |
| | | MC-LF | | | | | | | | | | | | | | | |
| 275 | Microcystis aeruginosa | MC-LR | MC-LR | C49H74N10O12 | 994,548767 | Arg | C6H14N4O2 | 174,111679 | 4 | Furyl-Ala | C7H9NO3 | 155,0582 | 19,0534 | 975,4953 | 976,5026 | yes | undetermined |
| | | MC-LW | | | | | | | | | | | | | | | |
| | | MC-LF | | | | | | | | | | | | | | | |
| 275 | Microcystis aeruginosa | MC-LR | MC-LR | C49H74N10O12 | 994,548767 | Arg | C6H14N4O2 | 174,111679 | 4 | Lys(Poc) | C10H16N2O4 | 228,1110 | -53,9993 | 1048,5481 | 1049,5554 | yes | undetermined |
| | | MC-LW | | | | | | | | | | | | | | | |
| | | MC-LF | | | | | | | | | | | | | | | |
| 275 | Microcystis aeruginosa | MC-LR | MC-LF | C52H71N7O12 | 985,5160707 | Phe | C9H11NO2 | 165,0789786 | 4 | Prg-Tyr | C12H13N03 | 219,0895 | -54,0106 | 1039,5266 | 1040,5339 | yes | yes |
| | | MC-LW | | | | | | | | | | | | | | | |
| | | MC-LF | | | | | | | | | | | | | | | |
| 275 | Microcystis aeruginosa | MC-LR | MC-LF | C52H71N7O12 | 985,5160707 | Phe | C9H11NO2 | 165,0789786 | 4 | Azido-L-Phe | C9H10N4O2 | 206,0804 | -41,0014 | 1026,5175 | 1027,5247 | yes | yes |
| | | MC-LW | | | | | | | | | | | | | | | |
| | | MC-LF | | | | | | | | | | | | | | | |
| 275 | Microcystis aeruginosa | MC-LR | MC-LR | C49H74N10O12 | 994,548767 | Arg | C6H14N4O2 | 174,111679 | 4 | Azido-Lys | C6H12N4O2 | 172,0960 | 2,0157 | 992,5331 | 993,5404 | yes | yes |
| | | MC-LW | | | | | | | | | | | | | | | |
| | | MC-LF | | | | | | | | | | | | | | | |
| 275 | Microcystis aeruginosa | MC-LR | MC-LR | C49H74N10O12 | 994,548767 | Arg | C6H14N4O2 | 174,111679 | 4 | Azido-Norval | C5H10N4O2 | 158,0804 | 16,0313 | 978,5175 | 979,5247 | yes | yes |
| | | MC-LW | | | | | | | | | | | | | | | |
| | | MC-LF | | | | | | | | | | | | | | | |
| 275 | Microcystis aeruginosa | MC-LR | MC-LW | C54H72N8O12 | 1024,52697 | Trp | C11H12N2O2 | 204,0898776 | 4 | Prg-Tyr | C12H13N03 | 219,0895 | -14,9997 | 1039,5266 | 1040,5339 | yes | yes |
| | | MC-LW | | | | | | | | | | | | | | | |
| | | MC-LF | | | | | | | | | | | | | | | |
| 275 | Microcystis aeruginosa | MC-LR | MC-LW | C54H72N8O12 | 1024,52697 | Trp | C11H12N2O2 | 204,0898776 | 4 | Azido-L-Phe | C9H10N4O2 | 206,0804 | -1,9905 | 1026,5175 | 1027,5247 | yes | yes |
| | | MC-LW | | | | | | | | | | | | | | | |
| | | MC-LF | | | | | | | | | | | | | | | |

**Table 2: Part 2 of summary of results of feeding one modified substrate to different cyanobacterial strains of the genera Microcystis and Planktothrix. MC - microcystin with letters behind MC indicating the amino acids at the variable position 2 and 4 in the one-letter-code.**

| **CBTstrain no.** | **Genera/ Species** | **NRP variants naturally produced by the strain** | **Naturally produced NRP variant which is effected by fed modified substrate** | **Sum formula of naturally produced NRP variant** | **Monoiso-topic mass of naturally produced NRP variant** | **Naturally incorporated amino acid which is replaced by modified substrate** | **Sum formula (zwitterion) of natural substrate** | **Monoisotopic mass (Zwitterion) of natural substrate** | **Position of naturally incor porated amino acid in NRP** | **Short name of modified substrate** | **Sum formula (zwitterion) of modified substrate** | **Monoisotopic mass (zwitter-ion) of modified substrate** | **Mass difference between natural und modified substrate** (Da) | **Calculated monoisotopic mass of mutasynthesis product (novel microcystin)** | **Measured monoisotopic mass of mutasyn-thesis product [M+H]+** | **MS Peak EIC (Mass spectrometry)** | **UV Peak PDA (HPLC)** |
|---|---|---|---|---|---|---|---|---|---|---|---|---|---|---|---|---|---|
| 329 | Planktothrix agardhii | (D-Asp3, E-Dhb7)MC-RR | (D-Asp3, E-Dhb7)MC-RR | C48H73N13O12 | 1023,5502 | Arg | C6H14N4O2 | 174,111679 | 4 | Nitro-Arg | C6H13N5O4 | 219,0968 | -44,9851 | 1068,5353 | 1069,5426 | yes | yes |
| 332 | Planktothrix rubescens | Anabaenopeptin A, B, E/Fund NZ857, (D-Asp3,E-Dhb7)MC-RR | (D-Asp3, E-Dhb7)MC-RR | C48H73N13O12 | 1023,5502 | Arg | C6H14N4O2 | 174,111679 | 2 | Prg-Tyr | C12H13NO3 | 219,0895 | -44,9779 | 1068,5281 | 1069,5353 | yes | yes |
| 480 | Microcystis aeruginosa | MC-LR (D-Asp3)MC-YR | MC-LR | C49H74N10O12 | 994,548767 | Leu | C6H13NO2 | 131,094635 | 2 | Azido-Norval | CSH10N4O2 | 158,0804 | -26,9857 | 1021,5345 | 1022,5418 | yes | undetermined |
| 480 | Microcystis aeruginosa | MC-LR (D-Asp3)MC-YR | MC-LR | C49H74N10O12 | 994,548767 | Arg | C6H14N4O2 | 174,111679 | 4 | Nitro-Arg | C6H13N5O4 | 219,0968 | -44,9851 | 1039,5338 | 1040,5411 | yes | yes |
| 480 | Microcystis aeruginosa | MC-LR (D-Asp3)MC-YR | MC-LR | C49H74N10O12 | 994,548767 | Arg | C6H14N4O2 | 174,111679 | 4 | Azido-Lys | C6H12N4O2 | 172,0960 | 2,0157 | 992,5331 | 993,5404 | yes | yes |
| 480 | Microcystis aeruginosa | MC-LR (D-Asp3)MC-YR | MC-YR | C52H72N10O13 | 1044,528032 | Arg | C6H14N4O2 | 174,111679 | 4 | Azido-Lys | C6H12N4O2 | 172,0960 | 2,0157 | 1042,5124 | 1043,5197 | yes | yes |
| 480 | Microcystis aeruginosa | MC-LR (D-Asp3)MC-YR | MC-YR | C52H72N10O13 | 1044,528032 | Tyr | C9H11NO3 | 181,073898 | 2 | Prg-Tyr | C12H13NO3 | 219,0895 | -38,0156 | 1082,5437 | 1083,5510 | yes | yes |
| 480 | Microcystis aeruginosa | MC-LR (D-Asp3)MC-YR | MC-YR | C52H72N10O13 | 1044,528032 | Arg | C6H14N4O2 | 174,111679 | 4 | Nitro-Arg | C6H13N5O4 | 219,0968 | -44,9851 | 1089,5131 | 1090,5204 | yes | yes |
| 480 | Microcystis aeruginosa | MC-LR (D-Asp3)MC-YR | MC-YR | C52H72N10O13 | 1044,528032 | Tyr | C9H11NO3 | 181,073898 | 2 | Azido-L-Phe | C9H10N4O2 | 206,0804 | -25,0065 | 1069,5345 | 1070,5418 | yes | yes |
| 480 | Microcystis aeruginosa | MC-LR (D-Asp3)MC-YR | MC-YR | C52H72N10O13 | 1044,528032 | Tyr | C9H11NO3 | 181,073898 | 2 | Prg-Tyr | C12H13NO3 | 219,0895 | -38,0156 | 1082,5437 | 1083,5510 | yes | yes |
| 480 | Microcystis aeruginosa | MC-LR (D-Asp3)MC-YR | MC-LR | C49H74N10O12 | 994,548767 | Leu | C6H13NO2 | 131,094635 | 2 | Azido-Lys | C6H12N4O2 | 172,0960 | -41,0014 | 1035,5502 | 1036,5574 | yes | yes |
| 480 | Microcystis aeruginosa | MC-LR (D-Asp3)MC-YR | MC-YR | C52H72N10O13 | 1044,528032 | Tyr | C9H11NO3 | 181,073898 | 2 | Azido-Lys | C6H12N4O2 | 172,0960 | 8,9779 | 1035,5502 | 1036,5574 | yes | yes |

**Table 3: Part 3 of summary of results of feeding one modified substrate to different cyanobacterial strains of the genera Microcystis and Planktothrix. MC - microcystin with letter behind MC indicating the amino acids at the variable position 2 and 4 in the one-letter-code**

| **CBTstrain no.** | **Genera/ Species** | **NRP variants naturally produced by the strain** | **Naturally produced NRP variant which is effected by fed modified substrate** | **Sum formula of naturally produced NRP variant** | **Monoiso-topic mass of naturally produced NRP variant** | **Naturally incorporated amino acid which is replaced by modified substrate** | **Sum formula (zwitterion) of natural substrate** | **Monoisotopic mass (Zwitterion) of natural substrate** | **Position of naturally incor porated amino acid in NRP** | **Short name of modified substrate** | **Sum formula (zwitterion) of modified substrate** | **Monoisotopic mass (zwitter-ion) of modified substrate** | **Mass difference between natural und modified substrate (Da)** | **Calculated monoisotopic mass of mutasynthesis product (novel microcystin)** | **Measured monoisotopic mass of mutasynthesis product [M+H]+** | **MS Peak EIC (Mass spectrometry)** | **UV Peak PDA (HPLC)** |
|---|---|---|---|---|---|---|---|---|---|---|---|---|---|---|---|---|---|
| 633 | Microcystis sp. | MC-RR | MC-RR | C49H75N13O12 | 1037,5658 | Arg | C6H14N4O2 | 174,111679 | 2 | Azido-L-Ala | C3H6N4O2 | 130,0491 | 44,0626 | 993,5032 | 994,5105 | yes | yes |
| 633 | Microcystis sp. | MC-RR | MC-RR | C49H75N13O12 | 1037,5658 | Arg | C6H14N4O2 | 174,111679 | 2 | Azido-Norval | CSH10N4O2 | 158,0804 | 16,0313 | 1021,5345 | 1022,5418 | yes | yes |
| 633 | Microcystis sp. | MC-RR | MC-RR | C49H75N13O12 | 1037,5658 | Arg | C6H14N4O2 | 174,111679 | 2 | Nitro-Arg | C6H13N5O4 | 219,0968 | -44,9851 | 1082,5509 | 1083,5582 | yes | yes |
| 633 | Microcystis sp. | MC-RR | MC-RR | C49H75N13O12 | 1037,5658 | Arg | C6H14N4O2 | 174,111679 | 2 | Azido-Lys | C6H12N4O2 | 172,0960 | 2,0157 | 1035,5501 | 1036,5574 | yes | yes |
| 786 | Nodularia sp. | NOD(MeAsp1?) | NOD(MeAspl?) | C41H60N8O10 | 824,4432402 | Arg | C7H16N4O2 | 174,111679 | 2 | H-homo-Arg-OH | C7H16N4O2 | 188,1273 | -14,0156 | 838,4589 | 839,4661 | yes | yes |
| 959 | Microcystis sp. | MC-LR | MC-LR | C49H74N10O12 | 994,548767 | Arg | C6H14N4O2 | 174,111679 | 4 | Nitro-Arg | C6H13N5O4 | 219,0968 | -44,9851 | 1039,5338 | 1040,5411 | yes | undetermined |
| | | MC-YR | | | | | | | | | | | | | | | |
| 959 | Microcystis sp. | MC-LR | MC-YR | C52H72N10O13 | 1044,528032 | Arg | C6H14N4O2 | 174,111679 | 4 | Nitro-Arg | C6H13N5O4 | 219,0968 | -44,9851 | 1089,5131 | 1090,5204 | yes | undetermined |
| | | MC-YR | | | | | | | | | | | | | | | |
| 959 | Microcystis sp. | MC-LR | MC-LR | C49H74N10O12 | 994,548767 | Arg | C6H14N4O2 | 174,111679 | 4 | Azi do-L-Al a | C3H6N4O2 | 130,0491 | 44,0626 | 950,4862 | 951,4934 | yes | yes |
| | | MC-YR | | | | | | | | | | | | | | | |
| 959 | Microcystis sp. | MC-LR | MC-YR | C52H72N10O13 | 1044,528032 | Arg | C6H14N4O2 | 174,111679 | 4 | Azi do-L-Al a | C3H6N4O2 | 130,0491 | 44,0626 | 1000,4654 | 1001,4727 | yes | yes |
| | | MC-YR | | | | | | | | | | | | | | | |
| 959 | Microcystis sp. | MC-LR | MC-LR | C49H74N10O12 | 994,548767 | Arg | C6H14N4O2 | 174,111679 | 4 | Azido-Norval | CSH10N4O2 | 158,0804 | 16,0313 | 978,5175 | 979,5247 | yes | yes |
| | | MC-YR | | | | | | | | | | | | | | | |
| 959 | Microcystis sp. | MC-LR | MC-YR | C52H72N10O13 | 1044,528032 | Arg | C6H14N4O2 | 174,111679 | 4 | Azido-Norval | CSH10N4O2 | 158,0804 | 16,0313 | 1028,4967 | 1029,5040 | yes | yes |
| | | MC-YR | | | | | | | | | | | | | | | |
| 959 | Microcystis sp. | MC-LR | MC-YR | C52H72N10O13 | 1044,528032 | Tyr | C9H11NO3 | 181,073898 | 2 | Prg-Tyr | C12H13NO3 | 219,0895 | -38,0156 | 1082,5437 | 1083,5510 | yes | yes |
| | | MC-YR | | | | | | | | | | | | | | | |
| 959 | Microcystis sp. | MC-LR | MC-YR | C52H72N10O13 | 1044,528032 | Tyr | C9H11NO3 | 181,073898 | 2 | Azido-L-Phe | C9H10N4O2 | 206,0804 | -25,0065 | 1069,5345 | 1070,5418 | yes | yes |
| | | MC-YR | | | | | | | | | | | | | | | |
| 1161 | Planktothrix rubescens | (D-Asp3, E-Dhb7)MC-RR, Anabaenopepti n A, B, E/F, Oscillamide Y | (D-Asp3, E-Dhb7)MC-RR | C48H73N13O12 | 1023,5502 | Arg | C6H14N4O2 | 174,111679 | 2 | Prg-Tyr | C12H13NO3 | 219,0895 | -44,9779 | 1068,5281 | 1069,5353 | yes | yes |
| 861R | Microcystis aeruginosa | MC-LY | MC-LY | C52H71N7O13 | 1001,510985 | Leu | C6H13NO2 | 131,094635 | 2 | Azido-Lys | C6H12N4O2 | 172,0960 | -41,0014 | 1042,5124 | 1043,5197 | yes | yes |
| | | MC-RY | | | | | | | | | | | | | | | |
| 861R | Microcystis aeruginosa | MC-LY | MC-RY | C52H72N10O13 | 1044,528032 | Arg | C6H14N4O2 | 174,111679 | 2 | Azido-Lys | C6H12N4O2 | 172,0960 | 2,0157 | 1042,5124 | 1043,5197 | yes | yes |
| | | MC-RY | | | | | | | | | | | | | | | |
| 861R | Microcystis aeruginosa | MC-LY | MC-RY | C52H72N10O13 | 1044,528032 | Tyr | C9H11NO3 | 181,073898 | 4 | Azido-Lys | C6H12N4O2 | 172,0960 | 8,9779 | 1035,5502 | 1036,5574 | yes | yes |
| | | MC-RY | | | | | | | | | | | | | | | |

**Table 4: Summary of results of feeding two modified substrates to different cyanobacterial culture of the genera Microcystis. MC - microcystin with letters behind MC indicating the amino acids at the variable position 2 and 4 in the one-letter-code.**

| **CBT strain no.** | **Genera/ Species** | **Microcystin variants naturally produced by the strain** | **Monoisotopic mass of naturally produced microcystin variant which is effected by the substrates** | **Naturally incorporated amino acid which is replaced by modified substrate 1 (position in MC)** | **Naturally incorporated amino acid which is replaced by modified substrate 2 (position in MC)** | **Short names of modified substrate 1** | **Monoisotopic mass (zwitterion) of modified substrate 1** | **Short names of modified substrate 2** | **Monoisotopic mass (zwitterion) of modified substrate 2** | **Mass difference between natural und modified substrate 1 (Da)** | **Mass difference between natural und modified substrate 2 (Da)** | **Calculated monoisotopic mass of mutasynthesis product (novel microcystin)** | **Measured monoisotopic mass of mutasynthesis product [M+H]+** | **MS Peak EIC (Mass spectro-metry)** | **UV Peak PDA (HPLC)** |
|---|---|---|---|---|---|---|---|---|---|---|---|---|---|---|---|
| 1 | Microcystis sp | MC-YR | 1044,528032 | Tyr(pos.4) | Arg(pos.2) | Prg-Tyr | 219,0895433 | Nitro-Arg | 219,0967539 | -38,0156453 | -44,985 | 1127,5288 | 1128,5360 | yes | yes |
| 1 | Microcystis sp. | MC-YR | 1044,528032 | Arg(pos.2) | Tyr(pos.4) | Nitro-Arg | 219,0967539 | Azido-L-Phe | 206,0803756 | -44,9850749 | -25,00647758 | 1114,5196 | 1115,5269 | yes | yes |
| 480 | Microcystis aeruginosa | MC-LR (D-Asp3)MC-YR | 1044,528032 | Arg(pos.4) | Tyr (pos.2) | Nitro-Arg | 219,0967539 | Prg-Tyr | 219,0895433 | -44,9850749 | -38,01564528 | 1127,5288 | 1128,5360 | yes | yes |
| 480 | Microcystis aeruginosa | MC-LR (D-Asp3)MC-YR | 1044,528032 | Arg(pos.4) | Tyr (pos.2) | Nitro-Arg | 219,0967539 | Azido-L-Phe | 206,0803756 | -44,9850749 | -25,00647758 | 1114,5196 | 1115,5269 | yes | yes |

### FIGURE CAPTIONS

**FIG. 1****:**
Upper left: General structure of Microcystins. X₂ and Z₄ indicate variable L-amino acids. D-Ala = D-Alanine, D-Me-Asp = D-methyl aspartic acid, Arg = Arginine, Adda = 3-amino-9-methoxy-2,6,8-trimethyl-10-phenyldeca-4,6-dienoic acid, D-Glu = D-glutamic acid, Mdha = N-methyldehydroalanine. Upper right: General structure of Nodularins. Arg₂ indicates the variable L-amino acid corresponding to Z₄ in the microcystin molecule. D-Me-Asp = D-methyl aspartic acid, Arg = Arginine, Adda = 3-amino-9-methoxy-2,6,8-trimethyl-10-phenyldeca-4,6-dienoic acid, D-Glu = D-glutamic acid, Mdhb = N-methyldehydrobutyrate.
Down right: General structure of anabaenopeptin A and schematic general structure of anabaenopeptin type peptides (incl. oscillamides): Anabaenopeptins (and oscillamides) are cyclic peptides that are characterized by a lysine in position 5 and the formation of the ring by an N-6-peptide bond between Lys and the carboxy group of the amino acid in position 6 A side chain of one amino acid unit is attached to the ring by an ureido bond formed between the a-N of Lys and the a-N of the side chain amino acid. All other positions in the ring and side chain are variable.
Down right: Chemical structure of cryptophycin-1. Cryptophycins are a class of macrocyclic depsipeptides produced as secondary metabolites by cyanobacteria of the genus Nostoc. Isolation of the first representative, cryptophycin-1, from cultivated Nostoc species ATCC 53789 was published in 1990 by researchers at Merck.

Fig. 2:
Comparison between different cultivation systems and scales and different mass spectrometry detections in the context of suitable screening approaches towards strains that are suited for feeding of modified substrates for modifying non-ribosomal peptides including CA (fig. 2A to 2 D)
**Fig. 2** **A:**
Detection of modified microcystins by two different mass spectrometry method after feeding of modified substrates to a Microcystis aeruginosa strain CBT 480 in a 50 ml scale (above of each of the four figures A, B, C, D detection with ESI-IT-ToF-MS; below of each of the four figures A, B, C, D detection with MALDI-ToF-MS).

| | |
|---|---|
| A: Control (no feeding with O-methyltyrosine) | B: Control (no feeding with homoarginine) |
| C: Feeding with O-methyltyrosine | D: Feeding with homoarginine |

Molecule masses of naturally produced microcystins:
995 Da = MC-LR, 1045 Da = MC-YR
Molecule masses of modified microcystins generated by feeding with O-methyltyrosine (OMetY) and homoarginine (hR)
1059 Da = MC-OMetYR or MC-YhR; 1009 Da = MC-LhR

**Fig. 2** **B:**
Detection of modified microcystins by two different mass spectrometry method after feeding of modified substrates to a Microcystis aeruginosa strain CBT 480 in a 6 ml scale (above of each of the two figures A/A' and B/B' detection with ESI-IT-ToF-MS; below of each of the two figures A/A' and B/B' detection with MALDI-ToF-MS).

| | |
|---|---|
| **A, A'**: CBT 480 culture fed with O-methyltyrosine | **B, B':** CBT 480 culture fed with homoarginine |

Molecule masses of naturally produced microcystins:
995Da = MC-LR, 1045 Da = MC-YR
Molecule masses of modified microcystins generated by feeding with O-methyltyrosine (OMetY) and homoarginine (hR)
1059 Da = MC-OMetYR or MC-YhR; 1009 Da = MC-LhR;

**Fig. 2** **C:**
Detection of modified microcystins by two different mass spectrometry method after feeding of modified substrates to a Microcystis aeruginosa strain CBT 480 with O-methyltyrosine in a 1.6 ml (dw-MTP) scale (ESI-IT-ToF-MS on the left; MALDI-ToF-MS on the right)
A, A': feeding of 300 µM O-methyltyrosine (OMetY), w/o DMSO
B, B': feeding of 30 µM O-methyltyrosine (O-MetY), w/o DMSO
C, C': feeding of 300 µM O-methyltyrosine (OMetY), w/ 1 % DMSO
D, D': feeding of 30 µM O-methyltyrosine (OMetY), w/ 1 % DMSO
E, E': control (no feeding)
Molecule masses of naturally produced microcystins:
   995Da = MC-LR, 1045 Da = MC-YR
   Molecule masses of modified microcystin generated by feeding with O-methyltyrosine 1059 Da = MC-OMetYR

**Fig. 2** **D:**
Detection of modified microcystins by two different mass spectrometry method after feeding of modified substrates to a Microcystis aeruginosa strain CBT 480 with homoarginine in a 1.6 ml (dw-MTP) sclale (ESI-IT-ToF-MS detection on the left; MALDI-ToF-MS detection on the right)
A, A': feeding of 300 µM homoarginine (hR), w/o DMSO
B, B': feeding of 30 µM homoarginine (hR), w/o DMSO
C, C': feeding of 300 µM homoarginine (hR), w/ 1 % DMSO
D, D': feeding of 30 µM homoarginine (hR), w/ 1 % DMSO
E, E': control (no feeding)
Molecule masses of naturally produced microcystins:
   995Da = MC-LR, 1045 Da = MC-YR
   Molecule masses of modified microcystins generated by feeding with homoarginine 1059 Da = MC-YhR; 1009 Da = MC-LhR

All modified microcystins could be detected with both MS methods. However, most samples resulting from feeding without the addition of DMSO of 1 % in the culture medium could not be detected with MALDI-ToF-MS but with ESI-IT-ToF-MS. Therefore, it is recommended to use DMSO for feeding experiments in screenings of small scale cultures (between 1 and 10 ml culture volumes) especially if the MS detection of modified non-ribosomal peptides is based on MALDI-ToF-MS.
On the other side MALDI-ToF-MS detection of modified non-ribosomal peptides after feeding of modified substrates to small scale cultures of 1.6 ml cultivated in deep-well-microtiter plates (dw-MTW) allows for high throughput screening (HTS). Both cultivation (with and without feeding of modified substrates) and sample preparation for MALDI-ToF-MS can be done using a pipetting robot allowing for the parallel test of diverse strains and substrates as described in Tillich et al. BMC Microbiology 2014, 14:239.
**FIG. 3****:**
McyBI represent the first of two enzyme modules of McyB and is responsible for the incorporation of the amino acid at the position 2 of the microcystin molecule. This is the amino acid leucine in case of the Microcystin aeruginosa strain PCC7806 whereas it is leucine OR tyrosine in the Microcystis aeruginosa strain CBT 480. The so called core motifs A2-A6 of the adenylation (A) domain of McyBI are highlighted in black (A2-A6) and the amino acids responsible for substrate (amino acid) recognition and activation during the biosynthesis of the respective microcystin are indicated by big and bold white letters. These amino acids form the active pocket of the A domains and the sequence in their one-letter amino acid code represent the so called specificity-conferring code of A domains which shall allow for the prediction of substrate specificity of A domains. The box and the arrow indicate the only difference in the amino acid sequence of McyBI of both strains. Only one of nine pocket-forming amino acids of the A domains of both strains is different between the strains and also the remaining parts of the A domain as well as of the whole biosynthetic gene clusters are almost identical between the strains leading to the conclusion that the incorporation of leucine and tyrosine at position 2 of the microcystin in the strain CBT 480 is a strain-specific feature but cannot be explained by differences in the DNA sequence of the biosynthetic gene clusters and amino acid sequence of the microcystin synthetases, resp.
**FIG. 4****:**
Exemplary embodiment No. 1: Incorporation of the modified substrate Azido-L-Phe (Phe = phenylalanine) into Microcystin-YR in position 2 produced by strain CBT 959. HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and for sample of cultivation with added modified substrate (d) in the positive ionization mode. Finally, (e) shows the averaged mass spectrum of the peak visible in chromatogram d). Detector signal intensities (y-Axis) are measured in milli-absorption units (mAU) und counts (dimensionless quantity) for PDA and mass spectrometry data, respectively.
The growth of strain CBT 959 could not be followed by measurement of optical density at 750 nm (OD_{750 nm}) as the cell formed aggregates making it impossible to measure reliable oD_{750 nm} values.

FIG. 5:
Exemplary embodiment No. 2: Incorporation of the modified substrate Prg-Tyr (Tyr=Tyrosine) into Microcystin YR in position 2 produced by strain CBT 480.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**FIG. 6****:**
Exemplary embodiment No. 2: Growths curve of CBT 480 cultures with and without Prg-Tyr (Tyr=Tyrosine) added.
FIG. 7:
Exemplary embodiment No. 3: Incorporation of the modified substrate Azido-Lys (Lys=Lysine) into Microcystin LR in position 4 produced by strain CBT 275.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

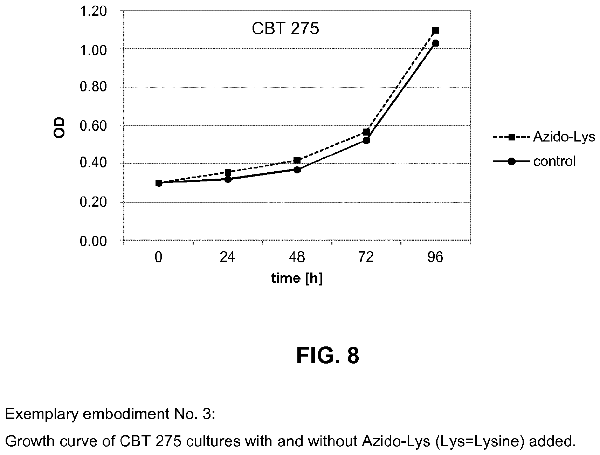

**FIG. 8****:** Exemplary embodiment No. 3: Growths curve of CBT 275 cultures with and without Azido-Lys (Lys=Lysine) added.
**FIG. 9****:**
Exemplary embodiment No. 4: Incorporation of the modified substrate Prg-Tyr (Tyr=Tyrosine) into Microcystin LW in position 4 produced by strain CBT 275.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**FIG. 10****:**
Exemplary embodiment No. 4: Growths curve of CBT 275 cultures with and without Prg-Tyr (Tyr=Tyrosine) added.
**FIG. 11****:**
Exemplary embodiment No. 5: Incorporation of the modified substrate Nitro-Arg (Arg=Arginine) into Microcystin YR in position 4 produced by strain CBT 1.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**FIG. 12****:**
Growths curve of CBT 1 cultures with and without Nitro-Arg (Arg=Arginine) added.
**Fig. 13****:**
Exemplary embodiment No. 6: Incorporation of the modified substrate Furyl-L-Ala (Ala=Alanine) into Microcystin LR in position 4 produced by strain CBT 275.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively. The PDA-Signal of the novel Furyl-Ala variant of Microcystin LR is not visible due to the low concentration.

**Fig. 14****:**
Exemplary embodiment No. 6: Growths curve of CBT 275 cultures with and without Furyl-Ala (Ala = Alanine) added.
**Fig. 15****:**
Exemplary embodiment No. 7: Incorporation of the modified substrate Nitro-Arg (Arg=Arginine) and Prg-Tyr (Tyr=Tyrosine) into Microcystin YR in position 2 and 4 respectively produced by strain CBT 480.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 16****:**
Exemplary embodiment No. 7: Growths curve of CBT 480 cultures with and without Nitro-Arg (Arg=Arginine) and Prg-Tyr (Tyr=Tyrosine) added.
**Fig. 17****:**
Exemplary embodiment No. 8: Incorporation of the modified substrate Nitro-Arg (Arg=Arginine) into Microcystin (D-Asp3, E-Dhb7)-RR in position 2 / 4 produced by strain CBT 329.
HPLC-PDA Chromatogram at 238 nm for sample of control cultivation (a) for sample of cultivation with added modified substrate (b). Extracted ion chromatogram from HPLC-MS data of mass value of double protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 18****:**
Exemplary embodiment No. 8: Growths curve of CBT 329 cultures with and without Nitro-Arg (Arg=Arginine) added.
**Fig. 19****:**
Exemplary embodiment No. 9: Incorporation of the modified substrate Azido-Lys (Lys=Lysine)into Microcystin YR in position 4 produced by strain CBT 1.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively. The PDA-Signal of the novel Azido-Lys (Lys=Lysine)variant of Microcystin YR is not visible due to overlapping peaks in the sample.

**Fig. 20****:**
Exemplary embodiment No. 9: Growths curve of CBT 1 cultures with and without Azido-Lys (Lys=Lysine) added.
**Fig. 21****:**
Exemplary embodiment No. 10: Incorporation of the modified substrate Azido-Norval (Norval=Norvaline) into Microcystin RR in position 2 produced by strain CBT 633.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 22****:**
Growths curve of CBT 633 cultures with and without Azido-Norval (Norval=Norvaline) added.
**Fig. 23****:**
Exemplary embodiment No. 11: Incorporation of the modified substrate H-homoarg-OH (homoarg=homoarginine) into Nodularin in position 2 produced by strain CBT 786.
HPLC-PDA Chromatogram at 238 nm for sample of control cultivation (a) for sample of cultivation with added modified substrate (b). Extracted ion chromatogram from HPLC-MS data of mass value of protonated molecular ion of novel Nodularin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 24****:**
Exemplary embodiment No. 12: Incorporation of the modified substrate Azido-L-Phe (Phe = phenylalanine) into Microcystin YR in position 2 produced by strain CBT 480 in a large scale (2 I) cultivation system.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 25****:**
Exemplary embodiment No. 13: Feeding of Microcystis aeruginosa strain CBT 480 with different amounts of modified substrate 4-azido-L-phenylalanine (0 µM, 10 µM, 30 µM) results an increasing amount of produced modified microcystin with increasing amount of fed modified substrate 4-azido-L-phenylalanine. This result allows for optimization of feeding protocols for respective productions of modified non-ribosomal peptides (here modified microcystins).
The upper part of the figure shoes overlaid HPLC-PDA Chromatograms at 238 nm for sample of control cultivation, sample of cultivation with added substrate 4-azido-L-phenylalanine of 10 µM in culture medium and sample of cultivation with added substrate 4-azido-L-phenylalanine of 30 µM in culture medium. The lower figure shows the averaged mass spectrum of the newly formed peak visible at about 10 min in the HPLC chromatogram. Detector signal intensities (y-Axis) are measured in milli-absorption units (mAU) and counts (dimensionless quantity) for PDA and mass spectrometry data, respectively.

**Fig. 26****:**
Exemplary embodiment No. 14 : Incorporation of the modified substrate Prg-Tyr (Tyr=Tyrosine) into (D-Asp³, E-Dhb⁷)Microcystin-RR in position 2 produced by strain CBT 280.
HPLC-PDA Chromatogram at 238 nm for sample of control cultivation (a) for sample of cultivation with added modified substrate (b). Extracted ion chromatogram from HPLC-MS data of mass value of protonated molecular ion of novel Microcystin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 27****:**
Exemplary embodiment No. 15 : Incorporation of the modified substrate Prg-Tyr (Tyr=Tyrosine) into Anabaenopeptin A in position 2 produced by strain CBT 280.
HPLC-PDA Chromatogram at 210 nm for sample of control cultivation (a) for sample of cultivation with added modified substrate (b). Extracted ion chromatogram from HPLC-MS data of mass value of protonated molecular ion of novel Anabaenopeptin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 28****:**
Exemplary embodiment No. 16 : Incorporation of the modified substrate Azido-Phe (Phe=Phenylalanine) into Anabaenopeptin NZ857 produced by strain CBT 332.
HPLC-PDA Chromatogram at 210 nm for sample of control cultivation (a) for sample of cultivation with added modified substrate (b). Extracted ion chromatogram from HPLC-MS data of mass value of protonated molecular ion of novel Anabaenopeptin variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 29****:**
Exemplary embodiment No. 17 : Incorporation of the modified substrate Azido-Phe (Phe=Phenylalanine) into Oscillamide Y produced by strain CBT 1161.
HPLC-PDA Chromatogram at 210 nm for sample of control cultivation (a) for sample of cultivation with added modified substrate (b). Extracted ion chromatogram from HPLC-MS data of mass value of protonated molecular ion of novel Oscillamide variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 30****:**
Exemplary embodiment No. 18 : Incorporation of the modified substrate Prg-Tyr (Tyr=Tyrosine) into Oscillamide Y produced by strain CBT 1161.
HPLC-PDA Chromatogram at 210 nm for sample of control cultivation (a) for sample of cultivation with added modified substrate (b). Extracted ion chromatogram from HPLC-MS data of mass value of protonated molecular ion of novel Oscillamide variant for sample of control cultivation (c) and sample of cultivation with added modified substrate (d) in the positive ionization mode. Finally, e) shows the averaged mass spectrum of the peak visible in chromatogram d). Detector signal intensities (y-Axis) are measured in milli-absorption units (mAU) und counts (dimensionless quantity) for PDA and mass spectrometry data respectively.

**Fig. 31****:**
Exemplary embodiment No. 19 : Incorporation of the modified substrate Prg-Tyr (Tyr=Tyrosine) into Cryptophycin 1 produced by strain CBT 567.
Extracted ion chromatogram from HPLC-MS data of mass value of protonated molecular ion of novel Cryptophycin variant for sample of control cultivation (a) and sample of cultivation with added modified substrate (b) in the positive ionization mode. Finally, c) shows the averaged mass spectrum of the additional peak in chromatogram b). Detector signal intensities (y-Axis) are measured in counts (dimensionless quantity).

**Fig. 32****:**
Exemplary embodiment No. 20: Produced ADCs and results of analytical SEC-HPLC. In analytical SEC-HPLCthe conjugates Microcystin-ADCl and Microcystin-ADC2 showed a high level of purity with 98.9% and 99.0% monomers. In both cases, aggregates and small fragments were detected with rates of 0.8% and 0.2%.
**Fig. 33****:**
Exemplary embodiment No. 21: Coomassie stained gel electrophoresis gels demonstrating the binding of Microcystin variants 1 and 2 as payloads on monoclonal antibodies. In Coomassie staining under reducing conditions all samples showed a signal for the heavy chain at app. 50 kDa and the light chain at app. 25 kDa. All conjugates showed an up-shift of the protein signal of the heavy and the light chain compared to the naked MAB indicating toxin conjugation to both antibody chains. For all ADCs a double-signal was detected for the light chain indicating both, conjugated and unconjugated species. In Coomassie staining under non-reducing conditions the naked antibody showed a double signal at app. 150 kDa for the intact antibody. The ADCs showed a variety of signals between 25 kDa and 150 kDa, since in both cases the toxin was conjugated to reduced interchain disulfides leading to instability of the antibody during incubation at 37°C.
**Fig. 34****:**
Exemplary embodiment No. 22: Successful in vitro proof of concept of Microcystin-based ADCs. The cell viability is monitored in an in-vitro-assay with a cancer cell line for the different concentrations of the Microcystin ADC for two Microcystin variants as payloads. The ADC carries a non-cleavable linker. For Microcystin-ADC-2 an EC₅₀ values of 220 pM was determined. Differences between structural payload variants underline huge potential of further structural optimizations.

## Claims

1. A method of producing a modified non-ribosomal peptide from cyanobacteria, comprising the steps of:
a) growing a non-ribosomal peptide producing cyanobacteria strain in culture media,
b) adding one or more modified substrates to said culture, and
c) growing the strain in the presence of said modified substrates,
d) wherein the modified substrate is a modified amino acid, which comprises an anchor group directly accessible or transformable for use in conjugation chemistry incl. click chemistry, for the attachment of a targeting moiety or a label.

2. The method according to claim 1, wherein the non-ribosomal peptide producing strain is selected such that the incorporation of the modified substrate into the non-ribosomal peptide occurs at a defined position.

3. The method according to claim 1 or 2, wherein the non-ribosomal peptide is microcystin and the one or more modified substrates are incorporated at any position other than Adda₅ and DGlu₆, which has the following general structure:
D-Ala₁-X₂-D-MeAsp₃-Z₄-Adda₅-DGlu₆-Mdha₇.

4. The method according to claim 3, wherein incorporation of said modified amino acid occurs in position X₂ and/or Z₄.

5. The method according to claim 1 or 2, wherein the non-ribosomal peptide is nodularin and the one or more modified substrates are incorporated at any position other than Adda₃ and DGlu₄, which has the following general structure:
D- MeAsp₁-Arg₂-Adda₃-DGlu₄-Mdhb₅.

6. The method according to claim 5, wherein incorporation of said modified amino acid occurs in position Arg₂.

7. The method according to one of the preceding claims, wherein one of the following microcystins is produced:
D-Ala₁-X₂-D-MeAsp₃-Z₄-Adda₅-DGlu₆-Mdha₇,
| Ala₁ | X₂ | D-MeAsp₃ | Z₄ | Adda₅ | DGlu₆ | Mdha₇ |
|---|---|---|---|---|---|---|
| D-Ala | Modified substrate | D-MeAsp | Modified substrate | Adda | D-Glu | Mdha |
| D-Ser | | D-Asp | | DM-Adda | D-Glu(OCH₃) | Dha |
| D-Leu | | | | (6Z)Adda | | L-Ser |
| | | | | ADM-Adda | | L-MeSer |
| | | | | | | Dhb |
| | | | | | | MeLan |
wherein "X₂" and/or "Z₄" comprise the at least one modified substrate.

8. Method according to one of the preceding claims, wherein one of the following nodularins is produced:
| MeAsp₁ | Arg₂ | Adda₃ | DGlu₄ | Mdhb₅ |
|---|---|---|---|---|
| D-MeAsp | Arg | Adda | D-Glu | Mdhb |
| D-Asp | Homo-Arg | DM-Adda | D-Glu(OCH₃) | Dhb |
| | | (6Z)Adda | | |
| | | Me-Adda | | |
wherein the position for
| | | |
|---|---|---|
| MeAsp₁ | Arg₂ | Mdhb₅ |
comprises the at least one modified substrate.

9. The method according to claim 8, wherein the nodularin comprises the modified substrate at the Arg₂ position.

10. The method according to one of the preceding claims, wherein the concentration of the modified substrate or amino acid in the media is between 5 and 500 µM and/or an additional ingredients is added: DMSO.

11. The method according to one of the preceding claims, wherein the conjugation chemistry reaction (incl. click chemistry reaction) of the modified amino acid is selected from the group consisting of copper(I)-catalyzed azide-alkyne cycloaddition, strain promoted azide-alkyne cycloaddition, alkyne-azide cycloaddition, or alkene-tetrazine inverse-demand Diels-Alder reaction as well as reactions exploiting the specific reactivities of primary amines, thiols, aldehydes, carboxyls, and oximes.

12. The method according to one of the preceding claims, wherein the cyanobacteria strain is selected from the group consisting of Microcystis, Planktothrix, Oscillatoria, Nostoc, Anabaena, Aphanizomenon, Hapalosiphon, Nodularia, Lyngbya, Phormidium, Spirulina, Halospirulina, Arthrospira, Trichodesmium, Leptolyngbya, Plectonema, Myxosarcina, Pleurocapsa, Pseudanabaena, Geitlerinema, Euhalothece, Calothrix, Tolypothrix, Scytonema, Fischerella, Mastigocladus, Westiellopsis, Stigonema, Chlorogloeopsis, Cyanospira, Cylindrospermopsis, Cylindrospermum, Microchaete, Rivularia, Autosira, Trichonema, Trichodesmium, Symploca, Starria, Prochlorothrix, Microcoleus, Limnothrix, Crinalium, Borzia, Chroococcidiopsis, Cyanocystis, Dermocarpella, Staniera, Xenococcus, Chamaesiphon, Chroococcus, Cyanobacterium, Cyanobium, Cyanothece, Dactylococcopsis, Gloeobacter, Gloeocapsa, Gloeothece.

13. A method of producing a compound for targeted therapy comprising a non-ribosomal peptide linked to a targeting moiety, with the steps:
A) Providing a targeting moiety and a non-ribosomal peptide comprising at least one modified amino acid, wherein the at least one modified amino acid comprises an anchor group directly accessible or transformable for use in conjugation chemistry incl. click chemistry by performing a method according to any one of the preceding claims,
B) Attaching said targeting moiety to said non-ribosomal peptide via chemical conjugation to said anchor group.

14. The method according to claim 13, wherein the targeting moiety is attached via a linker arranged between the modified amino acid and the targeting moiety.

15. The method according to claim 13 or 14, wherein the targeting moiety is an antibody.

## Patentansprüche

1. Ein Verfahren zur Herstellung eines modifizierten nicht-ribosomalen Peptids aus Cyanobakterien, umfassend die folgenden Schritte:
a) Wachsen eines ein nicht-ribosomales Peptid herstellenden Cyanobakterienstammes in Kulturmedium,
b) Zugeben eines oder mehrerer modifizierter Substrate zu der Kultur, und
c) Wachsen des Stammes in Gegenwart des oder der modifizierten Substrate,
d) wobei das modifizierte Substrat eine modifizierte Aminosäure ist, welche eine Ankergruppe umfasst, die zur Verwendung in Konjugationschemie einschließlich Clickchemie direkt zugänglich oder transformierbar ist, für die Anbindung einer zielführenden Einheit oder einer Markierung.

2. Das Verfahren nach Anspruch 1, wobei der das nicht-ribosomale Peptid herstellende Stamm derart ausgewählt ist, dass der Einbau des modifizierten Substrats in das nicht-ribosomale Peptid an einer definierten Position erfolgt.

3. Das Verfahren nach Anspruch 1 oder 2, wobei das nicht-ribosomale Peptid ein Microcystin ist, welches die folgende allgemeine Struktur aufweist:
D-Ala₁-X₂-D-MeAsp₃-Z₄-Adda₅-DGlu₆-Mdha₇
und das eine oder die mehreren modifizierten Substrate an einer beliebigen Position mit Ausnahme von Adda₅ und DGlu₆ eingebaut sind.

4. Das Verfahren nach Anspruch 3, wobei der Einbau der modifizierten Aminosäure an Position X₂ und/oder Z₄ erfolgt.

5. Das Verfahren nach Anspruch 1 oder 2, wobei das nicht-ribosomale Peptid ein Nodularin ist, welches die die folgende allgemeine Struktur aufweist:
D- MeAsp₁-Arg₂-Adda₃-DGlU₄- Mdhb₅
und das eine oder die mehreren modifizierten Substrate an einer beliebigen Position mit Ausnahme von Adda₃ und DGlu₄ eingebaut sind.

6. Das Verfahren nach Anspruch 5, wobei der Einbau der modifizierten Aminosäure an Position Arg₂ erfolgt.

7. Das Verfahren nach einem der vorhergehenden Ansprüche, wobei eines der folgenden Microcystine hergestellt wird:
D-Ala₁-X₂-D-MeAsp₃-Z₄-Adda₅-DGlu₆-Mdha₇,
| Ala₁ | X₂ | D-MeAsp₃ | Z₄ | Adda₅ | DGlu₆ | Mdha₇ |
|---|---|---|---|---|---|---|
| D-Ala | Modifiziertes Substrat | D-MeAsp | Modifiziertes Substrat | Adda | D-Glu | Mdha |
| D-Ser | | D-Asp | | DM-Adda | D-Glu(OCH₃) | Dha |
| D-Leu | | | | (6Z)Adda | | L-Ser |
| | | | | ADM-Adda | | L-MeSer |
| | | | | | | Dhb |
| | | | | | | MeLan |
wobei "X₂" und/oder "Z₄" das wenigstens eine modifizierte Substrat umfassen.

8. Das Verfahren nach einem der vorhergehenden Ansprüche, wobei eines der folgenden Nodularine hergestellt wird:
| MeAsp₁ | Arg₂ | Adda₃ | DGlu₄ | Mdhb₅ |
|---|---|---|---|---|
| D-MeAsp | Arg | Adda | D-Glu | Mdhb |
| D-Asp | Homo-Arg | DM-Adda | D-Glu(OCH₃) | Dhb |
| | | (6Z)Adda | | |
| | | Me-Adda | | |
wobei die Position für
| | | |
|---|---|---|
| MeAsp₁ | Arg₂ | Mdhb₅ |
das wenigstens eine modifizierte Substrat umfasst.

9. Das Verfahren nach Anspruch 8, wobei das Nodularin das modifizierte Substrat an der Arg₂-Position umfasst.

10. Das Verfahren nach einem der vorhergehenden Ansprüche, wobei die Konzentration des modifizierten Substrats oder der Aminosäure in dem Kulturmedium zwischen 5 und 500 µM liegt und/oder ein zusätzlicher Bestandteil zugegeben wird: DMSO.

11. Das Verfahren nach einem der vorhergehenden Ansprüche, wobei die Konjugationschemiereaktion (einschließlich Clickchemiereaktion) der modifizierten Aminosäure aus der Gruppe ausgewählt ist bestehend aus Kupfer(I)-katalysierter Azid-Alkin-Cycloaddition, spannungsgeförderter Azid-Alkin-Cycloaddition, Alkin-Azid-Cycloaddition oder Alken-Tetrazin-Diels-Alder-Reaktion mit inversem Bedarf, sowie Reaktionen, welche die spezifischen Reaktivitäten von primären Aminen, Thiolen, Aldehyden, Carboxylen und Oximen ausnutzen.

12. Das Verfahren nach einem der vorhergehenden Ansprüche, wobei der Cyanobakterienstamm aus der Gruppe ausgewählt ist bestehend aus Microcystis, Planktothrix, Oscillatoria, Nostoc, Anabaena, Aphanizomenon, Hapalosiphon, Nodularia, Lyngbya, Phormidium, Spirulina, Halospirulina, Arthrospira, Trichodesmium, Leptolyngbya, Plectonema, Myxosarcina, Pleurocapsa, Pseudanabaena, Geitlerinema, Euhalothece, Calothrix, Tolypothrix, Scytonema, Fischerella, Mastigocladus, Westiellopsis, Stigonema, Chlorogloeopsis, Cyanospira, Cylindrospermopsis, Cylindrospermum, Microchaete, Rivularia, Autosira, Trichonema, Trichodesmium, Symploca, Starria, Prochlorothrix, Microcoleus, Limnothrix, Crinalium, Borzia, Chroococcidiopsis, Cyanocystis, Dermocarpella, Staniera, Xenococcus, Chamaesiphon, Chroococcus, Cyanobacterium, Cyanobium, Cyanothece, Dactylococcopsis, Gloeobacter, Gloeocapsa, Gloeothece.

13. Ein Verfahren zur Herstellung einer Verbindung für eine gezielte Therapie umfassend ein nicht-ribosomales Peptid, welches mit einer zielführenden Einheit verknüpft ist, mit den folgenden Schritten:
A) Bereitstellen einer zielführenden Einheit und eines nicht-ribosomalen Peptids umfassend wenigstens eine modifizierte Aminosäure, wobei die wenigstens eine modifizierte Aminosäure eine Ankergruppe umfasst, die zur Verwendung in Konjugationschemie, einschließlich Clickchemie, direkt zugänglich oder transformierbar ist, durch Durchführung eines Verfahrens nach einem der vorhergehenden Ansprüche,
B) Anbinden der zielführenden Einheit an das nicht-ribosomale Peptid über chemische Konjugation an die Ankergruppe.

14. Das Verfahren nach Anspruch 13, wobei die zielführende Einheit über eine Verbindungseinheit angebunden ist, die zwischen der modifizierten Aminosäure und der zielführenden Einheit angeordnet ist.

15. Das Verfahren nach Anspruch 13 oder 14, wobei die zielführende Einheit ein Antikörper ist.

## Revendications

1. Procédé de production d'un peptide non ribosomique modifié à partir de cyanobactéries, comprenant les étapes consistant à:
a) faire croître un peptide non ribosomique produisant une souche de cyanobactérie dans un milieu de culture,
b) ajouter un ou plusieurs substrats modifiés à ladite culture, et
c) faire croître la souche en présence desdits substrats modifiés,
d) dans lequel le substrat modifié est
un acide aminé modifié, qui comprend un groupe d'ancrage directement accessible ou transformable pour être utilisé en chimie de conjugaison, y compris en chimie d'affinité, pour l'attachement d'une fraction de ciblage ou d'un marqueur.

2. Procédé selon la revendication 1, dans lequel la souche productrice de peptide non ribosomique est choisie de telle sorte que l'incorporation du substrat modifié dans le peptide non ribosomique se produit à une position définie.

3. Procédé selon la revendication 1 ou 2, dans lequel le peptide non ribosomique est la microcystine et le ou les substrats modifiés sont incorporés dans une position quelconque autre que Adda₅ et DGlu₆, qui a la structure générale suivante :
D-Ala₁-X₂-D-MeAsp₃-Z₄-Adda₅-DGlu₆-Mdha₇.

4. Procédé selon la revendication 3, dans lequel l'incorporation dudit acide aminé modifié se produit dans la position X₂ et/ou Z₄.

5. Procédé selon la revendication 1 ou 2, dans lequel le peptide non ribosomique est la nodularine et le ou les substrats modifiés sont incorporés dans une position quelconque autre que Adda₃ et DGlu₄, qui a la structure générale suivante :
D- MeAsp₁-Arg₂- Adda₃-DGlu₄- Mdhb₅.

6. Procédé selon la revendication 5, dans lequel l'incorporation dudit acide aminé modifié se produit en position Arg₂.

7. Procédé selon l'une quelconque des revendications précédentes, dans lequel l'une des microcystines suivantes est produite:
D-Ala₁-X₂-D-MeAsp₃-Z₄-Adda₅-DGlu₆-Mdha₇,
| | | | | | | |
|---|---|---|---|---|---|---|
| Ala₁ | X₂ | D-MeAsp₃ | Z₄ | Adda₅ | DGlu₆ | Mdha₇ |
| D-Ala | Substrat modifié | D-MeAsp | Substrat modifié | Adda | D-Glu | Mdha |
| D-Ser | | D-Asp | | DM-Adda | D-Glu(OCH₃) | Dha |
| D-Leu | | | | (6Z)Adda | | L-Ser |
| | | | | ADM-Adda | | L-MeSer |
| | | | | | | Dhb |
| | | | | | | MeLan |
dans lequel « X₂ » et/ou « Z₄ » comprennent l'au moins un substrat modifié.

8. Procédé selon l'une quelconque des revendications précédentes, dans lequel l'une des nodularines suivantes est produite:
| MeAsp₁ | Arg₂ | Adda₃ | DGlu₄ | Mdhb₅ |
|---|---|---|---|---|
| D-MeAsp | Arg | Adda | D-Glu | Mdhb |
| D-Asp | Homo-Arg | DM-Adda | D-Glu(OCH₃) | Dhb |
| | | (6Z)Adda | | |
| | | Me-Adda | | |
dans lequel la position de
| | | |
|---|---|---|
| MeAsp₁ | Arg₂ | Mdhb₅ |
comprend l'au moins un substrat modifié.

9. Procédé selon la revendication 8, dans lequel la nodularine comprend le substrat modifié en position Arg₂.

10. Procédé selon l'une quelconque des revendications précédentes, dans lequel la concentration du substrat modifié ou de l'acide aminé dans le milieu est comprise entre 5 et 500 µM et/ou un autre ingrédient est ajouté : du DMSO.

11. Procédé selon l'une quelconque des revendications précédentes, dans lequel la réaction de la chimie de conjugaison (y compris la chimie d'affinité) de l'acide aminé modifié est choisie dans le groupe constitué par une cycloaddition d'azide-alcyne catalysée par le cuivre (I), une cycloaddition d'azide-alcyne promue par contrainte, une cycloaddition d'alcyne-azoture, ou une réaction de Diels-Alder à demande inverse d'alcène-tétrazine, ainsi que des réactions exploitant les réactivités spécifiques d'aminés primaires, de thiols, d'aldéhydes, de carboxyles et d'oximes.

12. Procédé selon l'une quelconque des revendications précédentes, dans lequel la souche de cyanobactérie est choisi dans le groupe constitué de Microcystis, Planktothrix, Oscillatoria, Nostoc, Anabaena, Aphanizomenon, Hapalosiphon, Nodularia, Lyngbya, Phormidium, Spirulina, Halospirulina, Arthrospira, Trichodesmium, Leptolyngbya, Plectonema, Myxosarcina, Pleurocapsa, Pseudanabaena, Geitlerinema, Euhalothece, Calothrix, Tolypothrix, Scytonema, Fischerella, Mastigocladus, Westiellopsis, Stigonema, Chlorogloeopsis, Cyanospira, Cylindrospermopsis, Cylindrospermum, Microchaete, Rivularia, Autosira, Trichonema, Trichodesmium, Symploca, Starria, Prochlorothrix, Microcoleus, Limnothrix, Crinalium, Borzia, Chroococcidiopsis, Cyanocystis, Dermocarpella, Staniera, Xenococcus, Chamaesiphon, Chroococcus, Cyanobacterium, Cyanobium, Cyanothece, Dactylococcopsis, Gloeobacter, Gloeocapsa, Gloeothece.

13. Procédé de production d'un composé pour une thérapie ciblée, comprenant un peptide non ribosomique lié à une fraction de ciblage, au moyen des étapes consistant à :
A) fournir une fraction de ciblage et un peptide non ribosomique comprenant au moins un acide aminé modifié, dans lequel l'au moins un acide aminé modifié comprend un groupe d'ancrage directement accessible ou transformable pour une utilisation dans une chimie de conjugaison, y compris en chimie d'affinité, par la mise en œuvre d'un procédé selon l'une quelconque des revendications précédentes,
B) attacher ladite fraction de ciblage audit peptide non ribosomique par l'intermédiaire d'une conjugaison chimique audit groupe d'ancrage.

14. Procédé selon la revendication 13, dans lequel la fraction de ciblage est attachée par l'intermédiaire d'un lieur disposé entre l'acide aminé modifié et la fraction de ciblage.

15. Procédé selon la revendication 13 ou 14, dans lequel la fraction de ciblage est un anticorps.
